# EUROPEAN PATENT APPLICATION

(11) **EP 3 279 995 A1**
(43) Date of publication of application: **07.02.2018**
(21) Application number: 16772867.4
(22) Date of filing: 29.03.2016
(51) Int. Cl.: H01M 10/0567, H01G 11/64, H01M 10/052

(54) **ADDITIVE FOR NON-AQUEOUS ELECTROLYTE, NON-AQUEOUS ELECTROLYTE, AND POWER STORAGE DEVICE**

(30) Priority: 31.03.2015 JP 2015073142; 31.03.2015 JP 2015073146; 31.03.2015 JP 2015072756; 31.03.2015 JP 2015072768; 31.03.2015 JP 2015072758; 31.03.2015 JP 2015072764
(71) Applicant: Sumitomo Seika Chemicals Co., Ltd., Kako-gun, Hyogo 675-0145 (JP)
(72) Inventor: KONO Yuki, Kako-gun Hyogo 675-0145 (JP); TAKAI Yasuyuki, Kako-gun Hyogo 675-0145 (JP); FUJITA Koji, Kako-gun Hyogo 675-0145 (JP); YAMAMOTO Noriko, Kako-gun Hyogo 675-0145 (JP)
(74) Representative: Nederlandsch Octrooibureau
(86) International application number: PCT/JP2016/060201
(87) International publication number: WO 2016/158986

(57) **Abstract**

An additive for a non-aqueous electrolyte solution including a compound represented by the following formula (A1) or (A2).

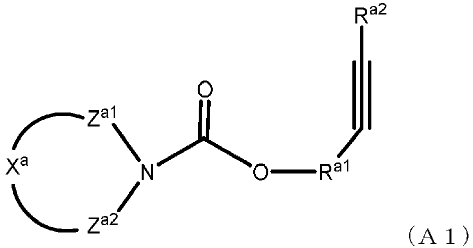

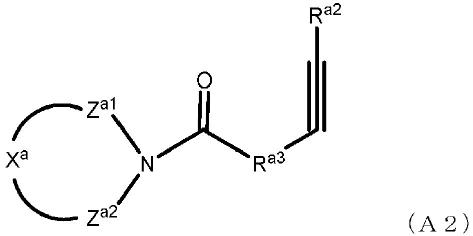

In the formula, X^{a} represents a group that forms a cyclic group together with a nitrogen atom, Z^{a1} and Z^{a2}, Z^{a1} and Z^{a2} each independently represent a sulfonyl or carbonyl group, R^{a1} represents an optionally substituted alkylene group of 1 to 4 carbon atoms, an alkenylene group of 3 or 4 carbon atoms or an alkynylene group of 3 or 4 carbon atoms, R^{a2} represents a hydrogen atom, a halogen atom or an optionally substituted alkyl group of 1 to 4 carbon atoms, R^{a3} represents oxygen or an optionally substituted alkylene group of 1 to 4 carbon atoms, alkenylene group of 3 or 4 carbon atoms or alkynylene group of 3 or 4 carbon atoms.

## Description

### Technical Field

The present invention relates to an additive for a non-aqueous electrolyte solution, to a non-aqueous electrolyte solution, and to a power storage device.

### Background Art

In recent years, as increasing attention is being focused on solving environmental problems and creating a society that recycles resources in a sustainable manner, extensive research is being conducted on non-aqueous electrolyte solution secondary batteries represented by lithium ion batteries. Lithium ion batteries have high working voltage and energy density, and are therefore used as power sources for laptop computers, cellular phones and the like. Because lithium ion batteries have higher energy density than lead-acid batteries or nickel cadmium batteries, they are seen as promising for realizing increasingly higher battery capacities.

However, lithium ion batteries have been problematic in that their battery capacity decreases with successive charge-discharge cycles. The cause of the capacity reduction is believed to be that prolonged charge-discharge cycles lead to decomposition of the electrolyte solution by electrode reaction, reduced impregnability of the electrolyte in the electrode active material layer, and reduced intercalation efficiency of lithium ion.

Methods of adding various additives to electrolyte solutions have been studied as methods of minimizing the reduction in capacity of batteries that occurs with charge-discharge cycles. Additives generally decompose during initial charge-discharge, forming a coating known as a solid electrolyte interface (SEI) on the electrode surface. Because the SEI is formed in the initial charge-discharge cycle, lithium ions are able to enter and leave the electrode through the SEI in subsequent charge-discharge while reducing consumption of electricity by decomposition of the electrolyte solution. In other words, it is thought that formation of the SEI minimizes deterioration of the secondary battery with repeated charge-discharge cycles, thus playing a major role in improving the cell characteristic, storage characteristic and load characteristic.

Examples of electrolyte solution additives include the cyclic monosulfonic acid esters disclosed in PTLs 1 to 3, the sulfur-containing aromatic compounds disclosed in PTL 4, the disulfide compounds disclosed in PTL 5 and the disulfonic acid esters disclosed in PTLs 6 to 9. In addition, PTLs 10 to 15 disclose electrolyte solutions containing cyclic carbonic acid esters or cyclic sulfones, PTL 16 discloses an electrolyte solution containing compounds having a nitrogen-containing cyclic group and an electron-withdrawing group, and PTL 17 discloses an electrolyte solution containing a compound that includes a sulfonamide group.

### Citation List

### Patent Literature

[PTL 1] Japanese Unexamined Patent Publication SHO No. 63-102173
[PTL 2] Japanese Unexamined Patent Publication No. 2000-003724
[PTL 3] Japanese Unexamined Patent Publication HEI No. 11-339850
[PTL 4] Japanese Unexamined Patent Publication HEI No. 05-258753
[PTL 5] Japanese Unexamined Patent Publication No. 2001-052735
[PTL 6] Japanese Unexamined Patent Publication No. 2009-038018
[PTL 7] Japanese Unexamined Patent Publication No. 2005-203341
[PTL 8] Japanese Unexamined Patent Publication No. 2004-281325
[PTL 9] Japanese Unexamined Patent Publication No. 2005-228631
[PTL 10] Japanese Unexamined Patent Publication HEI No. 04-87156
[PTL 11] Japanese Unexamined Patent Publication HEI No. 10-50342
[PTL 12] Japanese Unexamined Patent Publication HEI No. 08-45545
[PTL 13] Japanese Unexamined Patent Publication No. 2001-6729
[PTL 14] Japanese Unexamined Patent Publication SHO No. 63-102173
[PTL 15] Japanese Unexamined Patent Publication HEI No. 05-074486
[PTL 16] Japanese Unexamined Patent Publication No. 2014-127354
[PTL 17] Japanese Unexamined Patent Publication No. 2014-194866

### Non Patent Literature

[NPL 1] Geun-Chang, Hyung-Jin kim, Seung-ll Yu, Song-Hui Jun, Jong-Wook Choi, Myung-Hwan Kim. Journal of The Electrochemical Society, 147, 12, 4391(2000)

### Summary of Invention

### Technical Problem

Compounds with low Lowest Unoccupied Molecular Orbital (LUMO) energies are excellent electron acceptors, and are thought to be capable of forming stable SEI on the electrode surfaces of non-aqueous electrolyte solution secondary batteries and the like (see NPL 1, for example).

Some conventional additives, such as the compounds disclosed in PTLs 1 to 9, while exhibiting low LUMO energies, are chemically unstable and have been prone to deterioration by the effects of moisture and temperature. For example, disulfonic acid ester compounds exhibit low LUMO energy but have low stability and easily deteriorate in the presence of moisture. Therefore, when disulfonic acid ester compounds are stored for prolonged periods it has been necessary to conduct strict management for moisture content and temperature. Generally, Lithium ion batteries are required to have a heat-resistant temperature of about 60°C, and lithium ion capacitors are required to have a heat-resistant temperature of about 80°C. Therefore, increasing the high-temperature stability of additives for non-aqueous electrolyte solutions to be used in power storage devices has been an important issue.

Furthermore, in the case of electrolyte solutions containing conventional additives, when power storage devices are used over long periods of time while repeating charge-discharge cycles, the cell characteristics of the power storage devices are easily reduced, and therefore further improvement is desired in terms of the cycle characteristics.

The electrolyte solutions described in PTLs 10 to 14 can minimize irreversible reduction in capacity to some extent, due to the SEI formed on the negative electrode surface by electrochemical reductive decomposition. However, the SEI formed by the additives in such electrolyte solutions, while exhibiting excellent performance for electrode protection, have been inadequate from the standpoint of strength for withstanding prolonged use. Problems have therefore existed, that exposure of the negative electrode surface by decomposition of the SEI or generation of cracks in the SEI during use of the power storage device leads to decomposition of the electrolyte solution solvent, which deteriorates the cell characteristic. The electrolyte solution using a vinylene carbonate-based compound as an additive which is described in PTL 15 has had a problem in that it generates gas including carbon dioxide when the vinylene carbonate is decomposed on the electrode, and this has led to reduced battery performance. The gas generation is particularly notable at high temperatures, or when the charge-discharge cycle is repeated over long periods of time.

Thus, additives for non-aqueous electrolyte solutions have still been in need of improvement from the standpoint of storage stability, a cycle characteristic whereby performance is maintained after repeated charge-discharge cycles, or suppression of gas generation.

It is therefore the main object of the present invention to provide an additive for a non-aqueous electrolyte solution that has high storage stability, and that can improve cycle characteristics and suppress gas generation for power storage devices.

### Solution to Problem

The present inventors have found that compounds including a specific partial structure exhibit low LUMO energy and are chemically stable. The present inventors have also found that when such compounds are used as additives for non-aqueous electrolyte solutions, excellent cycle characteristics can be obtained and gas generation can be reduced, and the present invention has thereupon been completed.

Specifically, one aspect of the invention provides an additive for a non-aqueous electrolyte solution including a compound represented by the following formula (A1) or the following formula (A2).

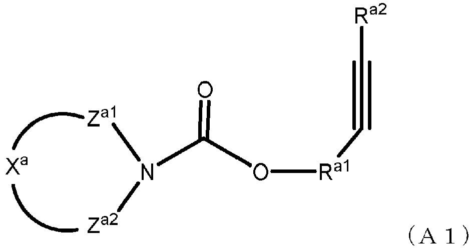

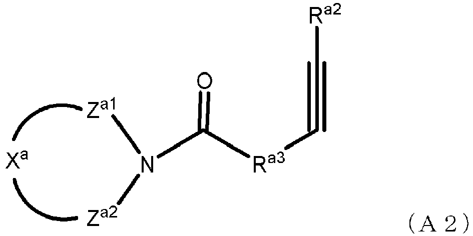

In the formula, X^{a} represents a group that forms a cyclic group together with a nitrogen atom, Z^{a1} and Z^{a2}, Z^{a1} and Z^{a2} each independently represent a sulfonyl or carbonyl group, R^{a1} represents an optionally substituted alkylene group of 1 to 4 carbon atoms, alkenylene group of 3 or 4 carbon atoms or alkynylene group of 3 or 4 carbon atoms, R^{a2} represents a hydrogen atom, a halogen atom or an optionally substituted alkyl group of 1 to 4 carbon atoms, R^{a3} represents an oxygen atom, an optionally substituted alkylene group of 1 to 4 carbon atoms, alkenylene group of 3 or 4 carbon atoms or alkynylene group of 3 or 4 carbon atoms.

The compound represented by formula (A1) or formula (A2) has a cyclic structure including a carboxylic acid amide bond or sulfonamide bond, and a monovalent group with a carbon-carbon triple bond and a carbonyl group is additionally bonded to the nitrogen atom. It is therefore believed that the compound represented by formula (A1) or formula (A2) undergoes ring opening by electrochemical reduction and forms a SEI with multiple polar groups including nitrogen atoms, oxygen atoms and sulfur atoms. The SEI with multiple polar groups presumably exhibits excellent ionic conductance and has sufficient strength to withstand prolonged use. In addition, it is thought that by having a carbon-carbon triple bond, the SEI starting at that origin can extend out three-dimensionally.

Another aspect of the invention provides an additive for a non-aqueous electrolyte solution including a compound represented by the following formula (B1).

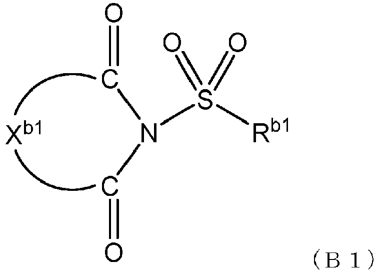

In formula (B1), X^{b1} represents a group that forms a cyclic group together with a nitrogen atom and two carbon atoms, and R^{b1} represents an optionally substituted alkoxy group of 1 to 4 carbon atoms, an optionally substituted alkenyl group of 2 to 4 carbon atoms, an optionally substituted alkynyl group of 2 to 4 carbon atoms, an optionally substituted aryloxy group, an optionally substituted alkenyloxy group of 2 to 6 carbon atoms, an optionally substituted alkynyloxy group of 2 to 4 carbon atoms or an optionally substituted amino group.

The compound represented by formula (B1) has a cyclic structure including a nitrogen atom and two carbonyl groups bonded to it, and it additionally has a sulfonyl group bonded to the nitrogen atom. It is therefore believed that the compound represented by formula (B1) undergoes ring opening by electrochemical reduction and forms a SEI with multiple polar groups including nitrogen atoms, oxygen atoms and sulfur atoms. The SEI with multiple polar groups presumably exhibits excellent ionic conductance and has sufficient strength to withstand prolonged use.

Another aspect of the invention provides an additive for a non-aqueous electrolyte solution including a compound represented by the following formula (C1).

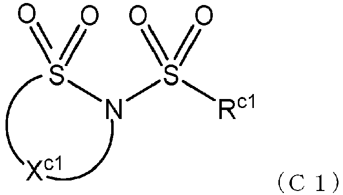

In formula (C1), X^{c1} represents a group that forms a cyclic group together with a sulfur atom and a nitrogen atom, and R^{c1} represents an optionally substituted alkyl group of 1 to 4 carbon atoms, an optionally substituted alkenyl group of 2 to 4 carbon atoms, an optionally substituted alkynyl group of 1 to 4 carbon atoms, an optionally substituted alkoxy group of 1 to 4 carbon atoms, an optionally substituted aryl group, an optionally substituted heteroaryl group, an optionally substituted aryloxy group, an optionally substituted heteroaryloxy group, an optionally substituted alkenyloxy group of 2 to 4 carbon atoms, an optionally substituted alkynyloxy group of 2 to 4 carbon atoms or an optionally substituted amino group.

The compound represented by formula (C1) has a cyclic structure including a sulfonamide bond, and it additionally has a sulfonyl group bonded to the sulfonamide bond. It is therefore believed that the compound represented by formula (C1) undergoes ring opening by electrochemical reduction and forms a SEI with multiple polar groups including nitrogen atoms and sulfur atoms. The SEI with multiple polar groups presumably exhibits excellent ionic conductance and has sufficient strength to withstand prolonged use.

Another aspect of the invention provides an additive for a non-aqueous electrolyte solution including a compound represented by the following formula (D1).

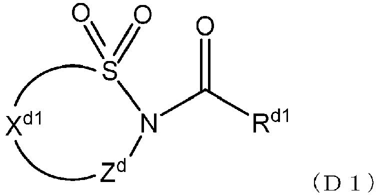

In formula (D1), X^{d1} represents a group that forms a cyclic group together with a sulfur atom, a nitrogen atom and Z^{d}, Z^{d} represents a sulfonyl or carbonyl group, and R^{d1} represents an optionally substituted alkyl group of 1 to 4 carbon atoms, an optionally substituted alkenyl group of 2 to 4 carbon atoms, an optionally substituted alkoxy group of 1 to 4 carbon atoms, an optionally substituted aryl group, an optionally substituted heteroaryl group, an optionally substituted aryloxy group, an optionally substituted heteroaryloxy group, an optionally substituted alkenyloxy group of 2 to 4 carbon atoms or an optionally substituted amino group.

The compound represented by formula (D1) has a cyclic structure including a nitrogen atom and a sulfonyl group and Z^{d} (a sulfonyl or carbonyl group) bonded to it, and it additionally has a carbonyl group bonded to the nitrogen atom. It is therefore believed that the compound represented by formula (D1) undergoes ring opening by electrochemical reduction and forms a SEI with multiple polar groups including nitrogen atoms, oxygen atoms and sulfur atoms. The SEI with multiple polar groups presumably exhibits excellent ionic conductance and has sufficient strength to withstand prolonged use.

Another aspect of the invention provides an additive for a non-aqueous electrolyte solution including a compound represented by the following formula (D2).

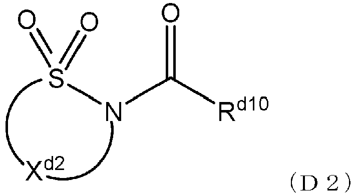

In formula (D2), X^{d2} represents a group that forms a cyclic group together with a sulfur atom and a nitrogen atom, and R^{d10} represents an optionally substituted alkenyl group of 2 to 4 carbon atoms, an optionally substituted alkoxy group of 1 to 4 carbon atoms, an optionally substituted aryl group, an optionally substituted heteroaryl group, an optionally substituted aryloxy group, an optionally substituted heteroaryloxy group, an optionally substituted alkenyloxy group of 2 to 4 carbon atoms or an optionally substituted amino group.

The compound represented by formula (D2) has a cyclic structure including a nitrogen atom and a sulfonyl group bonded to it, and it additionally has a carbonyl group bonded to the nitrogen atom. It is therefore believed that the compound represented by formula (D2) undergoes ring opening by electrochemical reduction and forms a SEI with multiple polar groups including nitrogen atoms, oxygen atoms and sulfur atoms. The SEI with multiple polar groups presumably exhibits excellent ionic conductance and has sufficient strength to withstand prolonged use.

Another aspect of the invention provides an additive for a non-aqueous electrolyte solution including a compound represented by the following formula (E1) or formula (E2).

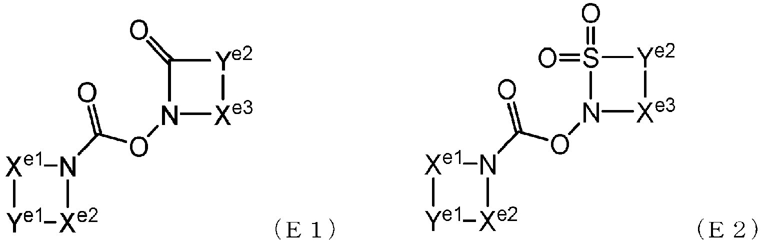

In formula (E1) or formula (E2), X^{e1}, X^{e2} and X^{e3} each independently represent an optionally substituted methylene, sulfonyl or carbonyl group. In formula (E1) or formula (E2), Y^{e1} and Y^{e2} each independently represent an optionally substituted hydrocarbon group of 1 to 6 carbon atoms.

Presumably, when the compound represented by formula (E1) or formula (E2) undergoes electrochemical reduction, the cyclic amide or cyclic sulfonamide undergoes ring opening and a SEI with multiple polar groups including N, O and S is formed. Since the SEI with multiple polar groups including N, O and S can exhibit excellent ionic conductance, it is considered to be a SEI with exceedingly high performance. In addition, the compound represented by formula (E1) or formula (E2) is thought to form the SEI by ring-opening polymerization of the cyclic amide or cyclic sulfonamide. Formation of the SEI in this manner presumably exhibits an effect of reducing the likelihood of disintegration of the SEI that takes place with charge-discharge, and decomposition of the electrolyte solution, improving the cell characteristics such as the cycle characteristic, charge-discharge capacity and internal resistance, and minimizing generation of gas.

Another aspect of the invention provides an additive for a non-aqueous electrolyte solution including a compound represented by the following formula (F1).

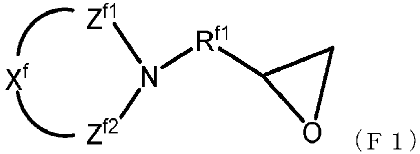

In formula (F1), X^{f} represents a group that forms a cyclic group together with a nitrogen atom, Z^{f1} and Z^{f2}, Z^{f1} and Z^{f2} each independently represent a sulfonyl or carbonyl group, and R^{f1} represents a divalent linker group.

The compound represented by formula (F1) has a cyclic structure including a carboxylic acid amide bond or sulfonamide bond, and a monovalent group glycidyl group is additionally bonded to the nitrogen atom. It is therefore believed that the compound represented by formula (F1) undergoes ring opening by electrochemical reduction and forms a SEI with multiple polar groups including nitrogen atoms, oxygen atoms and sulfur atoms. The SEI with multiple polar groups presumably exhibits excellent ionic conductance and has sufficient strength to withstand prolonged use. In addition, it is thought that the compound represented by formula (F1) polymerizes and forms the SEI by ring-opening polymerization of the cyclic structure and the glycidyl group. As a result, presumably a denser and stronger SEI is formed, exhibiting the effect of reducing the likelihood of disintegration of the SEI and decomposition of the electrolyte solution that accompany charge-discharge, and of improving the cell characteristic.

### Advantageous Effects of Invention

According to the invention there is provided an additive for a non-aqueous electrolyte solution that has high storage stability, and that can improve the cycle characteristics and suppress gas generation for power storage devices. An additive for a non-aqueous electrolyte solution according to several embodiments, when used in a power storage device such as a non-aqueous electrolyte solution secondary battery or electrical double layer capacitor, can form a stable SEI (solid electrolyte interface) on electrode surfaces to improve the cell characteristics such as the cycle characteristic, charge-discharge capacity and internal resistance.

### Brief Description of Drawings

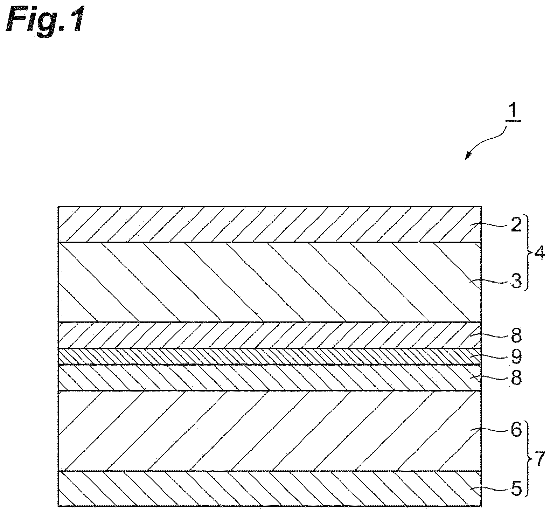

Fig. 1 is a cross-sectional view showing an embodiment of a power storage device.

### Description of Embodiments

A preferred embodiment of the invention will now be described in detail. However, the present invention is not limited to the embodiments described below.

The additive for a non-aqueous electrolyte solution according to one embodiment of the invention includes one or more kinds of compounds represented by the following formula (A1) or the following formula (A2).

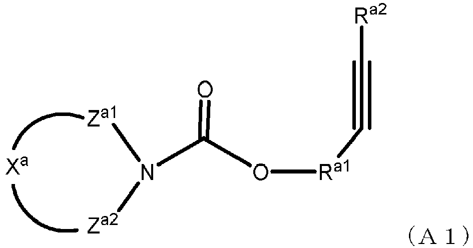

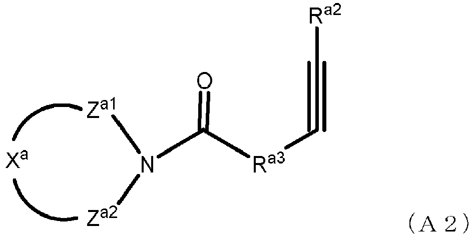

In the formulas, X^{a} represents a group that forms a cyclic group together with the nitrogen atom, Z^{a1} and Z^{a2}, and Z^{a1} and Z^{a2} each independently represent a sulfonyl group (-S(=O)₂-) or a carbonyl group (-C(=O)-). The cyclic group formed by X^{a} may be substituted. The cyclic group may be a 4- to 6-membered ring, and it may be a fused ring comprising two or more rings.

In the formula, R^{a1} represents an optionally substituted alkylene group of 1 to 4 carbon atoms, alkenylene group of 3 or 4 carbon atoms or alkynylene group of 3 or 4 carbon atoms, R^{a2} represents a hydrogen atom, a halogen atom or an optionally substituted alkyl group of 1 to 4 carbon atoms, R^{a3} represents an oxygen atom or an optionally substituted alkylene group of 1 to 4 carbon atoms, alkenylene group of 3 or 4 carbon atoms or alkynylene group of 3 or 4 carbon atoms.

The additive for a non-aqueous electrolyte solution may include a compound represented by formula (A1). Specific examples of compounds of formula (A1) include compounds represented by the following formulas (A1-a), (A1-b), (A1-c) or (A1-d). These compounds provide particularly excellent effects from the standpoint of cycle characteristics and internal resistance.

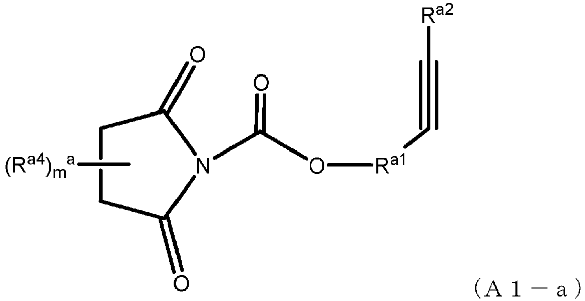

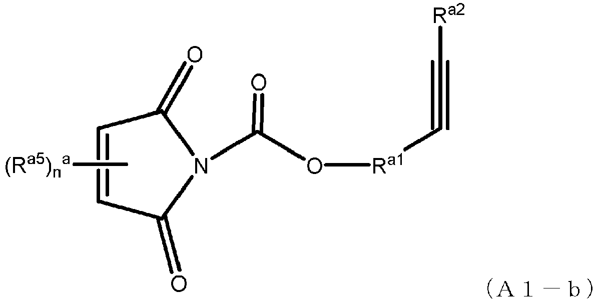

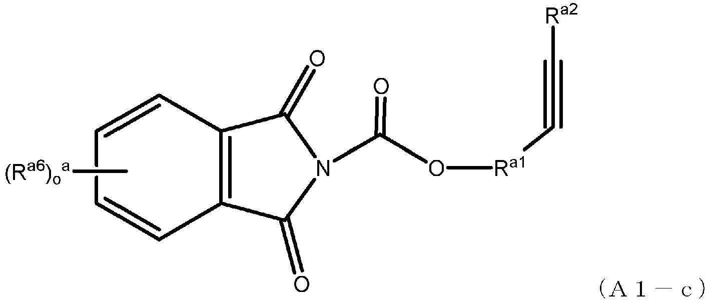

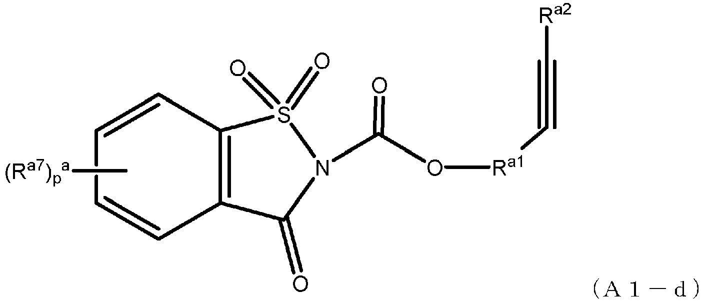

In formulas (A1-a), (A1-b), (A1-c) and (A1-d), R^{a4}, R^{a5}, R^{a6} and R^{a7} each represent an optionally substituted alkyl group of 1 to 4 carbon atoms, an optionally substituted alkoxy group of 1 to 4 carbon atoms, a nitro, amino or sulfonyl group, or a halogen atom. In the case of multiple R^{a4}, R^{a5}, R^{a6} or R^{a7} groups, each of the substituents may be the same or different. m^{a} represents an integer of 0 to 2, n^{a} represents an integer of 0 to 2, o^{a} represents an integer of 0 to 4 and p^{a} represents an integer of 0 to 4. Of these, from the viewpoint of availability and reactivity, m^{a} may be 0 or 1, n^{a} may be 0 or 1, o^{a} may be 0 or 1 and p^{a} may be 0 or 1.

Specific examples of compounds represented by formula (A2) include compounds represented by the following formulas (A2-a), (A2-b), (A2-c) or (A2-d). These compounds provide particularly excellent effects from the standpoint of cycle characteristics and the like.

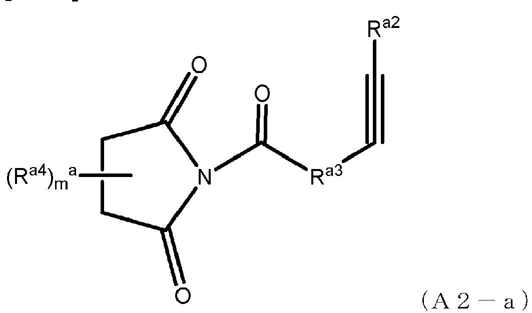

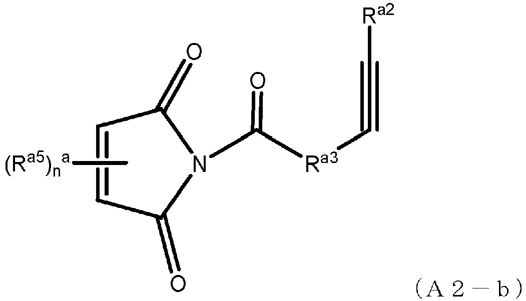

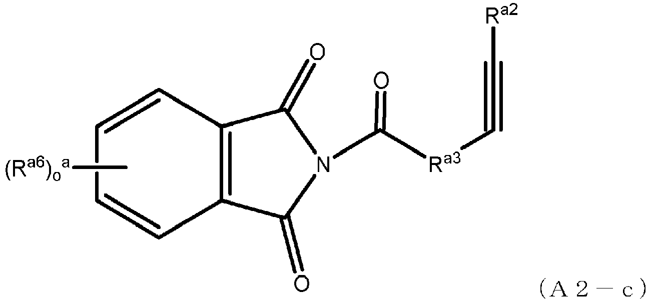

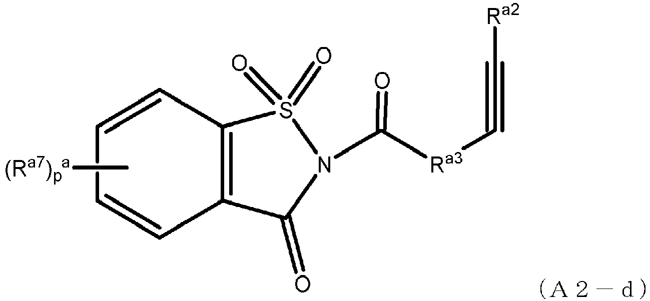

In formulas (A2-a), (A2-b), (A2-c) and (A2-d), R^{a4}, R^{a5}, R^{a6} and R^{a7}, and m^{a}, n^{a}, o^{a} and p^{a}, have the same respective definitions as R^{a4}, R^{a5}, R^{a6} and R^{a7} and m^{a}, n^{a}, o^{a} and p^{a} in formulas (A1-a), (A1-b), (A1-c) and (A1-d). In the case of multiple R^{a4}, R^{a5}, R^{a6} or R^{a7} groups, each of the substituents may be the same or different.

Specific examples of optionally substituted alkyl groups of 1 to 4 carbon atoms as R^{a4}, R^{a5}, R^{a6} or R^{a7} include methyl, trifluoromethyl, ethyl, n-propyl, isopropyl, n-butyl, isobutyl and t-butyl groups. Of these, from the viewpoint of availability and reactivity, R^{a4}, R^{a5}, R^{a6} and R^{a7} may be methyl groups.

Specific examples of optionally substituted alkoxy groups of 1 to 4 carbon atoms as R^{a4}, R^{a5}, R^{a6} or R^{a7} include methoxy, ethoxy, n-propoxy and n-butoxy, trifluoromethoxy, 2,2,2-trifluoroethyloxy and 1,1,2,2,2-pentafluoroethyloxy groups. Of these, from the viewpoint of availability and reactivity, R^{a4}, R^{a5}, R^{a6} and R^{a7} may be methoxy groups.

A halogen atom as R^{a4}, R^{a5}, R^{a6} or R^{a7} may be a fluorine, chlorine, bromine or iodine atom. Of these, from the viewpoint of availability and reactivity, R^{a4}, R^{a5}, R^{a6} and R^{a7} may be fluorine atoms.

R^{a1} in formulas (A1), (A1-a), (A1-b), (A1-c) and (A1-d) represents an optionally substituted alkylene group of 1 to 4 carbon atoms, alkenylene group of 3 or 4 carbon atoms or alkynylene group of 3 or 4 carbon atoms. R^{a3} in formulas (A2), (A2-a), (A2-b), (A2-c) and (A2-d) represents an oxygen atom, an optionally substituted alkylene group of 1 to 4 carbon atoms, alkenylene group of 3 or 4 carbon atoms or alkynylene group of 3 or 4 carbon atoms.

Specific examples of optionally substituted alkylene groups of 1 to 4 carbon atoms as R^{a1} or R^{a3} include methylene, ethylene, propylene, butene, and alkylene groups optionally containing halogen atoms, such as difluoromethylene and 1,1,2,2-tetrafluoroethylene.

Specific examples of alkenylene groups of 3 or 4 carbon atoms as R^{a1} or R^{a3} include 2-propynylene and 3-butenylene.

Specific examples of alkynylene groups of 3 or 4 carbon atoms as R^{a1} or R^{a3} include 2-propargylene groups.

R^{a2} in formulas (A1), (A1-a), (A1-b), (A1-c), (A1-d), (A2), (A2-a), (A2-b), (A2-c) and (A2-d) represents a hydrogen atom, a halogen atom, or an optionally substituted alkyl group of 1 to 4 carbon atoms. Of these, from the viewpoint of increasing the ionic conductivity of the SEI, R^{a2} may be a hydrogen atom or an optionally substituted alkyl group of 1 to 4 carbon atoms.

A halogen atom as R^{a2} may be a fluorine, chlorine, bromine or iodine atom. Of these, from the viewpoint of availability and reactivity, R^{a2} may be a fluorine atom.

Specific examples of optionally substituted alkyl groups of 1 to 4 carbon atoms as R^{a2} include methyl, ethyl, n-propyl, isopropyl, n-butyl, isobutyl, t-butyl, trifluoromethyl and 1,1-dichloroethyl groups.

Of the compounds of formula (A1), examples of compounds represented by formula (A1-a) include N-(1-(2-propynyl)oxycarbonyl)succinimide, N-(1-(2-butynyl)oxycarbonyl)succinimide, N-(1-(3 -butynyl)oxycarbonyl)succinimide and N-(1-fluoro-1-(2-propynyl)oxycarbonyl)succinimide. Examples of compounds represented by formula (A1-b) include N-(1-(2-propynyl))oxycarbonyl)maleimide, N-(1-(2-butynyl)oxycarbonyl)maleimide, N-(1-(2-butynyl)oxycarbonyl)maleimide and N-(1-fluoro-1-(2-propynyl)oxycarbonyl)maleimide. Examples of compounds represented by formula (A1-c) include N-(1-(2-propynyl)oxycarbonyl)phthalimide, N-(1-(2-butynyl)oxycarbonyl)phthalimide, N-(1-(3 -butynyl)oxycarbonyl)phthalimide and N-(1-fluoro-1-(2-propynyl)oxycarbonyl)phthalimide. Examples of compounds represented by formula (A1-d) include N-(1-(2-propynyl)oxycarbonyl)saccharin, N-(1-(2-butynyl)oxycarbonyl)saccharin, N-(1-(3 -butynyl)oxycarbonyl)saccharin and N-(1-fluoro-1 -(2-propynyl)oxycarbonyl)saccharin.

Of the compounds of formula (A2), examples of compounds represented by formula (A2-a) include N-(1-(2-propynyl)carbonyl)succinimide, N-(1-(2-butynyl)carbonyl)succinimide, N-(1-(3 -butynyl)carbonyl)succinimide and N-(1-fluoro-1-(2-propynyl)carbonyl)succinimide. Examples of compounds represented by formula (A2-b) include N-(1-(2-propynyl))carbonyl)maleimide, N-(1-(2-butynyl)carbonyl)maleimide, N-(1-(2-butynyl)carbonyl)maleimide and N-(1-fluoro-1-(2-propynyl)carbonyl)maleimide. Examples of compounds represented by formula (A2-c) include N-(1-(2-propynyl)carbonyl)phthalimide, N-(1-(2-butynyl)carbonyl)phthalimide, N-(1-(3-butynyl)carbonyl)phthalimide and N-(1-fluoro-1-(2-propynyl)carbonyl)phthalimide. Examples of compounds represented by formula (A2-d) include N-(1-(2-propynyl)carbonyl)saccharin, N-(1-(2-butynylcarbonyl)saccharin, N-(1-(3-butynyl)carbonyl)saccharin and N-(1-fluoro-1-(2-propynyl)carbonyl)saccharin.

A person skilled in the art can synthesize a compound of formula (A1) or formula (A2) using available starting materials and combining common reactions. For example, a compound of formula (A2) can be synthesized by a method of reacting a halide with the corresponding cyclic imide compound, or a method of reacting an alcohol and a cyclic imide with triphosgene.

A specific example of producing a compound wherein o^{a} is 0, R^{a2} is a hydrogen atom and R^{a3} is methylene in formula (A2-c) (N-(1-(2-propynyl)carbonyl)phthalimide), will now be described. First, phthalimide and triethylamine are dissolved in an organic solvent, and then propargyl chloroformate is added dropwise and the mixture is stirred at room temperature for 2 hours. Next, the obtained reaction product may be rinsed with water, crystallized and filtered to obtain the target compound. As a different method, first triphosgene is stirred in the presence of activated carbon, propargyl alcohol is added dropwise and the reaction mixture is stirred. Next, phthalimide and triethylamine are added dropwise to the reaction mixture, and stirring is continued. The obtained reaction product may be rinsed with water, crystallized and filtered to obtain the target compound.

The additive for a non-aqueous electrolyte solution according to another embodiment of the invention includes one or more kinds of compounds represented by the following formula (B1).

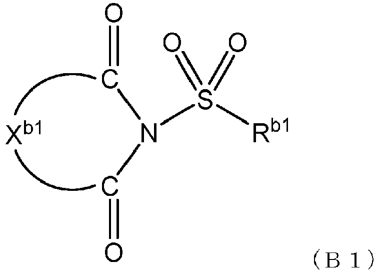

In formula (B1), X^{b1} represents a group that forms a cyclic group together with the nitrogen atom and two carbon atoms. The cyclic group formed by X^{b1} may be substituted. The cyclic group may be a 4- to 6-membered ring, and it may be a fused ring comprising two or more rings.

Specific examples of compounds of formula (B1) include compounds represented by the following formulas (B1-a), (B1-b) and (B1-c). These compounds provide a particularly excellent effect from the standpoint of minimizing gas generation.

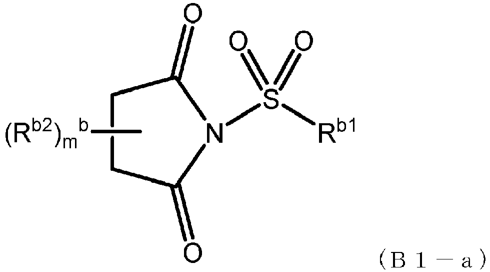

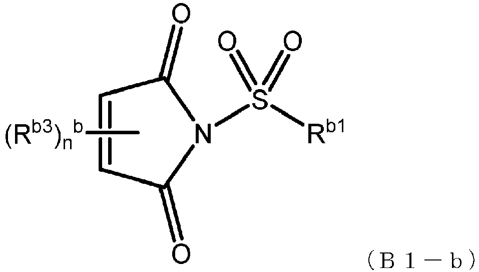

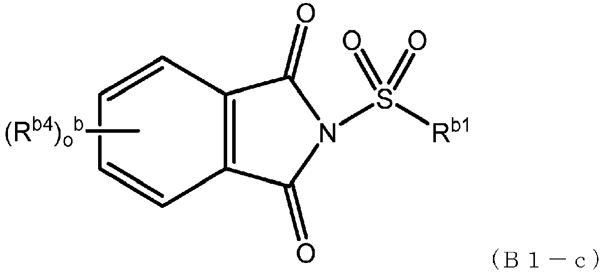

In formulas (B1-a), (B1-b) and (B1-c), R^{b2}, R^{b3} and R^{b4} each represent an optionally substituted alkyl group of 1 to 4 carbon atoms or optionally substituted alkoxy group of 1 to 4 carbon atoms, a nitro, amino or sulfonyl group or a halogen atom. m^{b} represents an integer of 0 to 4, n^{b} represents an integer of 0 to 2, and o^{b} represents an integer of 0 to 4. From the viewpoint of availability and reactivity, m^{b} may be 0 or 1, n^{b} may be 0 or 1 and o^{b} may be 0 or 1. When m^{b}, n^{b} and o^{b} are 2 or greater, multiple R^{b2}, R^{b3} and R^{b4} may respectively be the same or different, but they are preferably the same.

Specific examples of optionally substituted alkyl groups of 1 to 4 carbon atoms as R^{b2}, R^{b3} or R^{b4} include methyl, ethyl, n-propyl, isopropyl, n-butyl, isobutyl and t-butyl groups. Of these, from the viewpoint of availability and reactivity, R^{b2}, R^{b3} and R^{b4} may be methyl groups.

Specific examples of optionally substituted alkoxy groups of 1 to 4 carbon atoms as R^{b2}, R^{b3} or R^{b4} include methoxy, ethoxy, n-propoxy and n-butoxy groups. Of these, from the viewpoint of availability and reactivity, R^{b2}, R^{b3} and R^{b4} may be methoxy groups.

A halogen atom as R^{b2}, R^{b3} or R^{b4} may be a fluorine, chlorine, bromine or iodine atom. Of these, from the viewpoint of availability and reactivity, R^{b2}, R^{b3} and R^{b4} may be fluorine atoms.

In formulas (B1), (B1-a), (B1-b) and (B1-c), R^{b1} represents an optionally substituted alkoxy group of 1 to 4 carbon atoms, an optionally substituted alkenyl group of 2 to 4 carbon atoms, an optionally substituted alkynyl group of 2 to 4 carbon atoms, an optionally substituted aryloxy group, an optionally substituted alkenyloxy group of 2 to 6 carbon atoms, an optionally substituted alkynyloxy group of 2 to 4 carbon atoms or an optionally substituted amino group. Of these, R^{b1} may be an optionally substituted alkoxy group of 1 to 4 carbon atoms, an optionally substituted phenoxy group, an optionally substituted alkenyloxy group of 2 to 6 carbon atoms or an optionally substituted amino group, as this will allow formation of a SEI with excellent ionic conductivity. R^{b1} may be an alkenyloxy group of 2 to 6 carbon atoms, as this will promote formation of the SEI by polymerization of allyl groups on the negative electrode, and will allow formation of a satisfactory SEI.

Specific examples of optionally substituted alkoxy groups of 1 to 4 carbon atoms as R^{b1} include methoxy, ethoxy, n-propoxy, n-butoxy, trifluoromethoxy, trifluoroethoxy, trichloromethoxy and optionally substituted benzyloxy groups. An optionally substituted benzyloxy group may be, for example, a benzyloxy, 2-methylbenzyloxy, 3-methylbenzyloxy, 4-methylbenzyloxy, 2-ethylbenzyloxy, 3-ethylbenzyloxy, 4-ethylbenzyloxy, 2-methoxybenzyloxy, 3-methoxybenzyloxy, 4-methoxybenzyloxy, 2-ethoxybenzyloxy, 3-ethoxybenzyloxy, 4-ethoxybenzyloxy, 2-(dimethylamino)benzyloxy, 3 -(dimethylamino)benzyloxy, 4-(dimethylamino)benzyloxy, 2-fluorobenzyloxy, 3-fluorobenzyloxy, 4-fluorobenzyloxy, 2-chlorobenzyloxy, 3-chlorobenzyloxy, 4-chlorobenzyloxy, 2-bromobenzyloxy, 3-bromobenzyloxy or 4-bromobenzyloxy group.

Specific examples of optionally substituted alkenyl groups of 2 to 4 carbon atoms as R^{b1} include vinyl, allyl, isopropenyl, 1-butenyl, 2-butenyl, 3-butenyl, isobutenyl and 1,1-difluoro-1-propynyl groups. Of these, R^{b1} may be an allyl group.

Specific examples of optionally substituted alkynyl groups of 2 to 4 carbon atoms as R^{b1} include ethynyl, 1-propynyl, 2-propynyl, 1-butynyl, 2-butynyl and 3-butynyl groups.

Specific examples of optionally substituted aryloxy groups as R^{b1} include phenoxy, 2-methylphenoxy, 3-methylphenoxy, 4-methylphenoxy, 2-ethylphenoxy, 3-ethylphenoxy, 4-ethylphenoxy, 2-methoxyphenoxy, 3-methoxyphenoxy, 4-methoxyphenoxy, 2-ethoxyphenoxy, 3-ethoxyphenoxy, 4-ethoxyphenoxy, 2-(dimethylamino)phenoxy, 3 -(dimethylamino)phenoxy, 4-(dimethylamino)phenoxy, 2-fluorophenoxy, 3-fluorophenoxy, 4-fluorophenoxy, 2-chlorophenoxy, 3-chlorophenoxy, 4-chlorophenoxy, 2-bromophenoxy, 3-bromophenoxy and 4-bromophenoxy groups.

Specific examples of optionally substituted alkenyloxy groups of 2 to 6 carbon atoms as R^{b1} include 2-propenyloxy, 1-methyl-2-propenyloxy, 2-methyl-2-propenyloxy, 2-butenyloxy, 3-butenyloxy, 2-hexenyloxy and 5-hexenyloxy groups.

Specific examples of optionally substituted alkynyloxy groups of 2 to 4 carbon atoms as R^{b1} include ethynyloxy, 1-propynyloxy, 2-propynyloxy, 1-butynyloxy, 2-butynyloxy and 3-butynyloxy groups.

An optionally substituted amino group as R^{b1} may be represented by the following formula (B10), for example.

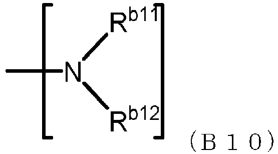

In formula (B10), R^{b11} and R^{b12} each independently represent a hydrogen atom, an optionally substituted alkyl group of 1 to 4 carbon atoms, an optionally substituted alkenyl group of 2 to 4 carbon atoms, an optionally substituted aryl group or an optionally substituted heteroaryl group, or R^{b11} and R^{b12} may be bonded together to form a divalent group.

Specific examples of optionally substituted alkyl groups of 1 to 4 carbon atoms as R^{b11} or R^{b12} include methyl, ethyl, n-propyl, isopropyl, n-butyl, isobutyl, t-butyl, trifluoromethyl, 1,1-dichloroethyl and optionally substituted benzyl groups. An optionally substituted benzyl group may be, for example, a benzyl, 2-methylbenzyl, 3-methylbenzyl, 4-methylbenzyl, 2-ethylbenzyl, 3-ethylbenzyl, 4-ethylbenzyl, 2-methoxybenzyl, 3-methoxybenzyl, 4-methoxybenzyl, 2-ethoxybenzyl, 3-ethoxybenzyl, 4-ethoxybenzyl, 2-(dimethylamino)benzyl, 3 -(dimethylamino)benzyl, 4-(dimethylamino)benzyl, 2-fluorobenzyl, 3-fluorobenzyl, 4-fluorobenzyl, 2-chlorobenzyl, 3-chlorobenzyl, 4-chlorobenzyl, 2-bromobenzyl, 3-bromobenzyl or 4-bromobenzyl group.

Specific examples of optionally substituted alkenyl groups of 2 to 4 carbon atoms as R^{b11} or R^{b12} include vinyl, allyl, isopropenyl, 1-butenyl, 2-butenyl, 3-butenyl, isobutenyl and 1,1-difluoro-1-propenyl groups. Of these, allyl groups may be selected for R^{b11} and R^{b12}.

Specific examples of optionally substituted aryl groups as R^{b11} or R^{b12} include phenyl, 2-methylphenyl, 3-methylphenyl, 4-methylphenyl, 2-ethylphenyl, 3-ethylphenyl, 4-ethylphenyl, 2-methoxyphenyl, 3-methoxyphenyl, 4-methoxyphenyl, 2-ethoxyphenyl, 3-ethoxyphenyl, 4-ethoxyphenyl, 2-(dimethylamino)phenyl, 3 -(dimethylamino)phenyl, 4-(dimethylamino)phenyl, 2-fluorophenyl, 3-fluorophenyl, 4-fluorophenyl, 2-chlorophenyl, 3-chlorophenyl, 4-chlorophenyl, 2-bromophenyl, 3-bromophenyl, 4-bromophenyl and trifluoromethylphenyl groups. Of these, R^{b11} and R^{b12} may be phenyl, 2-fluorophenyl, 3-fluorophenyl or 4-fluorophenyl groups, as they exhibit low LUMO energy which is susceptible to electrochemical reduction.

Specific examples of amino groups represented by formula (B10) include dimethylamino, n-butylmethylamino, allylamino, trifluoromethylphenylamino and benzylamino groups.

When R^{b1}, R^{b2}, R^{b3}, R^{b4}, R^{b11} and R^{b12} are alkyl, alkenyl, alkoxy, aryl, heteroaryl, aryloxy, heteroaryloxy or alkenyloxy groups, the substituents that may be bonded to them are selected from among, for example, halogen atoms, amino groups, alkyl groups of 1 to 4 carbon atoms, alkenyl groups of 2 to 4 carbon atoms, alkoxy groups of 1 to 4 carbon atoms, aryl groups, heteroaryl groups, aryloxy groups, heteroaryloxy groups and alkenyloxy groups of 2 to 4 carbon atoms. When the substituent is a halogen atom, and particularly a fluorine atom, the LUMO energy will tend to be low.

A person skilled in the art can synthesize a compound of formula (B1) using available starting materials and combining common reactions. For example, a compound of formula (B1) can be synthesized by a method of reacting a cyclic imide compound with the corresponding sulfonyl halide compound.

The additive for a non-aqueous electrolyte solution according to another embodiment of the invention includes one or more kinds of compounds represented by the following formula (C1).

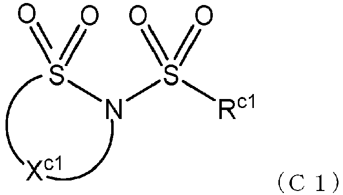

In formula (C1), X^{c1} represents a group that forms a cyclic group together with the sulfur atom and nitrogen atom. The cyclic group formed by X^{c1} may be substituted. The cyclic group may be a 4- to 6-membered ring, and it may be a fused ring comprising two or more rings. From the viewpoint of the effects on recycle properties and reducing gas generation, the cyclic group may include a sulfonyl group (-S(=O)₂-) and/or a carbonyl group (-C(=O)-), or it may include a sulfonyl or carbonyl group bonded to the nitrogen atom constituting the sulfonamide bond, as in the following formula (C2).

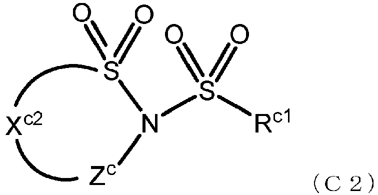

In formula (C2), x^{c2} represents a group that forms a cyclic group together with the sulfur atom, nitrogen atom and Z^{c}, and Z^{c} represents a sulfonyl or carbonyl group. The cyclic group formed by x^{c2} may be a 4- to 6-membered ring, and it may be a fused ring comprising two or more rings. R^{c1} has the same definition as R^{c1} in formula (C1).

Specific examples of compounds of formula (C2) include compounds represented by the following formula (C2-a), (C2-b) or (C2-c). These compounds provide a particularly excellent effect from the standpoint of minimizing gas generation.

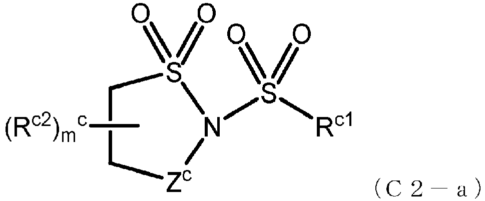

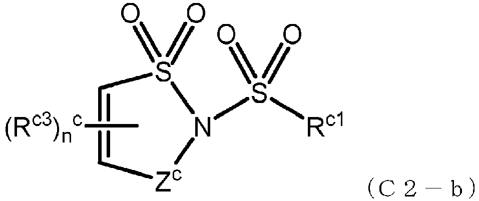

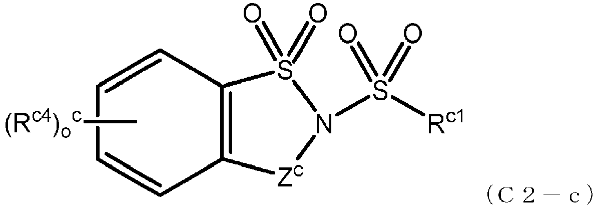

In formulas (C2-a), (C2-b) and (C2-c), R^{c2}, R^{c3} and R^{c4} each represent an optionally substituted alkyl group of 1 to 4 carbon atoms or an optionally substituted alkoxy group of 1 to 4 carbon atoms, a nitro, amino or sulfonyl group, or a halogen atom. m^{c} represents an integer of 0 to 4, n^{c} represents an integer of 0 to 2, and o^{c} represents an integer of 0 to 4. From the viewpoint of availability and reactivity, m^{c} may be 0 or 1, n^{c} may be 0 or 1 and o^{c} may be 0 or 1. R^{c1} has the same definition as R^{c1} in formula (C1). When m^{c}, n^{c} and o^{c} are 2 or greater, multiple R^{c2}, R^{c3} and R^{c4} may be the same or different, but they are preferably the same.

Specific examples of optionally substituted alkyl groups of 1 to 4 carbon atoms as R^{c2}, R^{c3} or R^{c4} include methyl, ethyl, n-propyl, isopropyl, n-butyl, isobutyl and t-butyl groups. Of these, from the viewpoint of availability and reactivity, R^{c2}, R^{c3} and R^{c4} may be methyl groups.

Specific examples of optionally substituted alkoxy groups of 1 to 4 carbon atoms as R^{c2}, R^{c3} or R^{c4} include methoxy, ethoxy, n-propoxy and n-butoxy groups. Of these, from the viewpoint of availability and reactivity, R^{c2}, R^{c3} and R^{c4} may be methoxy groups.

A halogen atom as R^{c2}, R^{c3} or R^{c4} may be a fluorine, chlorine, bromine or iodine atom. Of these, from the viewpoint of availability and reactivity, R^{c2}, R^{c3} and R^{c4} may be fluorine atoms.

R^{c1} in formulas (C1), (C2), (C2-a), (C2-b) and (C2-c) represents an optionally substituted alkyl group of 1 to 4 carbon atoms, an optionally substituted alkynyl group of 1 to 4 carbon atoms, an optionally substituted alkenyl group of 2 to 4 carbon atoms, an optionally substituted alkoxy group of 1 to 4 carbon atoms, an optionally substituted aryl group, an optionally substituted heteroaryl group, an optionally substituted aryloxy group, an optionally substituted heteroaryloxy group, an optionally substituted alkenyloxy group of 2 to 4 carbon atoms, an optionally substituted alkynyloxy group of 2 to 4 carbon atoms, or an optionally substituted amino group. Of these, R^{c1} may be an optionally substituted alkyl group of 1 to 4 carbon atoms, an optionally substituted alkoxy group of 1 to 4 carbon atoms, an optionally substituted phenoxy group, an optionally substituted alkenyloxy group of 2 to 4 carbon atoms or an optionally substituted amino group, as this will allow formation of a SEI with excellent ionic conductivity. R^{c1} may also be an alkenyloxy group of 2 to 4 carbon atoms, as this will promote formation of the SEI by polymerization of allyl groups on the negative electrode, and will allow formation of a satisfactory SEI. Of these, R^{c1} is preferably a methyl, trifluoromethyl, 2,2,2-trifluoroethyloxy, p-methylphenyloxy, dimethylamino, benzylmethylamino or allylamino group.

Specific examples of optionally substituted alkyl groups of 1 to 4 carbon atoms as R^{c1} include methyl, ethyl, n-propyl, isopropyl, n-butyl, isobutyl, t-butyl, trifluoromethyl, 1,1-dichloroethyl and optionally substituted benzyl groups. An optionally substituted benzyl group may be, for example, a benzyl, 2-methylbenzyl, 3-methylbenzyl, 4-methylbenzyl, 2-ethylbenzyl, 3-ethylbenzyl, 4-ethylbenzyl, 2-methoxybenzyl, 3-methoxybenzyl, 4-methoxybenzyl, 2-ethoxybenzyl, 3-ethoxybenzyl, 4-ethoxybenzyl, 2-(dimethylamino)benzyl, 3 -(dimethylamino)benzyl, 4-(dimethylamino)benzyl, 2-fluorobenzyl, 3-fluorobenzyl, 4-fluorobenzyl, 2-chlorobenzyl, 3-chlorobenzyl, 4-chlorobenzyl, 2-bromobenzyl, 3-bromobenzyl or 4-bromobenzyl group.

Specific examples of optionally substituted alkynyl groups of 2 to 4 carbon atoms as R^{c1} include ethynyl, 1-propynyl, 2-propynyl, 1-butynyl, 2-butynyl and 3-butynyl groups.

Specific examples of optionally substituted alkenyl groups of 2 to 4 carbon atoms as R^{c1} include vinyl, allyl, isopropenyl, 1-butenyl, 2-butenyl, 3-butenyl, isobutenyl and 1,1-difluoro-1-propenyl groups. Of these, R^{c1} may be an allyl group.

Specific examples of optionally substituted alkoxy groups of 1 to 4 carbon atoms as R^{c1} include methoxy, ethoxy, n-propoxy, n-butoxy, trifluoromethoxy, trichloromethoxy and optionally substituted benzyloxy groups. An optionally substituted benzyloxy group may be, for example, a benzyloxy, 2-methylbenzyloxy, 3-methylbenzyloxy, 4-methylbenzyloxy, 2-ethylbenzyloxy, 3-ethylbenzyloxy, 4-ethylbenzyloxy, 2-methoxybenzyloxy, 3-methoxybenzyloxy, 4-methoxybenzyloxy, 2-ethoxybenzyloxy, 3-ethoxybenzyloxy, 4-ethoxybenzyloxy, 2-(dimethylamino)benzyloxy, 3 -(dimethylamino)benzyloxy, 4-(dimethylamino)benzyloxy, 2-fluorobenzyloxy, 3-fluorobenzyloxy, 4-fluorobenzyloxy, 2-chlorobenzyloxy, 3-chlorobenzyloxy, 4-chlorobenzyloxy, 2-bromobenzyloxy, 3-bromobenzyloxy or 4-bromobenzyloxy group.

Specific examples of optionally substituted aryl groups or heteroaryl groups as R^{c1} include phenyl, 2-methylphenyl, 3-methylphenyl, 4-methylphenyl, 2-ethylphenyl, 3-ethylphenyl, 4-ethylphenyl, 2-methoxyphenyl, 3-methoxyphenyl, 4-methoxyphenyl, 2-ethoxyphenyl, 3-ethoxyphenyl, 4-ethoxyphenyl, 2-(dimethylamino)phenyl, 3 -(dimethylamino)phenyl, 4-(dimethylamino)phenyl, 2-fluorophenyl, 3-fluorophenyl, 4-fluorophenyl, 2-chlorophenyl, 3-chlorophenyl, 4-chlorophenyl, 2-bromophenyl, 3-bromophenyl, 4-bromophenyl, furyl, thienyl, pyridyl, oxazolyl, imidazolyl and quinolyl groups. Of these, R^{c1} may be phenyl, 2-fluorophenyl, 3-fluorophenyl or 4-fluorophenyl groups, as they exhibit low LUMO energy which is susceptible to electrochemical reduction.

Specific examples of optionally substituted aryloxy groups or heteroaryloxy groups as R^{c1} include phenoxy, 2-methylphenoxy, 3-methylphenoxy, 4-methylphenoxy, 2-ethylphenoxy, 3-ethylphenoxy, 4-ethylphenoxy, 2-methoxyphenoxy, 3-methoxyphenoxy, 4-methoxyphenoxy, 2-ethoxyphenoxy, 3-ethoxyphenoxy, 4-ethoxyphenoxy, 2-(dimethylamino)phenoxy, 3 -(dimethylamino)phenoxy, 4-(dimethylamino)phenoxy, 2-fluorophenoxy, 3-fluorophenoxy, 4-fluorophenoxy, 2-chlorophenoxy, 3-chlorophenoxy, 4-chlorophenoxy, 2-bromophenoxy, 3-bromophenoxy, 4-bromophenoxy, furyloxy, thienyloxy, pyridyloxy, oxazolyloxy, imidazolyloxy and quinolyloxy groups.

Specific examples of optionally substituted alkenyloxy groups of 2 to 4 carbon atoms as R^{c1} include 2-propenyloxy, 1-methyl-2-propenyloxy, 2-methyl-2-propenyloxy, 2-butenyloxy, 3-butenyloxy, 2-hexenyloxy and 5-hexenyloxy groups.

Specific examples of optionally substituted alkynyloxy groups of 2 to 4 carbon atoms as R^{c1} include ethynyloxy, 1-propynyloxy, 2-propynyloxy, 1-butynyloxy, 2-butynyloxy and 3-butynyloxy groups.

An optionally substituted amino group as R^{c1} may be represented by the following formula (C10), for example.

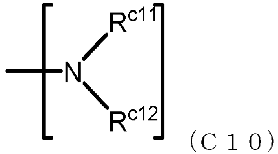

In formula (C10), R^{c11} and R^{c12} each independently represent a hydrogen atom, an optionally substituted alkyl group of 1 to 4 carbon atoms, an optionally substituted alkenyl group of 2 to 4 carbon atoms, an optionally substituted aryl group or an optionally substituted heteroaryl group, or R^{c11} and R^{c12} may be bonded together to form a divalent group.

Optionally substituted alkyl groups, optionally substituted alkenyl groups, optionally substituted aryl groups and optionally substituted heteroaryl groups as R^{c11} or R^{c12} may be the same ones mentioned as examples for R^{c1}. Specific examples of R^{c11} and R^{c12} include methyl, n-butyl, allyl, phenyl and benzyl groups. Specific examples of amino groups represented by formula (C10) include dimethylamino, n-butylmethylamino, allylamino, phenylmethylamino and benzylmethylamino groups.

When R^{c1}, R^{c2}, R^{c3}, R^{c4}, R^{c11} and R^{c12} are alkyl, alkenyl, alkynyl, alkoxy, aryl, heteroaryl, aryloxy, heteroaryloxy, alkenyloxyalkyl or alkynyloxy groups, the substituents that may be bonded to them are selected from among, for example, halogen atoms, amino groups, alkyl groups of 1 to 4 carbon atoms, alkenyl groups of 2 to 4 carbon atoms, alkoxy groups of 1 to 4 carbon atoms, aryl groups, heteroaryl groups, heteroaryl groups, aryloxy groups, heteroaryloxy groups and alkenyloxy groups of 2 to 4 carbon atoms. When the substituent is a halogen atom, and particularly a fluorine atom, the LUMO energy will tend to be low.

A person skilled in the art can synthesize a compound of formula (C1) using available starting materials and combining common reactions. For example, a compound of formula (C1) can be synthesized by a method of reacting a cyclic compound containing a sulfonamide bond, with the corresponding sulfonyl halide compound.

The additive for a non-aqueous electrolyte solution according to another embodiment of the invention includes one or more kinds of compounds represented by the following formula (D1).

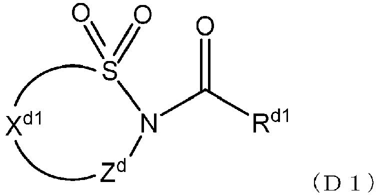

In formula (D1), X^{d1} represents a group that forms a cyclic group together with the sulfur atom, nitrogen atom and Z^{d}, and Z^{d} represents a sulfonyl or carbonyl group. The cyclic group formed by X^{d1} may be substituted. The cyclic group may be a 4- to 6-membered ring, and it may be a fused ring comprising two or more rings.

Specific examples of compounds of formula (D1) include compounds represented by the following formulas (D1-a), (D1-b) and (D1-c). These compounds provide particularly excellent effects from the standpoint of cycle characteristics and internal resistance.

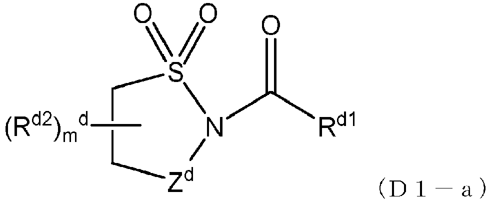

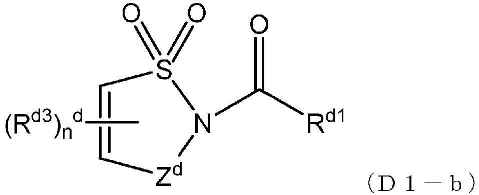

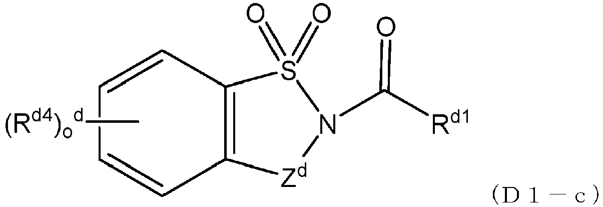

In formulas (D1-a), (D1-b) and (D1-c), R^{d2}, R^{d3} and R^{d4} each represent an optionally substituted alkyl group of 1 to 4 carbon atoms or an optionally substituted alkoxy group of 1 to 4 carbon atoms, a nitro, amino or sulfonyl group, or a halogen atom. Of these, from the viewpoint of availability and reactivity, R^{d2}, R^{d3} and R^{d4} may each be an optionally substituted alkyl group of 1 to 4 carbon atoms, an optionally substituted alkoxy group of 1 to 4 carbon atoms or a halogen atom.

m^{d} represents an integer of 0 to 4, n^{d} represents an integer of 0 to 2, and o^{d} represents an integer of 0 to 4. When m^{d}, n^{d} and o^{d} are each an integer of 2 or greater, multiple R^{d2}, R^{d3} and R^{d4} may be the same or different, but they are preferably the same.

From the viewpoint of availability and reactivity, m^{d} may be 0 or 1, n^{d} may be 0 or 1 and o^{d} may be 0 or 1. From the viewpoint of availability and reactivity, m^{d}, n^{d} and o^{d} may each be 0.

When o^{d} is 1, the location of substitution on R^{d4} may be the 4-position, for example. When o^{d} is 2, the location of substitution on R^{d4} may be the 4-position or 5-position, for example.

Specific examples of optionally substituted alkyl groups of 1 to 4 carbon atoms as R^{d2}, R^{d3} or R^{d4} include methyl, ethyl, n-propyl, isopropyl, n-butyl, isobutyl and t-butyl groups. Of these, from the viewpoint of availability and reactivity, R^{d2}, R^{d3} and R^{d4} may be methyl groups.

Specific examples of optionally substituted alkoxy groups of 1 to 4 carbon atoms as R^{d2}, R^{d3} or R^{d4} include methoxy, ethoxy, n-propoxy and n-butoxy groups. Of these, from the viewpoint of availability and reactivity, R^{d2}, R^{d3} and R^{d4} may be methoxy groups.

A halogen atom as R^{d2}, R^{d3} or R^{d4} may be a fluorine, chlorine, bromine or iodine atom. Of these, from the viewpoint of availability and reactivity, R^{d2}, R^{d3} and R^{d4} may be fluorine atoms.

Each R^{d1} in formulas (D1), (D1-a), (D1-b) and (D1-c) represents an optionally substituted alkyl group of 1 to 4 carbon atoms, an optionally substituted alkenyl group of 2 to 4 carbon atoms, an optionally substituted alkoxy group of 1 to 4 carbon atoms, an optionally substituted aryl group, an optionally substituted heteroaryl group, an optionally substituted aryloxy group, an optionally substituted heteroaryloxy group, an optionally substituted alkenyloxy group of 2 to 4 carbon atoms or an optionally substituted amino group. Of these, R^{d1} may be an optionally substituted alkoxy group of 1 to 4 carbon atoms, an optionally substituted aryloxy group, an optionally substituted heteroaryloxy group, an optionally substituted alkenyloxy group of 2 to 4 carbon atoms, or an optionally substituted amino group, as this will allow formation of a SEI with excellent ionic conductivity.

Other forms of the additive for a non-aqueous electrolyte solution according to the present embodiment includes one or more kinds of compounds represented by the following formula (D2).

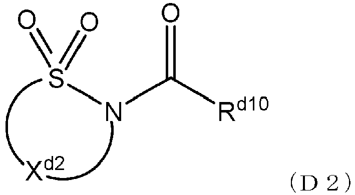

In formula (D2), X^{d2} represents a group that forms a cyclic group together with the sulfur atom and nitrogen atom. The cyclic group formed by X^{d2} may be substituted. The cyclic group may be a 4- to 6-membered ring, and it may be a fused ring comprising two or more rings.

In formula (D2), R^{d10} represents an optionally substituted alkenyl group of 2 to 4 carbon atoms, an optionally substituted alkoxy group of 1 to 4 carbon atoms, an optionally substituted aryl group, an optionally substituted heteroaryl group, an optionally substituted aryloxy group, an optionally substituted heteroaryloxy group, an optionally substituted alkenyloxy group of 2 to 4 carbon atoms or an optionally substituted amino group.

Specific examples of optionally substituted alkyl groups of 1 to 4 carbon atoms as R^{d1} include methyl, ethyl, n-propyl, isopropyl, n-butyl, isobutyl, t-butyl, trifluoromethyl, 1,1-dichloroethyl and optionally substituted benzyl groups. An optionally substituted benzyl group may be, for example, a benzyl, 2-methylbenzyl, 3-methylbenzyl, 4-methylbenzyl, 2-ethylbenzyl, 3-ethylbenzyl, 4-ethylbenzyl, 2-methoxybenzyl, 3-methoxybenzyl, 4-methoxybenzyl, 2-ethoxybenzyl, 3-ethoxybenzyl, 4-ethoxybenzyl, 2-(dimethylamino)benzyl, 3 -(dimethylamino)benzyl, 4-(dimethylamino)benzyl, 2-fluorobenzyl, 3-fluorobenzyl, 4-fluorobenzyl, 2-chlorobenzyl, 3-chlorobenzyl, 4-chlorobenzyl, 2-bromobenzyl, 3-bromobenzyl or 4-bromobenzyl group.

Specific examples of optionally substituted alkenyl groups of 2 to 4 carbon atoms as R^{d1} and R^{d10} include vinyl, allyl, isopropenyl, 1-butenyl, 2-butenyl, 3-butenyl, isobutenyl and 1,1-difluoro-1-propynyl groups. Of these, R^{d1} and R^{d10} may be allyl groups.

Specific examples of optionally substituted alkoxy groups of 1 to 4 carbon atoms as R^{d1} and R^{d10} include methoxy, ethoxy, n-propoxy, isopropoxy, n-butoxy, trifluoromethoxy, trichloromethoxy and optionally substituted benzyloxy groups. An optionally substituted benzyloxy group may be, for example, a benzyloxy, 2-methylbenzyloxy, 3-methylbenzyloxy, 4-methylbenzyloxy, 2-ethylbenzyloxy, 3-ethylbenzyloxy, 4-ethylbenzyloxy, 2-methoxybenzyloxy, 3-methoxybenzyloxy, 4-methoxybenzyloxy, 2-ethoxybenzyloxy, 3-ethoxybenzyloxy, 4-ethoxybenzyloxy, 2-(dimethylamino)benzyloxy, 3 -(dimethylamino)benzyloxy, 4-(dimethylamino)benzyloxy, 2-fluorobenzyloxy, 3-fluorobenzyloxy, 4-fluorobenzyloxy, 2-chlorobenzyloxy, 3-chlorobenzyloxy, 4-chlorobenzyloxy, 2-bromobenzyloxy, 3-bromobenzyloxy or 4-bromobenzyloxy group.

Specific examples of optionally substituted aryl groups or heteroaryl groups as R^{d1} and R^{d10} include phenyl, 2-methylphenyl, 3-methylphenyl, 4-methylphenyl, 2-ethylphenyl, 3-ethylphenyl, 4-ethylphenyl, 2-methoxyphenyl, 3-methoxyphenyl, 4-methoxyphenyl, 2-ethoxyphenyl, 3-ethoxyphenyl, 4-ethoxyphenyl, 2-(dimethylamino)phenyl, 3 -(dimethylamino)phenyl, 4-(dimethylamino)phenyl, 2-fluorophenyl, 3-fluorophenyl, 4-fluorophenyl, 2-chlorophenyl, 3-chlorophenyl, 4-chlorophenyl, 2-bromophenyl, 3-bromophenyl, 4-bromophenyl, furyl, thienyl, pyridyl, oxazolyl, imidazolyl and quinolyl groups. Of these, R^{d1} and R^{d10} may be phenyl, 2-fluorophenyl, 3-fluorophenyl or 4-fluorophenyl groups, as they exhibit low LUMO energy which is susceptible to electrochemical reduction.

Specific examples of optionally substituted aryloxy groups or heteroaryloxy groups as R^{d1} and R^{d10} include phenoxy, 2-methylphenoxy, 3-methylphenoxy, 4-methylphenoxy, 2-ethylphenoxy, 3-ethylphenoxy, 4-ethylphenoxy, 2-methoxyphenoxy, 3-methoxyphenoxy, 4-methoxyphenoxy, 2-ethoxyphenoxy, 3-ethoxyphenoxy, 4-ethoxyphenoxy, 2-(dimethylamino)phenoxy, 3 -(dimethylamino)phenoxy, 4-(dimethylamino)phenoxy, 2-fluorophenoxy, 3-fluorophenoxy, 4-fluorophenoxy, 2-chlorophenoxy, 3-chlorophenoxy, 4-chlorophenoxy, 2-bromophenoxy, 3-bromophenoxy, 4-bromophenoxy, furyloxy, thienyloxy, pyridyloxy, oxazolyloxy, imidazolyloxy and quinolyloxy groups.

Specific examples of optionally substituted alkenyloxy groups as R^{d1} and R^{d10} include 2-propenyloxy, 1-methyl-2-propenyloxy, 2-methyl-2-propenyloxy, 2-butenyloxy, 3-butenyloxy, 2-hexenyloxy and 5-hexenyloxy groups.

Optionally substituted amino groups as R^{d1} and R^{d10} may be represented by the following formula (D10), for example.

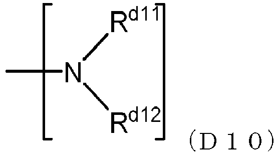

In formula (D10), R^{d11} and R^{d12} each independently represent a hydrogen atom, an optionally substituted alkyl group of 1 to 4 carbon atoms, an optionally substituted alkenyl group of 2 to 4 carbon atoms, an optionally substituted aryl group or an optionally substituted heteroaryl group, or R^{d11} and R^{d12} may be bonded together to form a divalent group.

Optionally substituted alkyl groups, optionally substituted alkenyl groups, optionally substituted aryl groups and optionally substituted heteroaryl groups as R^{d11} or R^{d12} may be the same ones mentioned as examples for R^{d1}. Specific examples of R^{d11} and R^{d12} include methyl, ethyl, n-butyl, allyl, phenyl and benzyl groups. Specific examples of amino groups represented by formula (D10) include dimethylamino, diethylamino, n-butylmethylamino, allylamino, diphenylamino and dibenzylamino groups.

When R^{d1}, R^{d2}, R^{d3}, R^{d4}, R^{d10}, R^{d11} and R^{d12} are alkyl, alkenyl, alkoxy, aryl, heteroaryl, aryloxy, heteroaryloxy or alkenyloxy groups, the substituents that may be bonded to them are selected from among, for example, halogen atoms, amino groups, alkyl groups of 1 to 4 carbon atoms, alkenyl groups of 2 to 4 carbon atoms, alkoxy groups of 1 to 4 carbon atoms, aryl groups, heteroaryl groups, heteroaryl groups, aryloxy groups, heteroaryloxy groups and alkenyloxy groups of 2 to 4 carbon atoms. When the substituent is a halogen atom, and particularly a fluorine atom, the LUMO energy will tend to be low.

When R^{d1}, R^{d2}, R^{d3}, R^{d4}, R^{d10}, R^{d11} and R^{d12} are benzyl or benzyloxy groups, the substituents that may be bonded to them are selected from among, for example, halogen atoms, amino groups, alkyl groups of 1 to 4 carbon atoms, alkenyl groups of 2 to 4 carbon atoms, alkoxy groups of 1 to 4 carbon atoms, aryl groups, heteroaryl groups, heteroaryl groups, aryloxy groups, heteroaryloxy groups and alkenyloxy groups of 2 to 4 carbon atoms. When the substituent is a halogen atom, and particularly a fluorine atom, the LUMO energy will tend to be low.

For formula (D1-c), specific examples of compounds wherein Z^{d} is a carbonyl group, R^{d1} is an optionally substituted alkyl group of 1 to 4 carbon atoms, an optionally substituted alkenyl group of 2 to 4 carbon atoms, an optionally substituted alkoxy group of 1 to 4 carbon atoms, an optionally substituted aryl group, an optionally substituted heteroaryl group, an optionally substituted aryloxy group, an optionally substituted heteroaryloxy group or an optionally substituted alkenyloxy group of 2 to 4 carbon atoms, R^{d4} is an optionally substituted alkyl group of 1 to 4 carbon atoms, an optionally substituted alkoxy group of 1 to 4 carbon atoms or a halogen atom, and o^{d} is an integer of 0 to 4, include N-benzoylsaccharin, N-phenoxycarbonylsaccharin, N-acetylsaccharin, N-methoxycarbonylsaccharin, N-propanoylsaccharin, N-ethoxycarbonylsaccharin, N-butanoylsaccharin, N-propoxycarbonylsaccharin, 2-benzoyl-4-methylsaccharin, 2-phenoxycarbonyl-4-methylsaccharin, 2-acetyl-4-methylsaccharin, 2-methoxycarbonyl-4-methylsaccharin, 2-propanoyl-4-methylsaccharin, 2-ethoxycarbonyl-4-methylsaccharin, 2-butanoyl-4-methylsaccharin, 2-propoxycarbonyl-4-methylsaccharin, 2-benzoyl-5-methylsaccharin, 2-phenoxycarbonyl-5-methylsaccharin, 2-acetyl-5-methylsaccharin, 2-methoxycarbonyl-5-methylsaccharin, 2-propanoyl-5-methylsaccharin, 2-ethoxycarbonyl-5-methylsaccharin, 2-butanoyl-5-methylsaccharin and 2-propoxycarbonyl-5-methylsaccharin.

A person skilled in the art can synthesize a compound of formula (D1) or formula (D2) using available starting materials and combining common reactions. For example, a compound of formula (D1) or formula (D2) can be synthesized by a method of reacting a halide with the corresponding cyclic compound.

The following are specific examples of compounds of formula (D1-c) wherein o^{d} is 0, Z^{d} is a carbonyl group and R^{d1} is a phenoxy group (N-phenoxycarbonylsaccharins). First, saccharin and triethylamine are dissolved in an organic solvent, and then phenyl chloroformate is added dropwise and the mixture is stirred at room temperature for 2 hours. Next, the obtained reaction product may be rinsed with water, crystallized and filtered to obtain the target compound.

The additive for a nonaqueous electrolyte solution according to another embodiment of the invention includes one or more kinds compounds represented by the following formula (E1) or formula (E2).

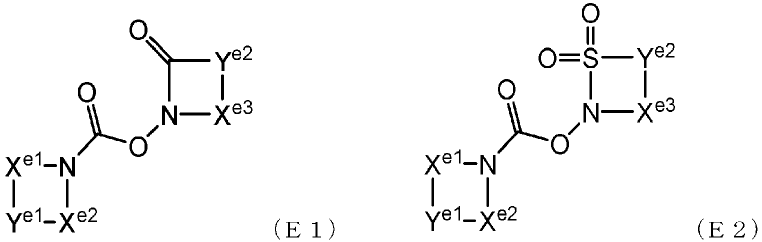

In formula (E1) or formula (E2), X^{e1}, X^{e2} and X^{e3} each independently represent an optionally substituted methylene, sulfonyl or carbonyl group. In formula (E1) or formula (E2), Y^{e1} and Y^{e2} each independently represent an optionally substituted hydrocarbon group of 1 to 6 carbon atoms. In formula (E1), X^{e3} may be a methylene or carbonyl group, and in formula (E2), X^{e3} may be a methylene, sulfonyl or carbonyl group.

Among the compounds represented by formula (E1) or formula (E2), X^{e1}, X^{e2} and X^{e3} may each independently be a sulfonyl or carbonyl group. In this case, the compound represented by formula (E1) or formula (E2) can form a SEI with low resistance, by having two ring-openable polymerization sites, and containing a sulfonyl group or carbonyl group. Thus, a compound represented by formula (E1) or formula (E2) will more easily exhibit effects of further improving cell characteristics and minimizing generation of gas.

In formula (E1) or formula (E2), optionally substituted hydrocarbon groups of 1 to 6 carbon atoms as Y^{e1} and Y^{e2} may include a multiple bond, and may be cyclic. Such optionally substituted hydrocarbon groups of 1 to 6 carbon atoms include -CH₂CH₂-, -CHFCHF-, -CH₂CH₂CH₂-, -CH=CH- and -C₆H₄-.

The compound represented by formula (E1) or formula (E2) may be a compound represented by formula (E3-a), formula (E3-b), formula (E3-c) or formula (E3-d), for example, from the viewpoint of availability and reactivity.

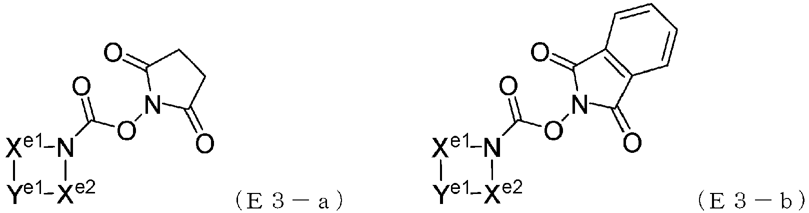

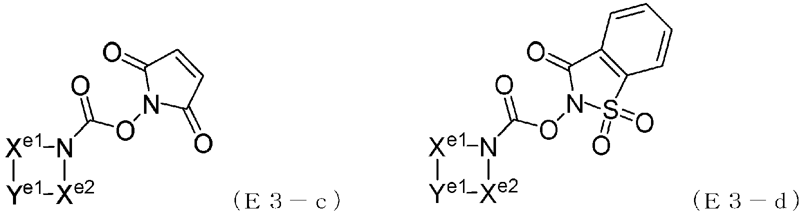

In formula (E3-a), formula (E3-b), formula (E3-c) and formula (E3-d), X^{e1}, X^{e2} and Y^{e1} have the same definitions as for X^{e1}, X^{e2} and Y^{e1} in formula (E1) or formula (E2).

Compounds represented by formula (E1) or formula (E2) include pthalimido-1-carboxylic acid-2,5-dioxopyrrolidine, 2,5-pyrroledione-1-carboxylic acid-2,5-dioxopyrrolidine, 2,5-dioxopyrrolidine-1-carboxylic acid-2,5-dioxopyrrolidine, o-benzsulfonimido-1-carboxylic acid-2,5-dioxopyrrolidine, benzimido-1-carboxylic acid pthalimide, 2,5-pyrroledione-1-carboxylic acid pthalimide, 2,5-dioxopyrrolidine-1-carboxylic acid pthalimide and o-benzsulfonimido-1-carboxylic acid phthalimide.

A person skilled in the art can synthesize a compound of formula (E1) or formula (E2) using available starting materials and combining common reactions. For example, a compound of formula (E1) or formula (E2) can be synthesized by a method of reacting a cyclic imide compound with the corresponding chloroformic acid ester in the presence of a base.

The additive for a nonaqueous electrolyte solution according to another embodiment of the invention includes one or more kinds of compounds represented by the following formula (F1).

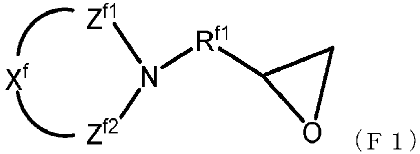

In formula (F1), X^{f} represents a group that forms a cyclic group together with the nitrogen atom, Z^{f1} and Z^{f2}, and Z^{f1} and Z^{f2} each independently represent a sulfonyl group (-S(=O)₂-) or a carbonyl group (-C(=O)-). The cyclic group formed by X^{f} may be substituted. The cyclic group may be a 4- to 6-membered ring, and it may be a fused ring comprising two or more rings.

In formula (F1), R^{f1} represents a divalent linker group. Specific examples of the divalent linker group include optionally substituted alkylene groups of 1 to 4 carbon atoms, divalent organic groups of 1 to 4 carbon atoms with ester bonds, divalent organic groups of 1 to 4 carbon atoms with ether bonds, and divalent organic groups of 1 to 4 carbon atoms with unsaturated bonds. Of these, from the viewpoint of availability and reactivity, R^{f1} may be an optionally substituted alkylene group of 1 to 4 carbon atoms, and it may be a methylene group.

Specific examples of compounds of formula (F1) include compounds represented by the following formulas (F1-a), (F1-b), (F1-c) or (F1-d). These compounds provide particularly excellent effects from the standpoint of cycle characteristics.

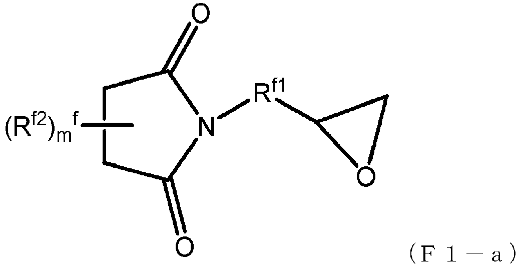

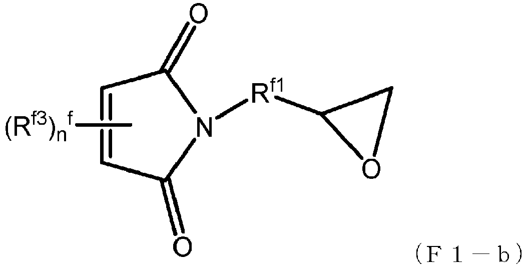

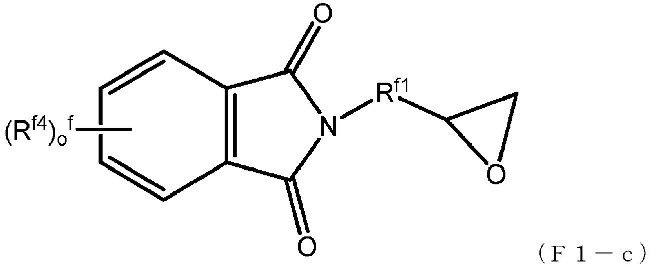

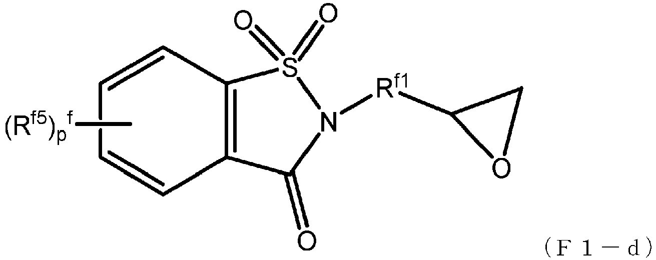

In formula (F1), R^{f1} represents a divalent linker group. In formula (F1-a), formula (F1-b), formula (F1-c)and formula (F1-d), R^{f1} may be the same as described for R^{f1} in formula (F1).
In formulas (F1-a), (F1-b), (F1-c) and (F1-d), R^{f2}, R^{f3}, R^{f4} and R^{f5} each represent an optionally substituted alkyl group of 1 to 4 carbon atoms, an optionally substituted alkoxy group of 1 to 4 carbon atoms, a nitro, amino or sulfonyl group or a halogen atom. In the case of multiple R^{f2}, R^{f3}, R^{f4} or R^{f5} groups, each of the substituents may be the same or different. m^{f} represents an integer of 0 to 2, n^{f} represents an integer of 0 to 2, o^{f} represents an integer of 0 to 4 and p^{f} represents an integer of 0 to 4. Of these, from the viewpoint of availability and reactivity, m^{f} may be 0 or 1, n^{f} may be 0 or 1, o^{f} may be 0 or 1 and p^{f} may be 0 or 1.

Specific examples of optionally substituted alkyl groups of 1 to 4 carbon atoms as R^{f2}, R^{f3}, R^{f4} or R^{f5} include methyl, ethyl, n-propyl, isopropyl, n-butyl, isobutyl and t-butyl groups. Of these, from the viewpoint of availability and reactivity, R^{f2}, R^{f3}, R^{f4} and R^{f5} may be methyl groups.

Specific examples of optionally substituted alkoxy groups of 1 to 4 carbon atoms as R^{f2}, R^{f3}, R^{f4} or R^{f5} include methoxy, ethoxy, n-propoxy, n-butoxy, trifluoromethoxy, 2,2,2-trifluoroethyloxy and 1,1,2,2,2-pentafluoroethyloxy groups. Of these, from the viewpoint of availability and reactivity, R^{f2}, R^{f3}, R^{f4} and R^{f5} may be methoxy groups.

A halogen atom as R^{f2}, R^{f3}, R^{f4} or R^{f5} may be a fluorine, chlorine, bromine or iodine atom. Of these, from the viewpoint of availability and reactivity, R^{f2}, R^{f3}, R^{f4} and R^{f5} may be a fluorine atom.

In a compound represented by formula (F1-c) or formula (F1-d), when o^{f} or p^{f} is 1, the position of R^{f4} or R^{f5} may be the 4-position. When o^{f} or p^{f} is 2, the positions of R^{f4} or R^{f5} may be the 4-position and 5-position.

Of the compounds of formula (F1), examples of compounds represented by formula (F1-a) include N-glycidyl succinimide, 3-methyl-N-glycidyl succinimide, 3-fluoro-N-glycidyl succinimide and 3,4-difluoro-N-glycidyl succinimide. Examples of compounds represented by formula (F1-b) include N-glycidylmaleimide, N-glycidyl-3-chloromaleimide, N-glycidyl-3-fluoromaleimide and N-glycidyl-3,4-dibromomaleimide. Examples of compounds represented by formula (F1-c) include N-glycidylphthalimide, N-glycidyl-4-methylphthalimide, N-glycidyl-4-aminophthalimide, N-glycidyl-4-nitrophthalimide, N-glycidyl-4-bromophthalimide and N-glycidyl-3,4,5,6-tetrachlorophthalimide. Compounds represented by formula (F1-d) include N-glycidylsaccharin, N-glycidyl-4-methylsaccharin, N-glycidyl-4-methoxysaccharin, N-glycidyl-5-bromosaccharin and N-glycidyl-5-chlorosaccharin.

A person skilled in the art can synthesize a compound of formula (F1) using available starting materials and combining common reactions. For example, a compound of formula (F1) can be synthesized by a method of reacting a halide with the corresponding cyclic imide compound.

A specific example of producing a compound of formula (F1-c) wherein o^{f} is 0 and R^{f1} is methylene (N-glycidylphthalimide) will now be described. First, phthalimide and triethylamine are dissolved in an organic solvent, and then epichlorhydrin is added dropwise and the mixture is stirred at room temperature for 2 hours. Next, the obtained reaction product may be rinsed with water, crystallized and filtered to obtain the target compound.

The compounds represented by formulas (A1), (A2), (B1), (C1), (D1), (D2), (E1), (E2) or (F1) exhibit low LUMO energy that is susceptible to electrochemical reduction, and therefore when used in power storage devices such as non-aqueous electrolyte solution secondary batteries, non-aqueous electrolyte solutions containing them as additives for a non-aqueous electrolyte solution can improve cell characteristics such as the cycle characteristic, charge-discharge capacity and internal resistance by forming stable SEI on the electrode surfaces. Furthermore, compounds represented by formulas (A1), (A2), (B1), (C1), (D1), (D2), (E1), (E2) or (F1) are stable against moisture and temperature change, and therefore additives for a non-aqueous electrolyte solution and non-aqueous electrolyte solutions that contain them can be stored for prolonged periods of time at room temperature.

The lowest unoccupied molecular orbital (LUMO) energy of a compound represented by formula (A1), (A2), (B1), (C1), (D1), (D2), (E1), (E2) or (F1) may be -3.0 eV or greater and may be up to 0.0 eV. If the LUMO energy is -3.0 eV or greater it will be easy to avoid formation of a SEI with high resistance on the negative electrode by excessive decomposition of the compound. If the LUMO energy is 0.0 eV or lower, it will be possible to more easily form a more stable SEI on the negative electrode surface. From the same viewpoint, the LUMO energy may be -2.9 eV or greater and may be -0.5 eV or lower. A person skilled in the art can, without excessive trial and error, find compounds that exhibit LUMO energy within these numerical ranges, among the compounds defined by formulas (A1), (A2), (B1), (C1), (D1), (D2), (E1), (E2) or (F1).

Throughout the present specification, the "lowest unoccupied molecular orbital (LUMO) energy" is the value calculated by combining the PM3 method, as a semiempirical molecular orbital calculation method, with the B3LYP method, as a density functional method. Specifically, the LUMO energy may be calculated using Gaussian 03 (Revision B.03 software by U.S. Gaussian).

The additive for a non-aqueous electrolyte solution according to the present embodiment may also include other components in addition to a compound represented by formula (A1), (A2), (B1), (C1), (D1), (D2), (E1), (E2) or (F1), such as compounds that can contribute to SEI formation. Alternatively, the compound represented by formula (A1), (A2), (B1), (C1), (D1), (D2), (E1), (E2) or (F1) itself may be used as the additive for a non-aqueous electrolyte solution. The additive for a non-aqueous electrolyte solution according to the present embodiment may also include other common components in ranges that do not interfere with the effect of the invention. Examples of such other common components include vinylene carbonate (VC), fluoroethylene carbonate (FEC), 1,3-propanesultone (PS), negative electrode protective agents, positive electrode protective agents, flame retardants and overcharge protection agents.

The non-aqueous electrolyte solution for the present embodiment contains the additive for a non-aqueous electrolyte solution, a non-aqueous solvent and an electrolyte. The content of the additive for a non-aqueous electrolyte solution (or the compound represented by formula (A1), (A2), (B1), (C1), (D1), (D2), (E1), (E2) or (F1)) in the non-aqueous electrolyte solution may be 0.005 mass% or greater, and it may be 10 mass% or lower, based on the total mass of the non-aqueous electrolyte solution. If the content is 0.005 mass% or greater, it will be easier to adequately form a stable SEI by electrochemical reaction on the electrode surface. If the content is 10 mass% or lower, the additive for a non-aqueous electrolyte solution can be more easily dissolved in the non-aqueous solvent. Furthermore, by avoiding excessive increase in the content of the additive for a non-aqueous electrolyte solution, it is possible to minimize viscosity increase of the non-aqueous electrolyte solution and to ensure mobility of ions more easily. If the mobility of ions is not adequately ensured, there will be a possibility that the conductivity of the non-aqueous electrolyte solution is not adequately ensured, and the charge-discharge characteristic of the power storage device may thereby be hindered. From the same viewpoint, the lower limit for the content of the additive for a non-aqueous electrolyte solution (or the compound represented by formula (A1), (A2), (B1), (C1), (D1), (D2), (E1), (E2) or (F1)) may be 0.01 mass%.

The non-aqueous electrolyte solution may include two or more kinds of additives for a non-aqueous electrolyte solution (two or more kinds of compounds that form a SEI). In this case, the total content of the additives for a non-aqueous electrolyte solution may be 0.005 mass% or greater and 10 mass% or lower, based on the total mass of the non-aqueous electrolyte solution. Examples of other additives include vinylene carbonate (VC), fluoroethylene carbonate (FEC) and 1,3-propanesultone (PS).

An aprotic solvent may be selected as the non-aqueous solvent from the viewpoint of maintaining a low viscosity of the obtained non-aqueous electrolyte solution. The aprotic solvent may be at least one selected from the group consisting of cyclic carbonates, straight-chain carbonates, aliphatic carboxylic acid esters, lactones, lactams, cyclic ethers, straight-chain ethers, sulfones, nitriles and their halogenated derivatives. Of these, a cyclic carbonate, and/or straight-chain carbonate may be selected as the aprotic solvent.

Examples of cyclic carbonates include ethylene carbonate, propylene carbonate and butylene carbonate. Examples of straight-chain carbonates include dimethyl carbonate, diethyl carbonate and ethylmethyl carbonate. Examples of aliphatic carboxylic acid esters include methyl acetate, ethyl acetate, methyl propionate, ethyl propionate, methyl butyrate, methyl isobutyrate and trimethyl methyl acetate. Examples of lactones include γ-butyrolactone. Examples of lactams include ε-caprolactam and N-methylpyrrolidone. Examples of cyclic ethers include tetrahydrofuran, 2-methyltetrahydrofuran, tetrahydropyran and 1,3-dioxolane. Examples of straight-chain ethers include 1,2-diethoxyethane and ethoxymethoxyethane. Examples of sulfones include sulfolane. Examples of nitriles include acetonitrile. Examples of halogenated derivatives include 4-fluoro-1,3-dioxolan-2-one, 4-chloro-1,3-dioxolan-2-one and 4,5-difluoro-1,3-dioxolan-2-one. These non-aqueous solvents may be used alone or in combinations of two or more. These non-aqueous solvents are particularly suitable for use in non-aqueous electrolyte solution secondary batteries such as lithium ion batteries, and electrical double layer capacitors such as lithium ion capacitors, for example.

The electrolyte in the non-aqueous electrolyte solution may be a lithium salt serving as an ion source of lithium ions. Of these, the electrolyte may be at least one selected from the group consisting of LiAlCl₄, LiBF₄, LiPF₆, LiClO₄, LiAsF₆ and LiSbF₆. LiBF₄ and/or LiPF₆ may be selected from the viewpoint of a high dissociation degree, increasing the ionic conductance of the electrolyte solution, and minimizing deterioration in the performance of power storage devices due to prolonged use, by oxidation-reduction resistance. These electrolytes may be used alone or in combinations of two or more. LiBF₄ and LiPF₆ may be combined with one or more each of a cyclic carbonate and a straight-chain carbonate, as non-aqueous solvents. In particular, LiBF₄ and/or LiPF₆ may be combined with ethylene carbonate and diethyl carbonate.

The concentration of electrolytes in the non-aqueous electrolyte solution may be 0.1 mol/L or greater, and it may be 2.0 mol/L or lower. If the electrolyte concentration is 0.1 mol/L or greater, it will be easier to adequately ensure the conductivity of the non-aqueous electrolyte solution. It will therefore be easier to obtain stable discharge characteristics and charge properties for power storage devices. If the electrolyte concentration is 2.0 mol/L or lower, increase in the non-aqueous electrolyte solution viscosity will be reduced and it will be particularly easier to ensure the mobility of ions. If the mobility of ions is not adequately ensured, there will be a possibility that the conductivity of the electrolyte solution is not adequately ensured, and the charge-discharge characteristic of the power storage device may thereby be hindered. From the same viewpoint, the electrolyte concentration may be 0.5 mol/L or greater, and it may be 1.5 mol/L or lower.

The power storage device of the present embodiment comprises mainly a non-aqueous electrolyte solution, and a positive electrode and negative electrode. Specific examples of power storage devices include non-aqueous electrolyte solution secondary batteries (such as lithium ion batteries) and electrical double layer capacitors (such as lithium ion capacitors). The non-aqueous electrolyte solution of the present embodiment is particularly effective for use in lithium ion batteries and lithium ion capacitors.

Fig. 1 is a cross-sectional view schematically showing an embodiment of a power storage device. The power storage device 1 shown in Fig. 1 is a non-aqueous electrolyte solution secondary battery. The power storage device 1 comprises a positive plate 4 (positive electrode), a negative plate 7 (negative electrode) opposing the positive plate 4, a non-aqueous electrolyte solution 8 disposed between the positive plate 4 and the negative plate 7, and a separator 9 provided in the non-aqueous electrolyte solution 8. The positive plate 4 has a positive electrode collector 2 and a positive electrode active material layer 3 provided on its non-aqueous electrolyte solution 8 side. The negative plate 7 has a negative electrode collector 5 and a negative electrode active material layer 6 provided on its non-aqueous electrolyte solution 8 side. The non-aqueous electrolyte solution 8 used may be the non-aqueous electrolyte solution of the embodiment described above. In Fig. 1, a non-aqueous electrolyte solution secondary battery is shown as the power storage device, but the power storage devices to which the non-aqueous electrolyte solution may be applied are not limited to the one shown, and they may be other power storage devices such as electrical double layer capacitors.

The positive electrode collector 2 and negative electrode collector 5 may each be a metal foil made of metal such as aluminum, copper, nickel or stainless steel.

The positive electrode active material layer 3 includes a positive electrode active material. The positive electrode active material may be a lithium-containing complex oxide. Specific examples of lithium-containing complex oxides include LiMnO₂, LiFeO₂, LiCoO₂, LiMn₂O₄, Li₂FeSiO₄, LiNi_{1/3}Co_{1/3}Mn_{1/3}O₂ and LiFePO₄.

The negative electrode active material layer 6 includes a negative electrode active material. The negative electrode active material may be, for example, a material that can occlude and release lithium. Specific examples of such materials include carbon materials such as graphite and amorphous carbon, and oxide materials such as indium oxide, silicon oxide, tin oxide, zinc oxide and lithium oxide. The negative electrode active material may also be lithium metal, or a metal material that can form an alloy with lithium. Specific examples of metals that can form alloys with lithium include Cu, Sn, Si, Co, Mn, Fe, Sb and Ag. A binary or ternary alloy comprising such a metal and lithium may be used as the negative electrode active material. These negative electrode active materials may be used alone or, two or more may be used in combination.

The separator 9 may be a porous film made of polyethylene, polypropylene, fluorine resin or the like, for example.

The specific forms, such as the shapes and thicknesses, of each of the members forming the power storage device may be appropriately determined by a person skilled in the art. The construction of the power storage device is not limited to the embodiment shown in Fig. 1 and may be modified as appropriate.

### EXAMPLES

The invention will now be explained in greater detail by the following examples, with the understanding that the invention is not restricted only to the examples.

### <Experimental Example A>

### 1. Preparation of non-aqueous electrolyte solution

### (Example A1)

Ethylene carbonate (EC) and diethyl carbonate (DEC) were mixed in a volume compositional ratio of EC:DEC = 30:70 to obtain a mixed non-aqueous solvent. In the obtained mixed non-aqueous solvent there was dissolved LiPF₆ as an electrolyte to a concentration of 1.0 mol/L. To the obtained solution there was added compound A1 shown in Table 1 as an additive for a non-aqueous electrolyte solution, to prepare a non-aqueous electrolyte solution. The content ratio of the additive for a non-aqueous electrolyte solution (compound A1) was 0.5 mass% with respect to the total mass of the non-aqueous electrolyte solution.

### (Example A2)

A non-aqueous electrolyte solution was prepared in the same manner as Example A1, except that the content ratio of compound A1 was 1.0 mass%.

### (Example A3)

A non-aqueous electrolyte solution was prepared in the same manner as Example A1, except that the additive for a non-aqueous electrolyte solution was changed from compound A1 to compound A2 shown in Table 1, and the content ratio was 1.0 mass%.

### (Example A4)

A non-aqueous electrolyte solution was prepared in the same manner as Example A1, except that the additive for a non-aqueous electrolyte solution was changed from compound A1 to compound A3 shown in Table 1, and the content ratio was 1.0 mass%.

### (Example A5)

A non-aqueous electrolyte solution was prepared in the same manner as Example A1, except that the additive for a non-aqueous electrolyte solution was changed from compound A1 to compound A4 shown in Table 1, and the content ratio was 1.0 mass%.

### (Example A6)

A non-aqueous electrolyte solution was prepared in the same manner as Example A1, except that the additive for a non-aqueous electrolyte solution was changed from compound A1 to compound A5 shown in Table 1, and the content ratio was 1.0 mass%.

### (Example A7)

A non-aqueous electrolyte solution was prepared in the same manner as Example A1, except that the additive for a non-aqueous electrolyte solution was changed from compound A1 to compound A6 shown in Table 1, and the content ratio was 1.0 mass%.

### (Example A8)

A non-aqueous electrolyte solution was prepared in the same manner as Example A1, except that the additive for a non-aqueous electrolyte solution was changed from compound A1 to compound A7 shown in Table 1, and the content ratio was 1.0 mass%.

### (Example A9)

A non-aqueous electrolyte solution was prepared in the same manner as Example A1, except that the additive for a non-aqueous electrolyte solution was changed from compound A1 to compound A8 shown in Table 1, and the content ratio was 1.0 mass%.

### (Comparative Example A1)

A non-aqueous electrolyte solution was prepared in the same manner as Example A1, except that compound A1 was not added.

### (Comparative Example A2)

A non-aqueous electrolyte solution was prepared in the same manner as Example A1, except that the additive for a non-aqueous electrolyte solution was changed from compound A1 to 1,3-propanesultone, and the content ratio was 1.0 mass%.

### (Comparative Example A3)

A non-aqueous electrolyte solution was prepared in the same manner as Example A1, except that the additive for a non-aqueous electrolyte solution was changed from compound A1 to vinylene carbonate (VC), and the content ratio was 1.0 mass%.

### (Comparative Example A4)

A non-aqueous electrolyte solution was prepared in the same manner as Comparative Example A3, except that the content ratio of the vinylene carbonate (VC) was 2.0 mass%.

### (Comparative Example A5)

A non-aqueous electrolyte solution was prepared in the same manner as Example A1, except that the additive for a non-aqueous electrolyte solution was changed from compound A1 to fluoroethylene carbonate (FEC), and the content ratio was 1.0 mass%.

### (Comparative Example A6)

A non-aqueous electrolyte solution was prepared in the same manner as Comparative Example A5, except that the content ratio of the fluoroethylene carbonate (FEC) was 2.0 mass%.

### (Comparative Example A7)

A non-aqueous electrolyte solution was prepared in the same manner as Example A1, except that the additive for a non-aqueous electrolyte solution was changed from compound A1 to phthalimide, and the content ratio was 1.0 mass%.

### (Comparative Example A8)

A non-aqueous electrolyte solution was prepared in the same manner as Example A1, except that the additive for a non-aqueous electrolyte solution was changed from compound A1 to maleimide, and the content ratio was 1.0 mass%.

### (Comparative Example A9)

A non-aqueous electrolyte solution was prepared in the same manner as Example A1, except that the additive for a non-aqueous electrolyte solution was changed from compound A1 to succinimide, and the content ratio was 1.0 mass%.

### (Comparative Example A10)

A non-aqueous electrolyte solution was prepared in the same manner as Example A1, except that the additive for a non-aqueous electrolyte solution was changed from compound A1 to saccharin, and the content ratio was 1.0 mass%.

### 2. Evaluation

### (Measurement of LUMO energy)

The LUMO (lowest unoccupied molecular orbital) energies of compounds A1, A2, A3, A4, A5, A6, A7 and A8 used in the Examples were determined by calculating the semiempirical molecular orbitals using Gaussian 03 software. The calculated LUMO energies are shown in Table 1.

**Table 1**

| | Compound structure | LUMO energy (eV) | | Compound structure | LUMO energy (eV) |
|---|---|---|---|---|---|
| Compo und A1 | 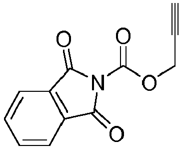 | -2.44 | Compo und A5 | 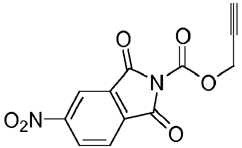 | -2.45 |
| Compo und A2 | 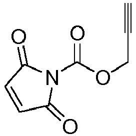 | -2.22 | Compo und A6 | 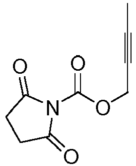 | -2.01 |
| Compo und A3 | 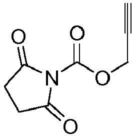 | -2.19 | Compo und A7 | 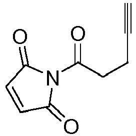 | -2.11 |
| Compo und A4 | 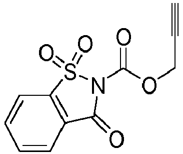 | -2.32 | Compo und A8 | 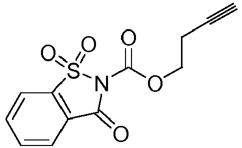 | -2.28 |

### (Stability)

The compounds A1, A2, A3, A4, A5, A6, A7 and A8 used in the examples and the fluoroethylene carbonate (FEC) used in Comparative Examples A5 and A6 were supplied for a preservation test in which they were allowed to stand for 90 days in a steady temperature and humidity environment at a temperature of 40 ±2°C and a humidity of 75 ±5%. The ¹H-nuclear magnetic resonance spectrum (¹H-NMR) of each additive for a non-aqueous electrolyte solution was measured before and after the preservation test, and the stability of each compound was evaluated based on the following scale. Table 2 shows the evaluation results for stability.
G: No change in ¹H-NMR spectrum peaks before and after preservation test.
F: Slight change in ¹H-NMR spectrum peaks before and after preservation test.
P: Distinct change in ¹H-NMR spectrum peaks before and after preservation test.

**Table 2**

| Additive | Stability |
|---|---|
| Compound A1 | G |
| Compound A2 | G |
| Compound A3 | G |
| Compound A4 | G |
| Compound A5 | G |
| Compound A6 | G |
| Compound A7 | G |
| Compound A8 | G |
| FEC | P |

As shown in Table 2, the fluoroethylene carbonate (FEC) used in Comparative Examples A5 and A6 was presumably partially hydrolyzed, and had inferior stability. Compounds A1, A2, A3, A4, A5, A6, A7 and A8 used in the examples, on the other hand, exhibited virtually no change and had excellent stability.

### (Fabrication of positive electrode)

LiMn₂O₄ as a positive electrode active material and carbon black as a conductivity imparting agent were dry blended. The obtained mixture was uniformly dispersed in N-methyl-2-pyrrolidone (NMP) dissolving polyvinylidene fluoride (PVDF) as a binder, to prepare a slurry. The obtained slurry was coated onto both sides of an aluminum metal foil (rectilinear, thickness: 20 µm). After drying the coating film to remove the NMP, the entire foil was pressed to obtain a positive electrode sheet having an aluminum metal foil as the positive electrode collector and a positive electrode active material layer formed on both sides of it. The solid ratio of the positive electrode active material layer of the obtained positive electrode sheet was positive electrode active material:conductivity imparting agent:PVDF = 90:5:5, as the mass ratio.

### (Preparation of negative electrode)

As a negative electrode sheet there was used a commercially available graphite-coated electrode sheet (product name: Electrode sheet negative electrode monolayer, by Hohsen Co.).

### (Fabrication of non-aqueous electrolyte solution secondary battery)

Cell elements were fabricated by laminating a negative electrode sheet, a polyethylene separator, a positive electrode sheet, a polyethylene separator and a negative electrode sheet, in that order, in the different non-aqueous electrolyte solutions obtained in the examples and comparative examples. Each cell element was inserted into a bag formed from a laminate film having a resin layer covering both sides of aluminum (thickness: 40 µm), with the positive electrode sheet and negative electrode sheet ends protruding out from the bag. Next, each of the non-aqueous electrolyte solutions obtained in the examples and comparative examples was infused into the bag. The bag was vacuum sealed to obtain a sheet-like non-aqueous electrolyte solution secondary battery. In order to increase the adhesiveness between the electrodes, the sheet-like non-aqueous electrolyte solution secondary battery was sandwiched between glass plates and pressed.

### (Evaluation of service capacity maintenance factor and internal resistance ratio)

Each of the obtained non-aqueous electrolyte solution secondary batteries was subjected to a charge-discharge cycle test at 25°C, with a charge rate of 0.3 C, a discharge rate of 0.3 C, a charge final voltage of 4.2 V and a discharge final voltage of 2.5 V. The service capacity maintenance factor (%) after 200 cycles and the internal resistance ratio after 200 cycles are shown in Table 3. The "service capacity maintenance factor (%)" after 200 cycles is the value obtained by dividing the service capacity (mAh) after a 200 cycle test by the service capacity (mAh) after a 10 cycle test, and multiplying by 100. Also, the "internal resistance ratio" after 200 cycles is represented by the relative value of the resistance after a 200 cycle test, where the resistance before the cycle test is defined as 1.

**Table 3**

| | Electrolyte | Solvent | Additive | Service capacity maintenan ce factor (%) | Internal resistanc e ratio |
|---|---|---|---|---|---|
| Ex. A1 | LiPF₆ 1.0 mol/L | EC/DEC (30/70) vol% | Compound A1 0.5 mass% | 91 | 1.30 |
| Ex. A2 | LiPF₆ 1.0 mol/L | EC/DEC (30/70) vol% | Compound A1 1.0 mass% | 93 | 1.38 |
| Ex. A3 | LiPF₆ 1.0 mol/L | EC/DEC (30/70) vol% | Compound A2 1.0 mass% | 92 | 1.42 |
| Ex. A4 | LiPF₆ 1.0 mol/L | EC/DEC (30/70) vol% | Compound A3 1.0 mass% | 94 | 1.40 |
| Ex. A5 | LiPF₆ 1.0 mol/L | EC/DEC (30/70) vol% | Compound A4 1.0 mass% | 94 | 1.44 |
| Ex. A6 | LiPF₆ 1.0 mol/L | EC/DEC (30/70) vol% | Compound A5 1.0 mass% | 93 | 1.38 |
| Ex. A7 | LiPF₆ 1.0 mol/L | EC/DEC (30/70) vol% | Compound A6 1.0 mass% | 93.2 | 1.44 |
| Ex. A8 | LiPF₆ 1.0 mol/L | EC/DEC (30/70) vol% | Compound A7 1.0 mass% | 92 | 1.43 |
| Ex. A9 | LiPF₆ 1.0 mol/L | EC/DEC (30/70) vol% | Compound A8 1.0 mass% | 92 | 1.44 |
| Comp. Ex. A1 | LiPF₆ 1.0 mol/L | EC/DEC (30/70) vol% | None | 79 | 1.83 |
| Comp. Ex. A2 | LiPF₆ 1.0 mol/L | EC/DEC (30/70) vol% | 1,3-Propanesultone 1.0 mass% | 81 | 1.68 |
| Comp. Ex. A3 | LiPF₆ 1.0 mol/L | EC/DEC (30/70) vol% | VC 1.0 mass% | 81 | 1.69 |
| Comp. Ex. A4 | LiPF₆ 1.0 mol/L | EC/DEC (30/70) vol% | VC 2.0 mass% | 82 | 1.53 |
| Comp. Ex. A5 | LiPF₆ 1.0 mol/L | EC/DEC (30/70) vol% | FEC 1.0 mass% | 84 | 1.66 |
| Comp. Ex. A6 | LiPF₆ 1.0 mol/L | EC/DEC (30/70) vol% | FEC 2.0 mass% | 86 | 1.67 |
| Comp. Ex. A7 | LiPF₆ 1.0 mol/L | EC/DEC (30/70) vol% | Phthalimide 1.0 mass% | 87 | 1.45 |
| Comp. Ex. A8 | LiPF₆ 1.0 mol/L | EC/DEC (30/70) vol% | Maleimide 1.0 mass% | 86 | 1.55 |
| Comp. Ex. A9 | LiPF₆ 1.0 mol/L | EC/DEC (30/70) vol% | Succinimide 1.0 mass% | 85 | 1.49 |
| Comp. Ex. A10 | LiPF₆ 1.0 mol/L | EC/DEC (30/70) vol% | Saccharin 1.0 mass% | 87 | 1.42 |

### (Measurement of gas generation volume)

Separately from the battery used for the cycle test, there was prepared a non-aqueous electrolyte solution secondary battery having the same construction, containing each of the electrolyte solutions of the examples and comparative examples. A procedure of charging each battery to 4.2 V with a current corresponding to 0.2 C and then discharging to 3 V with a current corresponding to 0.2 C, at 25°C, was repeated for 3 cycles to stabilize the battery. Next, charging was again carried out to 4.2 V with the charge rate at 0.3 C, after which the battery was stored at a high temperature of 60°C for 168 hours. The battery was then cooled to room temperature, the volume of the battery was measured by Archimedes' method, and the gas generation volume was determined from the volume change before and after storage.

**Table 4**

| | Electrolyte | Solvent | Additive | Gas generation volume (ml) |
|---|---|---|---|---|
| Ex. A10 | LiPF₆ 1.0 mol/L | EC/DEC (30/70) vol% | Compound A1 1.0 mass% | 0.34 |
| Ex. A11 | LiPF₆ 1.0 mol/L | EC/DEC (30/70) vol% | Compound A2 1.0 mass% | 0.29 |
| Ex. A12 | LiPF₆ 1.0 mol/L | EC/DEC (30/70) vol% | Compound A3 1.0 mass% | 0.28 |
| Ex. A13 | LiPF₆ 1.0 mol/L | EC/DEC (30/70) vol% | Compound A4 1.0 mass% | 0.27 |
| Ex. A14 | LiPF₆ 1.0 mol/L | EC/DEC (30/70) vol% | Compound A5 1.0 mass% | 0.28 |
| Ex. A15 | LiPF₆ 1.0 mol/L | EC/DEC (30/70) vol% | Compound A6 1.0 mass% | 0.30 |
| Ex. A16 | LiPF₆ 1.0 mol/L | EC/DEC (30/70) vol% | Compound A7 1.0 mass% | 0.31 |
| Ex. A17 | LiPF₆ 1.0 mol/L | EC/DEC (30/70) vol% | Compound A8 1.0 mass% | 0.32 |
| Comp. Ex. A11 | LiPF₆ 1.0 mol/L | EC/DEC (30/70) vol% | None | 0.54 |
| Comp. Ex. A12 | LiPF₆ 1.0 mol/L | EC/DEC (30/70) vol% | FEC 1.0 mass% | 0.58 |
| Comp. Ex. A13 | LiPF₆ 1.0 mol/L | EC/DEC (30/70) vol% | VC 1.0 mass% | 0.59 |

From Table 3 and Table 4 it is seen that the non-aqueous electrolyte solution secondary batteries using the non-aqueous electrolyte solutions of each of the examples which included compounds A1, A2, A3, 4A, 5A, 6A, 7A or 8A, as compounds of formula (A1) and formula (A2), were excellent in terms of both service capacity maintenance factor during the cycle test and minimizing gas generation with charging, compared to the non-aqueous electrolyte solution secondary batteries using the non-aqueous electrolyte solutions of the comparative examples. This strongly suggests that the compounds of formula (A1) or formula (A2), when used in non-aqueous electrolyte solution secondary batteries, form SEI that are stable against charge-discharge cycles and high temperature storage. It also confirmed that the compounds of formula (A1) or formula (A2) are also excellent in terms of low increase in internal resistance with charge-discharge cycling.

### <Experimental Example B>

### 1. Preparation of non-aqueous electrolyte solution

### (Example B1)

Ethylene carbonate (EC) and diethyl carbonate (DEC) were mixed in a volume ratio of EC:DEC = 30:70 to prepare a mixed non-aqueous solvent, and LiPF₆ as an electrolyte was dissolved in the mixture to a concentration of 1.0 mol/L. To the obtained solution there was added compound B1 shown in Table 5 as an additive for a non-aqueous electrolyte solution, to prepare a non-aqueous electrolyte solution. The content ratio of the additive for a non-aqueous electrolyte solution (compound B1) was 0.5 mass% with respect to the total mass of the non-aqueous electrolyte solution.

### (Example B2)

A non-aqueous electrolyte solution was prepared in the same manner as Example B1, except that the content ratio of compound B1 was 1.0 mass%.

### (Example B3)

A non-aqueous electrolyte solution was prepared in the same manner as Example B1, except that the additive for a non-aqueous electrolyte solution was changed from compound B1 to compound B2 shown in Table 5, and the content ratio was 1.0 mass%.

### (Example B4)

A non-aqueous electrolyte solution was prepared in the same manner as Example B1, except that the additive for a non-aqueous electrolyte solution was changed from compound B1 to compound B3 shown in Table 5, and the content ratio was 1.0 mass%.

### (Example B5)

A non-aqueous electrolyte solution was prepared in the same manner as Example B1, except that the additive for a non-aqueous electrolyte solution was changed from compound B1 to compound B4 shown in Table 5, and the content ratio was 1.0 mass%.

### (Example B6)

A non-aqueous electrolyte solution was prepared in the same manner as Example B1, except that the additive for a non-aqueous electrolyte solution was changed from compound B1 to compound B5 shown in Table 5, and the content ratio was 1.0 mass%.

### (Example B7)

A non-aqueous electrolyte solution was prepared in the same manner as Example B1, except that the additive for a non-aqueous electrolyte solution was changed from compound B1 to compound B6 shown in Table 5, and the content ratio was 1.0 mass%.

### (Example B8)

A non-aqueous electrolyte solution was prepared in the same manner as Example B1, except that the additive for a non-aqueous electrolyte solution was changed from compound B1 to compound B7 shown in Table 5, and the content ratio was 1.0 mass%.

### (Example B9)

A non-aqueous electrolyte solution was prepared in the same manner as Example B1, except that the additive for a non-aqueous electrolyte solution was changed from compound B1 to compound B8 shown in Table 5, and the content ratio was 1.0 mass%.

### (Example B10)

A non-aqueous electrolyte solution was prepared in the same manner as Example B1, except that the additive for a non-aqueous electrolyte solution was changed from compound B1 to compound B9 shown in Table 5, and the content ratio was 1.0 mass%.

### (Comparative Example B1)

A non-aqueous electrolyte solution was prepared in the same manner as Example B1, except that compound B1 was not added.

### (Comparative Example B2)

A non-aqueous electrolyte solution was prepared in the same manner as Example B1, except that the additive for a non-aqueous electrolyte solution was changed from compound B1 to 1,3-propanesultone, and the content ratio was 1.0 mass%.

### (Comparative Example B3)

A non-aqueous electrolyte solution was prepared in the same manner as Example B1, except that the additive for a non-aqueous electrolyte solution was changed from compound B1 to vinylene carbonate (VC), and the content ratio was 1.0 mass%.

### (Comparative Example B4)

A non-aqueous electrolyte solution was prepared in the same manner as Comparative Example B3, except that the content ratio of the vinylene carbonate (VC) was 2.0 mass%.

### (Comparative Example B5)

A non-aqueous electrolyte solution was prepared in the same manner as Example B1, except that the additive for a non-aqueous electrolyte solution was changed from compound B1 to fluoroethylene carbonate (FEC), and the content ratio was 1.0 mass%.

### (Comparative Example B6)

A non-aqueous electrolyte solution was prepared in the same manner as Comparative Example B5, except that the content ratio of the fluoroethylene carbonate (FEC) was 2.0 mass%.

### (Comparative Example B7)

A non-aqueous electrolyte solution was prepared in the same manner as Example B1, except that the additive for a non-aqueous electrolyte solution was changed from compound B1 to compound B10 shown in Table 5, and the content ratio was 1.0 mass%.

### (Comparative Example B8)

A non-aqueous electrolyte solution was prepared in the same manner as Example B1, except that the additive for a non-aqueous electrolyte solution was changed from compound B1 to compound B11 shown in Table 5, and the content ratio was 1.0 mass%.

### (Comparative Example B9)

A non-aqueous electrolyte solution was prepared in the same manner as Example B1, except that the additive for a non-aqueous electrolyte solution was changed from compound B1 to compound B12 shown in Table 5, and the content ratio was 1.0 mass%.

### 2. Evaluation

### (Calculation of LUMO energy)

The LUMO (lowest unoccupied molecular orbital) energies of compounds B1, B2, B3, B4, B5, B6, B7, B8 and B9 used in the Examples were determined by calculating the semiempirical molecular orbitals using Gaussian 03 software. The calculated LUMO energies are shown in Table 5.

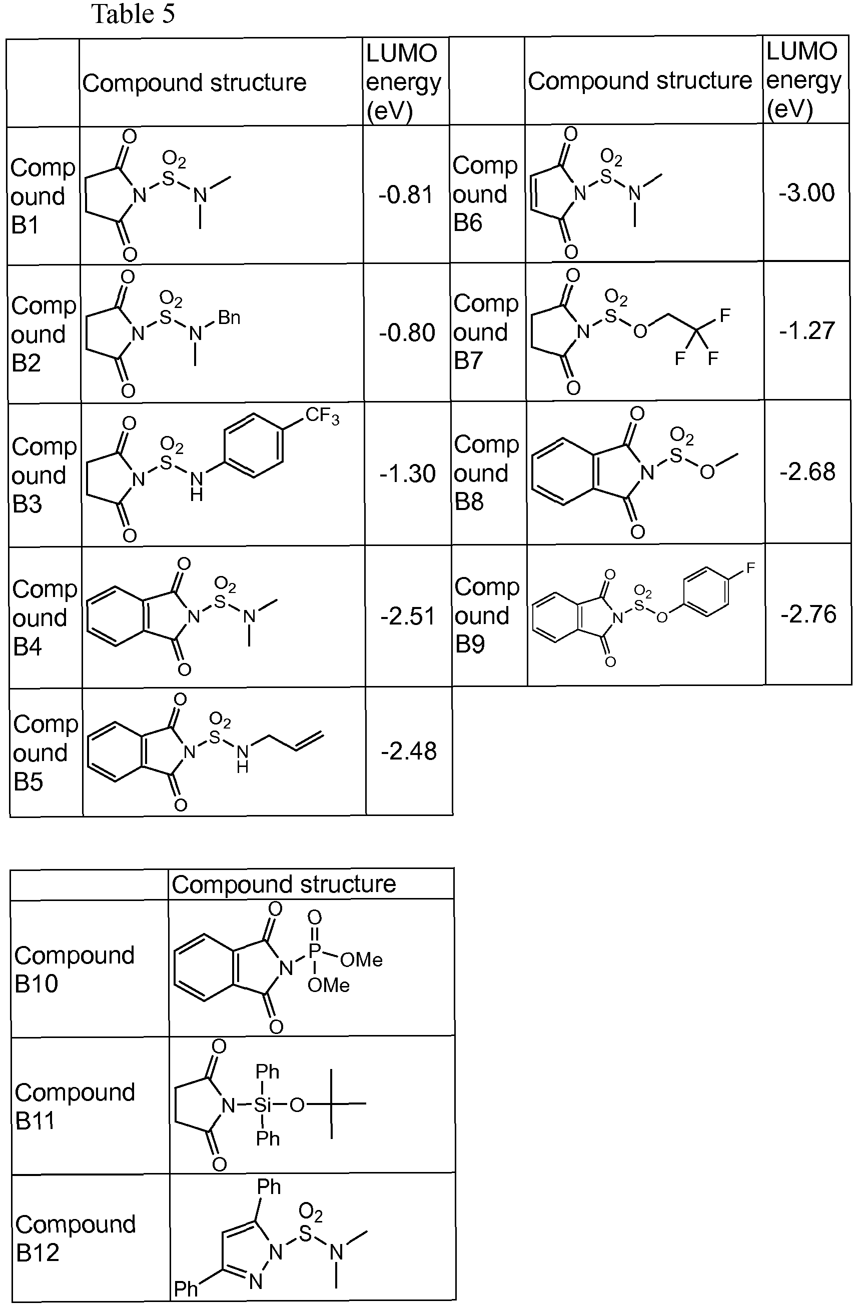

### (Stability)

The compounds B1, B2, B3, B4, B5, B6, B7, B8 and B9 used in the examples and the fluoroethylene carbonate (FEC) used in Comparative Examples B5 and B6 were supplied for a preservation test in which they were allowed to stand for 90 days in a steady temperature and humidity environment at a temperature of 40 ±2°C and a humidity of 75 ±5%. The ¹H-nuclear magnetic resonance spectrum (¹H-NMR) of each additive for a non-aqueous electrolyte solution was measured before and after the preservation test, and the stability of each compound was evaluated based on the following scale. Table 6 shows the evaluation results for stability.
G: No change in ¹H-NMR spectrum peaks before and after preservation test.
F: Slight change in ¹H-NMR spectrum peaks before and after preservation test.
P: Distinct change in ¹H-NMR spectrum peaks before and after preservation test.

**Table 6**

| Additive | Stability |
|---|---|
| Compound B1 | G |
| Compound B2 | G |
| Compound B3 | G |
| Compound B4 | G |
| Compound B5 | G |
| Compound B6 | G |
| Compound B7 | G |
| Compound B8 | G |
| Compound B9 | G |
| FEC | P |

As shown in Table 6, the fluoroethylene carbonate (FEC) used in Comparative Examples B5 and B6 was presumably partially hydrolyzed, and had inferior stability. Compounds B1, B2, B3, B4, B5, B6, B7, B8 and B9 used in the examples, on the other hand, exhibited virtually no change and had excellent stability.

### (Fabrication of positive electrode)

Lithium cobaltate (LiCoO₂) as a positive electrode active material and carbon black as a conductivity imparting agent were dry blended. The obtained mixture was uniformly dispersed in N-methyl-2-pyrrolidone (NMP) dissolving polyvinylidene fluoride (PVDF) as a binder, to prepare a slurry. The obtained slurry was coated onto both sides of an aluminum metal foil (rectilinear, thickness: 20 µm). After drying the coating film to remove the NMP, the entire foil was pressed to obtain a positive electrode sheet having an aluminum metal foil as the positive electrode collector and a positive electrode active material layer formed on both sides. The solid ratio of the positive electrode active material layer of the positive electrode sheet was positive electrode active material:conductivity imparting agent:PVDF = 90:5:5, as the mass ratio.

### (Fabrication of negative electrode)

Graphite powder as a negative electrode active material and carbon black as a conductivity imparting agent were dry blended. The obtained mixture was uniformly dispersed in N-methyl-2-pyrrolidone (NMP) dissolving polyvinylidene fluoride (PVDF) as a binder, to prepare a slurry. The obtained slurry was coated onto one side of a copper foil (rectilinear, thickness: 10 µm). After drying the coating film to remove the NMP, the entire foil was pressed to obtain a negative electrode sheet having a copper foil as the negative electrode collector and a negative electrode active material layer formed on one side thereof. The solid ratio of the negative electrode active material layer of the negative electrode sheet was negative electrode active material:conductivity imparting agent:PVDF = 93:3:4, as the mass ratio.

### (Fabrication of non-aqueous electrolyte solution secondary battery)

Cell elements were fabricated by laminating a polyethylene separator in the order of a negative electrode, a separator, a positive electrode, a separator and a negative electrode in the different non-aqueous electrolyte solutions obtained in the examples and comparative examples. Each cell element was inserted into a bag formed from a laminate film having an aluminum layer (thickness: 40 µm) and a resin layer covering both sides thereof, with the positive electrode sheet and negative electrode sheet ends protruding out from the bag. Next, each of the non-aqueous electrolyte solutions obtained in the examples and comparative examples was infused into the bag. The bag was vacuum sealed to obtain a sheet-like non-aqueous electrolyte solution secondary battery. In order to increase the adhesiveness between the electrodes, the sheet-like non-aqueous electrolyte solution secondary battery was sandwiched between glass plates and pressed.

### (Evaluation of service capacity maintenance factor and internal resistance ratio)

A procedure of charging the non-aqueous electrolyte solution secondary battery to 4.2 V with a current corresponding to 0.2 C and then discharging to 3 V with a current corresponding to 0.2 C, at 25°C, was repeated for 2 cycles to stabilize the battery.

It was then subjected to a charge-discharge cycle test with a charge rate of 0.3 C, a discharge rate of 0.3 C, a charge final voltage of 4.2 V and a discharge final voltage of 2.5 V. Table 7 shows the service capacity maintenance factor (%) after 200 cycles and the internal resistance ratio after 200 cycles, for each battery.

The "service capacity maintenance factor (%) after 200 cycles" is the ratio (percentage) of the service capacity (mAh) after a 200 cycle test with respect to the service capacity (mAh) after a 10 cycle test. Also, the "internal resistance ratio after 200 cycles" is the relative value of the resistance after a 200 cycle test, where the resistance before the cycle test is defined as 1.

### (Measurement of gas generation volume)

Separately from the battery used for the cycle test, there was prepared a non-aqueous electrolyte solution secondary battery having the same construction, containing each of the electrolyte solutions of the examples and comparative examples. A procedure of charging the battery to 4.2 V with a current corresponding to 0.2 C and then discharging to 3 V with a current corresponding to 0.2 C, at 25°C, was repeated for 2 cycles to stabilize the battery.

Next, charging of the battery was again carried out to 4.2 V with the charge rate at 0.3 C, after which the battery was allowed to stand in an environment of 60°C for 168 hours. The battery was then cooled to room temperature. The gas generation volume of the battery after standing was measured by Archimedes' method. The results are shown in Table 7.

**Table 7**

| | Electrolyte | Solvent | Additive | Service capacity maintena nce factor (%) | Internal resistanc e ratio | Gas generati on volume (ml) |
|---|---|---|---|---|---|---|
| Ex. B1 | LiPF₆ 1.0 mol/L | EC/DEC (30/70) vol% | Compound B1 0.5 mass% | 92 | 1.20 | 0.61 |
| Ex. B2 | LiPF₆ 1.0 mol/L | EC/DEC (30/70) vol% | Compound B1 1.0 mass% | 95 | 1.35 | 0.62 |
| Ex. B3 | LiPF₆ 1.0 mol/L | EC/DEC (30/70) vol% | Compound B2 1.0 mass% | 89 | 1.33 | 0.84 |
| Ex. B4 | LiPF₆ 1.0 mol/L | EC/DEC (30/70) vol% | Compound B3 1.0 mass% | 95 | 1.31 | 0.97 |
| Ex. B5 | LiPF₆ 1.0 mol/L | EC/DEC (30/70) vol% | Compound B4 1.0 mass% | 92 | 1.28 | 0.57 |
| Ex. B6 | LiPF₆ 1.0 mol/L | EC/DEC (30/70) vol% | Compound B5 1.0 mass% | 93 | 1.34 | 0.81 |
| Ex. B7 | LiPF₆ 1.0 mol/L | EC/DEC (30/70) vol% | Compound B6 1.0 mass% | 94 | 1.30 | 0.74 |
| Ex. B8 | LiPF₆ 1.0 mol/L | EC/DEC (30/70) vol% | Compound B7 1.0 mass% | 91 | 1.38 | 0.88 |
| Ex. B9 | LiPF₆ 1.0 mol/L | EC/DEC (30/70) vol% | Compound B8 1.0 mass% | 94 | 1.34 | 0.91 |
| Ex. B10 | LiPF₆ 1.0 mol/L | EC/DEC (30/70) vol% | Compound B9 1.0 mass% | 95 | 1.32 | 0.76 |
| Comp. Ex. B1 | LiPF₆ 1.0 mol/L | EC/DEC (30/70) vol% | None | 79 | 1.83 | 1.50 |
| Comp. Ex. B2 | LiPF₆ 1.0 mol/L | EC/DEC (30/70) vol% | 1,3-Propanesultone 1.0 mass% | 82 | 1.68 | 1.15 |
| Comp. Ex. B3 | LiPF₆ 1.0 mol/L | EC/DEC (30/70) vol% | VC 1.0 mass% | 83 | 1.70 | 1.64 |
| Comp. Ex. B4 | LiPF₆ 1.0 mol/L | EC/DEC (30/70) vol% | VC 2.0 mass% | 84 | 1.89 | 1.83 |
| Comp. Ex. B5 | LiPF₆ 1.0 mol/L | EC/DEC (30/70) vol% | FEC 1.0 mass% | 80 | 1.88 | 1.69 |
| Comp. Ex. B6 | LiPF₆ 1.0 mol/L | EC/DEC (30/70) vol% | FEC 2.0 mass% | 82 | 1.68 | 1.95 |
| Comp. Ex. B7 | LiPF₆ 1.0 mol/L | EC/DEC (30/70) vol% | Compound B10 1.0 mass% | 85 | 1.43 | 1.24 |
| Comp. Ex. B8 | LiPF₆ 1.0 mol/L | EC/DEC (30/70) vol% | Compound B11 1.0 mass% | 86 | 1.42 | 1.18 |
| Comp. Ex. B9 | LiPF₆ 1.0 mol/L | EC/DEC (30/70) vol% | Compound B12 1.0 mass% | 85 | 1.66 | 1.19 |

From Table 7 it is seen that the non-aqueous electrolyte solution secondary batteries using the non-aqueous electrolyte solutions of each of the examples which included compound B1, B2, B3, B4, B5, B6, B7, B8 or B9 as compounds of formula (B1), were excellent in terms of both service capacity maintenance factor during the cycle test and minimizing stored gas generation with charging, compared to the non-aqueous electrolyte solution secondary batteries using the non-aqueous electrolyte solutions of the comparative examples. This strongly suggests that the compounds of formula (B1), when used in non-aqueous electrolyte solution secondary batteries, form SEI that are stable against charge-discharge cycles and high temperature storage. It also confirmed that the compounds of formula (B1) are excellent in terms of low increase in internal resistance with charge-discharge cycling.

### <Experimental Example C>

### 1. Preparation of non-aqueous electrolyte solution

### (Example C1)

Ethylene carbonate (EC) and diethyl carbonate (DEC) were mixed in a volume ratio of EC:DEC = 30:70 to prepare a mixed non-aqueous solvent, and LiPF₆ as an electrolyte was dissolved in the mixture to a concentration of 1.0 mol/L. To the obtained solution there was added compound C1 shown in Table 8 as an additive for a non-aqueous electrolyte solution, to prepare a non-aqueous electrolyte solution. The content ratio of the additive for a non-aqueous electrolyte solution (compound C1) was 0.5 mass% based on the total mass of the non-aqueous electrolyte solution.

### (Example C2)

A non-aqueous electrolyte solution was prepared in the same manner as Example C1, except that the content ratio of compound C1 was 1.0 mass%.

### (Example C3)

A non-aqueous electrolyte solution was prepared in the same manner as Example C1, except that the additive for a non-aqueous electrolyte solution was changed from compound C1 to compound C2 shown in Table 8, and the content ratio was 1.0 mass%.

### (Example C4)

A non-aqueous electrolyte solution was prepared in the same manner as Example C1, except that the additive for a non-aqueous electrolyte solution was changed from compound C1 to compound C3 shown in Table 8, and the content ratio was 1.0 mass%.

### (Example C5)

A non-aqueous electrolyte solution was prepared in the same manner as Example C1, except that the additive for a non-aqueous electrolyte solution was changed from compound C1 to compound C4 shown in Table 8, and the content ratio was 1.0 mass%.

### (Example C6)

A non-aqueous electrolyte solution was prepared in the same manner as Example C1, except that the additive for a non-aqueous electrolyte solution was changed from compound C1 to compound C5 shown in Table 8, and the content ratio was 1.0 mass%.

### (Example C7)

A non-aqueous electrolyte solution was prepared in the same manner as Example C1, except that the additive for a non-aqueous electrolyte solution was changed from compound C1 to compound C6 shown in Table 8, and the content ratio was 1.0 mass%.

### (Example C8)

A non-aqueous electrolyte solution was prepared in the same manner as Example C1, except that the additive for a non-aqueous electrolyte solution was changed from compound C1 to compound C7 shown in Table 8, and the content ratio was 1.0 mass%.

### (Example C9)

A non-aqueous electrolyte solution was prepared in the same manner as Example C1, except that the additive for a non-aqueous electrolyte solution was changed from compound C1 to compound C8 shown in Table 8, and the content ratio was 1.0 mass%.

### (Example C10)

A non-aqueous electrolyte solution was prepared in the same manner as Example C1, except that the additive for a non-aqueous electrolyte solution was changed from compound C1 to compound C9 shown in Table 8, and the content ratio was 1.0 mass%.

### (Comparative Example C1)

A non-aqueous electrolyte solution was prepared in the same manner as Example C1, except that compound C1 was not added.

### (Comparative Example C2)

A non-aqueous electrolyte solution was prepared in the same manner as Example C1, except that the additive for a non-aqueous electrolyte solution was changed from compound C1 to 1,3-propanesultone, and the content ratio was 1.0 mass%.

### (Comparative Example C3)

A non-aqueous electrolyte solution was prepared in the same manner as Example C1, except that the additive for a non-aqueous electrolyte solution was changed from compound C1 to vinylene carbonate (VC), and the content ratio was 1.0 mass%.

### (Comparative Example C4)

A non-aqueous electrolyte solution was prepared in the same manner as Comparative Example C3, except that the content ratio of the vinylene carbonate (VC) was 2.0 mass%.

### (Comparative Example C5)

A non-aqueous electrolyte solution was prepared in the same manner as Example C1, except that the additive for a non-aqueous electrolyte solution was changed from compound C1 to fluoroethylene carbonate (FEC), and the content ratio was 1.0 mass%.

### (Comparative Example C6)

A non-aqueous electrolyte solution was prepared in the same manner as Comparative Example C5, except that the content ratio of the fluoroethylene carbonate (FEC) was 2.0 mass%.

### (Comparative Example C7)

A non-aqueous electrolyte solution was prepared in the same manner as Example C1, except that the additive for a non-aqueous electrolyte solution was changed from compound C1 to compound C10 shown in Table 8, and the content ratio was 1.0 mass%.

### (Comparative Example C8)

A non-aqueous electrolyte solution was prepared in the same manner as Example C1, except that the additive for a non-aqueous electrolyte solution was changed from compound C1 to compound C11 shown in Table 8, and the content ratio was 1.0 mass%.

### (Comparative Example C9)

A non-aqueous electrolyte solution was prepared in the same manner as Example C1, except that the additive for a non-aqueous electrolyte solution was changed from compound C1 to compound C12 shown in Table 8, and the content ratio was 1.0 mass%.

### 2. Evaluation

### (Calculation of LUMO energy)

The LUMO (lowest unoccupied molecular orbital) energies of compounds C1, C2, C3, C4, C5, C6, C7, C8 and C9 used in the Examples were determined by calculating the semiempirical molecular orbitals using Gaussian 03 software. The calculated LUMO energies are shown in Table 8.

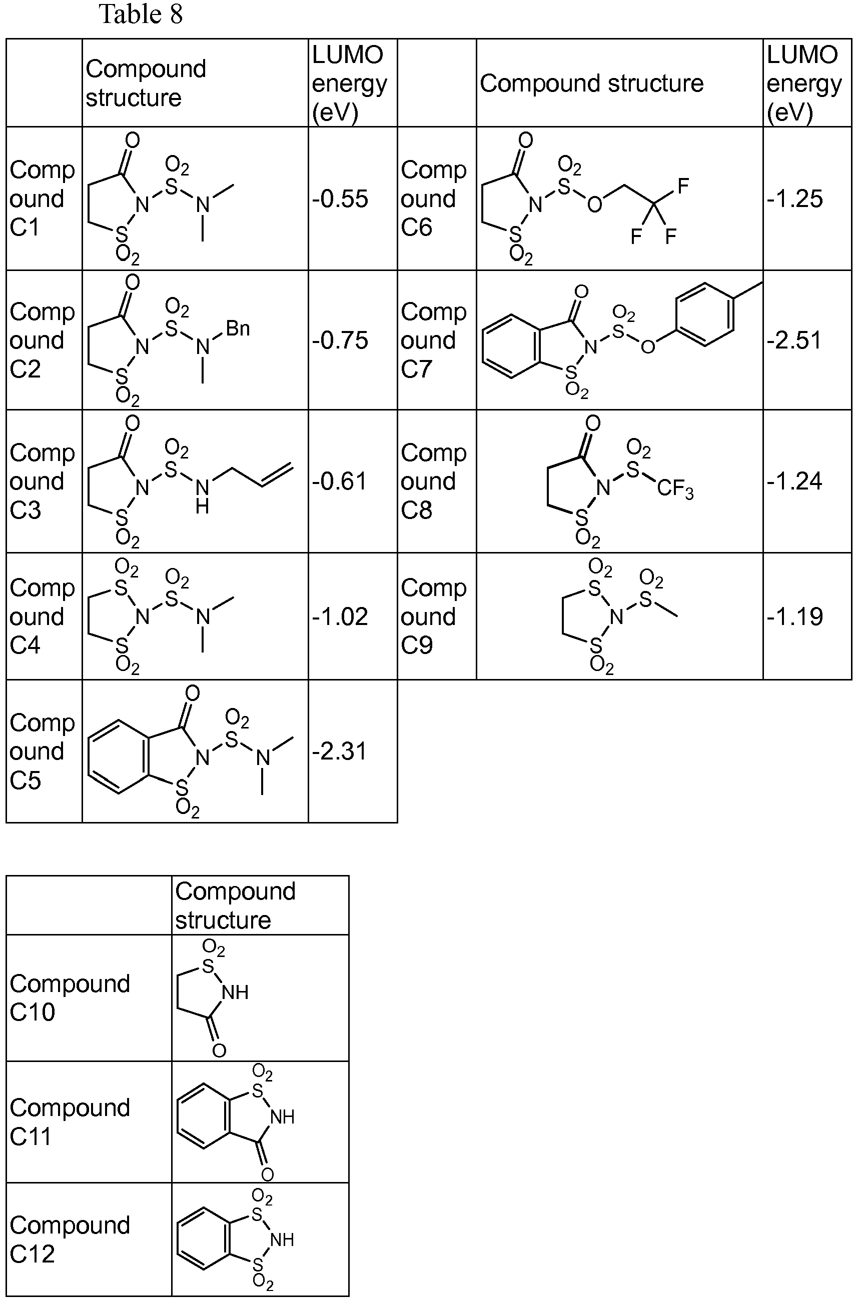

### (Stability)

The compounds C1, C2, C3, C4, C5, C6, C7, C8 and C9 used in the examples and the fluoroethylene carbonate (FEC) used in Comparative Examples C5 and C6 were supplied for a preservation test in which they were allowed to stand for 90 days in a steady temperature and humidity environment at a temperature of 40 ±2°C and a humidity of 75 ±5%. The ¹H-nuclear magnetic resonance spectrum (¹H-NMR) of each additive for a non-aqueous electrolyte solution was measured before and after the preservation test, and the stability of each compound was evaluated based on the following scale. Table 9 shows the evaluation results for stability.
G: No change in ¹H-NMR spectrum peaks before and after preservation test.
F: Slight change in ¹H-NMR spectrum peaks before and after preservation test.
P: Distinct change in ¹H-NMR spectrum peaks before and after preservation test.

**Table 9**

| Additive | Stability |
|---|---|
| Compound C1 | G |
| Compound C2 | G |
| Compound C3 | G |
| Compound C4 | G |
| Compound C5 | G |
| Compound C6 | G |
| Compound C7 | G |
| Compound C8 | G |
| Compound C9 | G |
| FEC | P |

As shown in Table 9, the fluoroethylene carbonate (FEC) used in Comparative Examples C5 and C6 was presumably partially hydrolyzed, and had inferior stability. Compounds C1, C2, C3, C4, C5, C6, C7, C8 and C9 used in the examples, on the other hand, exhibited virtually no change and had excellent stability.

### (Fabrication of positive electrode)

Lithium cobaltate (LiCoO₂) as a positive electrode active material and carbon black as a conductivity imparting agent were dry blended. The obtained mixture was uniformly dispersed in N-methyl-2-pyrrolidone (NMP) dissolving polyvinylidene fluoride (PVDF) as a binder, to prepare a slurry. The obtained slurry was coated onto both sides of an aluminum metal foil (rectilinear, thickness: 20 µm). After drying the coating film to remove the NMP, the entire foil was pressed to obtain a positive electrode sheet having an aluminum metal foil as the positive electrode collector and a positive electrode active material layer formed on both sides. The solid ratio of the positive electrode active material layer of the positive electrode sheet was positive electrode active material:conductivity imparting agent:PVDF = 90:5:5, as the mass ratio.

### (Fabrication of negative electrode)

Graphite powder as a negative electrode active material and carbon black as a conductivity imparting agent were dry blended. The obtained mixture was uniformly dispersed in N-methyl-2-pyrrolidone (NMP) dissolving polyvinylidene fluoride (PVDF) as a binder, to prepare a slurry. The obtained slurry was coated onto one side of a copper foil (rectilinear, thickness: 10 µm). After drying the coating film to remove the NMP, the entire foil was pressed to obtain a negative electrode sheet having a copper foil as the negative electrode collector and a negative electrode active material layer formed on one side thereof. The solid ratio of the negative electrode active material layer of the negative electrode sheet was negative electrode active material:conductivity imparting agent:PVDF = 93:3:4, as the mass ratio.

### (Fabrication of non-aqueous electrolyte solution secondary battery)

Cell elements were fabricated by laminating a polyethylene separator in the order of a negative electrode, a separator, a positive electrode, a separator and a negative electrode in the different non-aqueous electrolyte solutions obtained in the examples and comparative examples. Each cell element was inserted into a bag formed from a laminate film having an aluminum layer (thickness: 40 µm) and a resin layer covering both sides thereof, with the positive electrode sheet and negative electrode sheet ends protruding out from the bag. Next, each of the non-aqueous electrolyte solutions obtained in the examples and comparative examples was infused into the bag. The bag was vacuum sealed to obtain a sheet-like non-aqueous electrolyte solution secondary battery. In order to increase the adhesiveness between the electrodes, the sheet-like non-aqueous electrolyte solution secondary battery was sandwiched between glass plates and pressed.

### (Evaluation of service capacity maintenance factor and internal resistance ratio)

A procedure of charging the non-aqueous electrolyte solution secondary battery to 4.2 V with a current corresponding to 0.2 C and then discharging to 3 V with a current corresponding to 0.2 C, at 25°C, was repeated for 2 cycles to stabilize the battery.

It was then subjected to a charge-discharge cycle test with a charge rate of 0.3 C, a discharge rate of 0.3 C, a charge final voltage of 4.2 V and a discharge final voltage of 2.5 V. Table 10 shows the service capacity maintenance factor (%) after 200 cycles and the internal resistance ratio after 200 cycles, for each battery.

The "service capacity maintenance factor (%) after 200 cycles" is the ratio (percentage) of the service capacity (mAh) after a 200 cycle test with respect to the service capacity (mAh) after a 10 cycle test. Also, the "internal resistance ratio after 200 cycles" is the relative value of the resistance after a 200 cycle test, where the resistance before the cycle test is defined as 1.

### (Measurement of gas generation volume)

Separately from the battery used for the cycle test, there was prepared a non-aqueous electrolyte solution secondary battery having the same construction, containing each of the electrolyte solutions of the examples and comparative examples. A procedure of charging the battery to 4.2 V with a current corresponding to 0.2 C and then discharging to 3 V with a current corresponding to 0.2 C, at 25°C, was repeated for 2 cycles to stabilize the battery.

Next, charging of the battery was again carried out to 4.2 V with the charge rate at 0.3 C, after which the battery was allowed to stand in an environment of 60°C for 168 hours. The battery was then cooled to room temperature. The gas generation volume of the battery after standing was measured by Archimedes' method. The results are shown in Table 10.

**Table 10**

| | Electrolyte | Solvent | Additive | Service capacity mainten ance factor (%) | Internal resistan ce ratio | Gas generation volume (ml) |
|---|---|---|---|---|---|---|
| Ex. C1 | LiPF₆ 1.0 mol/L | EC/DEC (30/70) vol% | Compound C1 0.5 mass% | 94 | 1.25 | 0.70 |
| Ex. C2 | LiPF₆ 1.0 mol/L | EC/DEC (30/70) vol% | Compound C1 1.0 mass% | 95 | 1.30 | 0.75 |
| Ex. C3 | LiPF₆ 1.0 mol/L | EC/DEC (30/70) vol% | Compound C2 1.0 mass% | 94 | 1.35 | 0.69 |
| Ex. C4 | LiPF₆ 1.0 mol/L | EC/DEC (30/70) vol% | Compound C3 1.0 mass% | 90 | 1.29 | 0.68 |
| Ex. C5 | LiPF₆ 1.0 mol/L | EC/DEC (30/70) vol% | Compound C4 1.0 mass% | 93 | 1.25 | 0.82 |
| Ex. C6 | LiPF₆ 1.0 mol/L | EC/DEC (30/70) vol% | Compound C5 1.0 mass% | 95 | 1.32 | 0.85 |
| Ex. C7 | LiPF₆ 1.0 mol/L | EC/DEC (30/70) vol% | Compound C6 1.0 mass% | 92 | 1.35 | 0.65 |
| Ex. C8 | LiPF₆ 1.0 mol/L | EC/DEC (30/70) vol% | Compound C7 1.0 mass% | 90 | 1.37 | 0.80 |
| Ex. C9 | LiPF₆ 1.0 mol/L | EC/DEC (30/70) vol% | Compound C8 1.0 mass% | 94 | 1.26 | 0.84 |
| Ex. C10 | LiPF₆ 1.0 mol/L | EC/DEC (30/70) vol% | Compound C9 1.0 mass% | 95 | 1.28 | 0.75 |
| Comp. Ex. C1 | LiPF₆ 1.0 mol/L | EC/DEC (30/70) vol% | None | 79 | 1.83 | 1.50 |
| Comp. Ex. C2 | LiPF₆ 1.0 mol/L | EC/DEC (30/70) vol% | 1,3-Propanesultone 1.0 mass% | 82 | 1.68 | 1.15 |
| Comp. Ex. C3 | LiPF₆ 1.0 mol/L | EC/DEC (30/70) vol% | VC 1.0 mass% | 83 | 1.70 | 1.64 |
| Comp. Ex. C4 | LiPF₆ 1.0 mol/L | EC/DEC (30/70) vol% | VC 2.0 mass% | 84 | 1.89 | 1.83 |
| Comp. Ex. C5 | LiPF₆ 1.0 mol/L | EC/DEC (30/70) vol% | FEC 1.0 mass% | 80 | 1.88 | 1.69 |
| Comp. Ex. C6 | LiPF₆ 1.0 mol/L | EC/DEC (30/70) vol% | FEC 2.0 mass% | 82 | 1.68 | 1.95 |
| Comp. Ex. C7 | LiPF₆ 1.0 mol/L | EC/DEC (30/70) vol% | Compound C10 1.0 mass% | 87 | 1.75 | 1.40 |
| Comp. Ex. C8 | LiPF₆ 1.0 mol/L | EC/DEC (30/70) vol% | Compound C11 1.0 mass% | 87 | 1.81 | 1.39 |
| Comp. Ex. C9 | LiPF₆ 1.0 mol/L | EC/DEC (30/70) vol% | Compound C12 1.0 mass% | 85 | 1.85 | 1.89 |

From Table 10 it is seen that the non-aqueous electrolyte solution secondary batteries using the non-aqueous electrolyte solutions of each of the examples which included compound C1, C2, C3, C4, C5, C6, C7, C8 or C9 as compounds of formula (C1), were excellent in terms of both service capacity maintenance factor during the cycle test and minimizing gas generation with charging, compared to the non-aqueous electrolyte solution secondary batteries using the non-aqueous electrolyte solutions of the comparative examples. This strongly suggests that the compounds of formula (C1), when used in non-aqueous electrolyte solution secondary batteries, form SEI that are stable against charge-discharge cycles and high temperature storage. It also confirmed that the compounds of formula (C1) are excellent in terms of low increase in internal resistance with charge-discharge cycling.

### <Experimental Example D>

### [Examples D1-D10, Comparative Examples D1-D9]

### 1. Preparation of non-aqueous electrolyte solution

### (Example D1)

Ethylene carbonate (EC) and diethyl carbonate (DEC) were mixed in a volume ratio of EC:DEC = 30:70 to prepare a mixed non-aqueous solvent, and LiPF₆ as an electrolyte was dissolved in the mixture to a concentration of 1.0 mol/L. To the obtained solution there was added compound D1 shown in Table 11 as an additive for a non-aqueous electrolyte solution, to prepare a non-aqueous electrolyte solution. The content ratio of the additive for a non-aqueous electrolyte solution (compound D1) was 0.5 mass% with respect to the total mass of the non-aqueous electrolyte solution.

### (Example D2)

A non-aqueous electrolyte solution was prepared in the same manner as Example D1, except that the content ratio of compound D1 was 1.0 mass%.

### (Example D3)

A non-aqueous electrolyte solution was prepared in the same manner as Example D1, except that the additive for a non-aqueous electrolyte solution was changed from compound D1 to compound D2 shown in Table 11, and the content ratio was 1.0 mass%.

### (Example D4)

A non-aqueous electrolyte solution was prepared in the same manner as Example D1, except that the additive for a non-aqueous electrolyte solution was changed from compound D1 to compound D3 shown in Table 11, and the content ratio was 1.0 mass%.

### (Example D5)

A non-aqueous electrolyte solution was prepared in the same manner as Example D1, except that the additive for a non-aqueous electrolyte solution was changed from compound D1 to compound D4 shown in Table 11, and the content ratio was 1.0 mass%.

### (Example D6)

A non-aqueous electrolyte solution was prepared in the same manner as Example D1, except that the additive for a non-aqueous electrolyte solution was changed from compound D1 to compound D5 shown in Table 11, and the content ratio was 1.0 mass%.

### (Example D7)

A non-aqueous electrolyte solution was prepared in the same manner as Example D1, except that the additive for a non-aqueous electrolyte solution was changed from compound D1 to compound D6 shown in Table 11, and the content ratio was 1.0 mass%.

### (Example D8)

A non-aqueous electrolyte solution was prepared in the same manner as Example D1, except that the additive for a non-aqueous electrolyte solution was changed from compound D1 to compound D7 shown in Table 11, and the content ratio was 1.0 mass%.

### (Example D9)

A non-aqueous electrolyte solution was prepared in the same manner as Example D1, except that the additive for a non-aqueous electrolyte solution was changed from compound D1 to compound D8 shown in Table 11, and the content ratio was 1.0 mass%.

### (Example D10)

A non-aqueous electrolyte solution was prepared in the same manner as Example D1, except that the additive for a non-aqueous electrolyte solution was changed from compound D1 to compound D9 shown in Table 11, and the content ratio was 1.0 mass%.

### (Comparative Example D1)

A non-aqueous electrolyte solution was prepared in the same manner as Example D1, except that compound D1 was not added.

### (Comparative Example D2)

A non-aqueous electrolyte solution was prepared in the same manner as Example D1, except that the additive for a non-aqueous electrolyte solution was changed from compound D1 to 1,3-propanesultone, and the content ratio was 1.0 mass%.

### (Comparative Example D3)

A non-aqueous electrolyte solution was prepared in the same manner as Example D1, except that the additive for a non-aqueous electrolyte solution was changed from compound D1 to vinylene carbonate (VC), and the content ratio was 1.0 mass%.

### (Comparative Example D4)

A non-aqueous electrolyte solution was prepared in the same manner as Comparative Example D3, except that the content ratio of the vinylene carbonate (VC) was 2.0 mass%.

### (Comparative Example D5)

A non-aqueous electrolyte solution was prepared in the same manner as Example D1, except that the additive for a non-aqueous electrolyte solution was changed from compound D1 to fluoroethylene carbonate (FEC), and the content ratio was 1.0 mass%.

### (Comparative Example D6)

A non-aqueous electrolyte solution was prepared in the same manner as Comparative Example D5, except that the content ratio of the fluoroethylene carbonate (FEC) was 2.0 mass%.

### (Comparative Example D7)

A non-aqueous electrolyte solution was prepared in the same manner as Example D1, except that the additive for a non-aqueous electrolyte solution was changed from compound D1 to compound D10 shown in Table 11, and the content ratio was 1.0 mass%.

### (Comparative Example D8)

A non-aqueous electrolyte solution was prepared in the same manner as Example D1, except that the additive for a non-aqueous electrolyte solution was changed from compound D1 to compound D11 shown in Table 11, and the content ratio was 1.0 mass%.

### (Comparative Example D9)

A non-aqueous electrolyte solution was prepared in the same manner as Example D1, except that the additive for a non-aqueous electrolyte solution was changed from compound D1 to compound D12 shown in Table 11, and the content ratio was 1.0 mass%.

### 2. Evaluation

### (Measurement of LUMO energy)

The LUMO (lowest unoccupied molecular orbital) energies of compounds D1, D2, D3, D4, D5, D6, D7, D8 and D9 used in the Examples were determined by calculating the semiempirical molecular orbitals using Gaussian 03 software. The calculated LUMO energies are shown in Table 11.

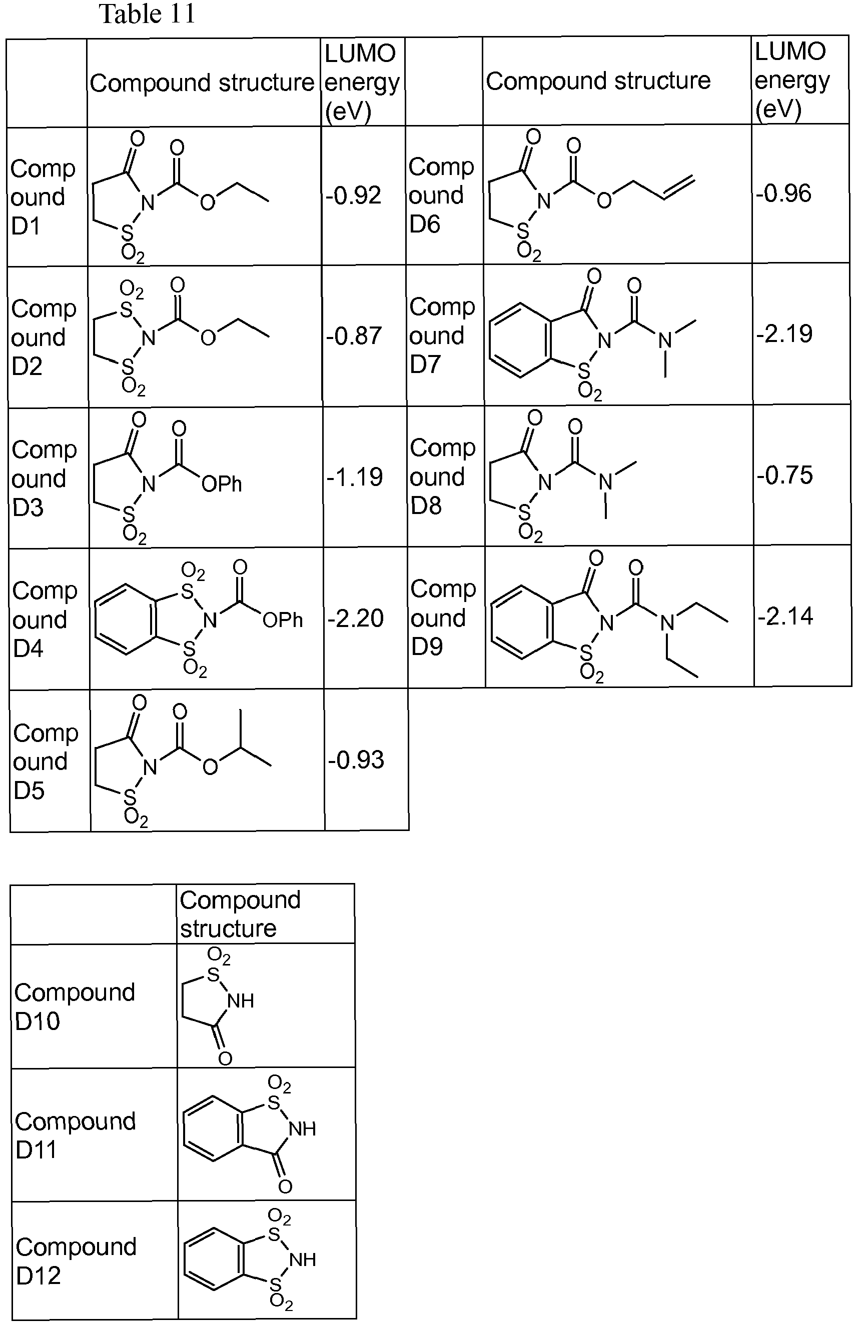

### (Stability)

The compounds D1, D2, D3, D4, D5, D6, D7, D8 and D9 used in the examples and the fluoroethylene carbonate (FEC) used in Comparative Examples D5 and D6 were supplied for a preservation test in which they were allowed to stand for 90 days in a steady temperature and humidity environment at a temperature of 40 ±2°C and a humidity of 75 ±5%. The ¹H-nuclear magnetic resonance spectrum (¹H-NMR) of each additive for a non-aqueous electrolyte solution was measured before and after the preservation test, and the stability of each compound was evaluated based on the following scale. Table 12 shows the evaluation results for stability.
G: No change in ¹H-NMR spectrum peaks before and after preservation test.
F: Slight change in ¹H-NMR spectrum peaks before and after preservation test.
P: Distinct change in ¹H-NMR spectrum peaks before and after preservation test.

**Table 12**

| Additive | Stability |
|---|---|
| Compound D1 | G |
| Compound D2 | G |
| Compound D3 | G |
| Compound D4 | G |
| Compound D5 | G |
| Compound D6 | G |
| Compound D7 | G |
| Compound D8 | G |
| Compound D9 | G |
| FEC | P |

As shown in Table 12, the fluoroethylene carbonate (FEC) used in Comparative Examples D5 and D6 was presumably partially hydrolyzed, and had inferior stability. Compounds D1, D2, D3, D4, D5, D6, D7, D8 and D9 used in the examples, on the other hand, exhibited virtually no change and had excellent stability.

### (Fabrication of positive electrode)

Lithium cobaltate (LiCoO₂) as a positive electrode active material and carbon black as a conductivity imparting agent were dry blended. The obtained mixture was uniformly dispersed in N-methyl-2-pyrrolidone (NMP) dissolving polyvinylidene fluoride (PVDF) as a binder, to prepare a slurry. The obtained slurry was coated onto both sides of an aluminum metal foil (rectilinear, thickness: 20 µm). After drying the coating film to remove the NMP, the entire foil was pressed to obtain a positive electrode sheet having an aluminum metal foil as the positive electrode collector and a positive electrode active material layer formed on both sides. The solid ratio of the positive electrode active material layer of the positive electrode sheet was positive electrode active material:conductivity imparting agent:PVDF = 90:5:5, as the mass ratio.

### (Fabrication of negative electrode)

Graphite powder as a negative electrode active material and carbon black as a conductivity imparting agent were dry blended. The obtained mixture was uniformly dispersed in N-methyl-2-pyrrolidone (NMP) dissolving polyvinylidene fluoride (PVDF) as a binder, to prepare a slurry. The obtained slurry was coated onto one side of a copper foil (rectilinear, thickness: 10 µm). After drying the coating film to remove the NMP, the entire foil was pressed to obtain a negative electrode sheet having a copper foil as the negative electrode collector and a negative electrode active material layer formed on one side thereof. The solid ratio of the negative electrode active material layer of the negative electrode sheet was negative electrode active material:conductivity imparting agent:PVDF = 93:3:4, as the mass ratio.

### (Fabrication of non-aqueous electrolyte solution secondary battery)

Cell elements were fabricated by laminating a negative electrode sheet, a polyethylene separator, a positive electrode sheet, a polyethylene separator and a negative electrode sheet, in that order, in the non-aqueous electrolyte solutions obtained in the examples and comparative examples. Each cell element was inserted into a bag formed from a laminate film having an aluminum layer (thickness: 40 µm) and a resin layer covering both sides thereof, with the positive electrode sheet and negative electrode sheet ends protruding out from the bag. Next, each of the non-aqueous electrolyte solutions obtained in the examples and comparative examples was infused into the bag. The bag was vacuum sealed to obtain a sheet-like non-aqueous electrolyte solution secondary battery. In order to increase the adhesiveness between the electrodes, the sheet-like non-aqueous electrolyte solution secondary battery was sandwiched between glass plates and pressed.

### (Evaluation of service capacity maintenance factor and internal resistance ratio)

A procedure of charging the non-aqueous electrolyte solution secondary battery to 4.2 V with a current corresponding to 0.2 C and then discharging to 3 V with a current corresponding to 0.2 C, at 25°C, was repeated for 2 cycles to stabilize the battery.

It was then subjected to a charge-discharge cycle test with a charge rate of 0.3 C, a discharge rate of 0.3 C, a charge final voltage of 4.2 V and a discharge final voltage of 2.5 V. Table 13 shows the service capacity maintenance factor (%) after 200 cycles and the internal resistance ratio after 200 cycles, for each battery.

The "service capacity maintenance factor (%) after 200 cycles" is the ratio (percentage) of the service capacity (mAh) after a 200 cycle test with respect to the service capacity (mAh) after a 10 cycle test. Also, the "internal resistance ratio after 200 cycles" is the relative value of the resistance after a 200 cycle test, where the resistance before the cycle test is defined as 1.

### (Measurement of gas generation volume)

Separately from the battery used for the cycle test, there was prepared a non-aqueous electrolyte solution secondary battery having the same construction, containing each of the electrolyte solutions of the examples and comparative examples. A procedure of charging the battery to 4.2 V with a current corresponding to 0.2 C and then discharging to 3 V with a current corresponding to 0.2 C, at 25°C, was repeated for 2 cycles to stabilize the battery.

Next, charging of the battery was again carried out to 4.2 V with the charge rate at 0.3 C, after which the battery was allowed to stand in an environment of 60°C for 168 hours. The battery was then cooled to room temperature. The gas generation volume of the battery after standing was measured by Archimedes' method. The results are shown in Table 13.

**Table 13**

| | Electrolyte | Solvent | Additive | Service capacity mainten ance factor (%) | Internal resistan ce ratio | Gas generation volume (ml) |
|---|---|---|---|---|---|---|
| Ex. D1 | LiPF₆ 1.0 mol/L | EC/DEC (30/70) vol% | Compound D1 0.5 mass% | 93 | 1.26 | 0.80 |
| Ex. D2 | LiPF₆ 1.0 mol/L | EC/DEC (30/70) vol% | Compound D1 1.0 mass% | 92 | 1.32 | 0.74 |
| Ex. D3 | LiPF₆ 1.0 mol/L | EC/DEC (30/70) vol% | Compound D2 1.0 mass% | 95 | 1.33 | 0.73 |
| Ex. D4 | LiPF₆ 1.0 mol/L | EC/DEC (30/70) vol% | Compound D3 1.0 mass% | 96 | 1.25 | 0.70 |
| Ex. D5 | LiPF₆ 1.0 mol/L | EC/DEC (30/70) vol% | Compound D4 1.0 mass% | 94 | 1.24 | 0.88 |
| Ex. D6 | LiPF₆ 1.0 mol/L | EC/DEC (30/70) vol% | Compound D5 1.0 mass% | 94 | 1.26 | 0.85 |
| Ex. D7 | LiPF₆ 1.0 mol/L | EC/DEC (30/70) vol% | Compound D6 1.0 mass% | 95 | 1.38 | 0.66 |
| Ex. D8 | LiPF₆ 1.0 mol/L | EC/DEC (30/70) vol% | Compound D7 1.0 mass% | 96 | 1.35 | 0.77 |
| Ex. D9 | LiPF₆ 1.0 mol/L | EC/DEC (30/70) vol% | Compound D8 1.0 mass% | 95 | 1.30 | 0.83 |
| Ex. D10 | LiPF₆ 1.0 mol/L | EC/DEC (30/70) vol% | Compound D9 1.0 mass% | 92 | 1.28 | 0.79 |
| Comp. Ex. D1 | LiPF₆ 1.0 mol/L | EC/DEC (30/70) vol% | None | 79 | 1.83 | 1.50 |
| Comp. Ex. D2 | LiPF₆ 1.0 mol/L | EC/DEC (30/70) vol% | 1,3-Propanesultone 1.0 mass% | 82 | 1.68 | 1.15 |
| Comp. Ex. D3 | LiPF₆ 1.0 mol/L | EC/DEC (30/70) vol% | VC 1.0 mass% | 83 | 1.70 | 1.64 |
| Comp. Ex. D4 | LiPF₆ 1.0 mol/L | EC/DEC (30/70) vol% | VC 2.0 mass% | 84 | 1.89 | 1.83 |
| Comp. Ex. D5 | LiPF₆ 1.0 mol/L | EC/DEC (30/70) vol% | FEC 1.0 mass% | 80 | 1.88 | 1.69 |
| Comp. Ex. D6 | LiPF₆ 1.0 mol/L | EC/DEC (30/70) vol% | FEC 2.0 mass% | 82 | 1.68 | 1.95 |
| Comp. Ex. D7 | LiPF₆ 1.0 mol/L | EC/DEC (30/70) vol% | Compound D10 1.0 mass% | 87 | 1.75 | 1.40 |
| Comp. Ex. D8 | LiPF₆ 1.0 mol/L | EC/DEC (30/70) vol% | Compound D11 1.0 mass% | 87 | 1.81 | 1.39 |
| Comp. Ex. D9 | LiPF₆ 1.0 mol/L | EC/DEC (30/70) vol% | Compound D12 1.0 mass% | 85 | 1.85 | 1.89 |

From Table 13 it is seen that the non-aqueous electrolyte solution secondary batteries using the non-aqueous electrolyte solutions of each of the examples which included compounds D1, D2, D3, D4, D5, D6, D7, D8 or D9, as compounds of formula (D1) and formula (D2), were excellent in terms of both service capacity maintenance factor during the cycle test and minimizing stored gas generation with charging, compared to the non-aqueous electrolyte solution secondary batteries using the non-aqueous electrolyte solutions of the comparative examples. This strongly suggests that the compounds of formula (D1) and formula (D2), when used in non-aqueous electrolyte solution secondary batteries, form SEI that are stable against charge-discharge cycles and high temperature storage. It also confirmed that the compounds of formula (D1) and formula (D2) are excellent in terms of low increase in internal resistance with charge-discharge cycling.

### [Examples D11-D20, Comparative Examples D10-D19]

### 1. Preparation of non-aqueous electrolyte solution

### (Example D11)

Ethylene carbonate (EC) and diethyl carbonate (DEC) were mixed in a volume ratio of EC:DEC = 30:70 to prepare a mixed non-aqueous solvent, and LiPF₆ as an electrolyte was dissolved in the mixture to a concentration of 1.0 mol/L. To the obtained solution there was added compound D13 shown in Table 14 as an additive for a non-aqueous electrolyte solution, to prepare a non-aqueous electrolyte solution. The content ratio of the additive for a non-aqueous electrolyte solution (compound D13) was 1.0 mass% with respect to the total mass of the non-aqueous electrolyte solution.

### (Example D12)

A non-aqueous electrolyte solution was prepared in the same manner as Example D11, except that the additive for a non-aqueous electrolyte solution was changed from compound D13 to compound D14 shown in Table 14.

### (Example D13)

A non-aqueous electrolyte solution was prepared in the same manner as Example D11, except that the additive for a non-aqueous electrolyte solution was changed from compound D13 to compound D15 shown in Table 14.

### (Example D14)

A non-aqueous electrolyte solution was prepared in the same manner as Example D11, except that the additive for a non-aqueous electrolyte solution was changed from compound D13 to compound D16 shown in Table 14.

### (Example D15)

A non-aqueous electrolyte solution was prepared in the same manner as Example D11, except that the additive for a non-aqueous electrolyte solution was changed from compound D13 to compound D17 shown in Table 14.

### (Comparative Example D10)

A non-aqueous electrolyte solution was prepared in the same manner as Example D11, except that compound D13 was not added.

### (Comparative Example D11)

A non-aqueous electrolyte solution was prepared in the same manner as Example D11, except that the additive for a non-aqueous electrolyte solution was changed from compound D13 to 1,3-propanesultone.

### (Comparative Example D12)

A non-aqueous electrolyte solution was prepared in the same manner as Example D11, except that the additive for a non-aqueous electrolyte solution was changed from compound D13 to vinylene carbonate (VC).

### (Comparative Example D13)

A non-aqueous electrolyte solution was prepared in the same manner as Comparative Example D12, except that the content ratio of the vinylene carbonate (VC) was 2.0 mass%.

### (Comparative Example D14)

A non-aqueous electrolyte solution was prepared in the same manner as Example D11, except that the additive for a non-aqueous electrolyte solution was changed from compound D13 to fluoroethylene carbonate (FEC).

### (Comparative Example D15)

A non-aqueous electrolyte solution was prepared in the same manner as Comparative Example D14, except that the content ratio of the fluoroethylene carbonate (FEC) was 2.0 mass%.

### (Comparative Example D16)

A non-aqueous electrolyte solution was prepared in the same manner as Example D11, except that the additive for a non-aqueous electrolyte solution was changed from compound D13 to saccharin.

### 2. Evaluation

### (Calculation of LUMO energy)

The LUMO (lowest unoccupied molecular orbital) energies of compounds D13, D14, D15, D16 and D17 used in the Examples were determined by calculating the semiempirical molecular orbitals using Gaussian 03 software. The calculated LUMO energies are shown in Table 14.

**Table 14**

| | Compound structure | LUMO energy (eV) |
|---|---|---|
| Compound D13 | 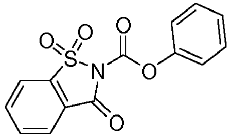 | -2.35 |
| Compound D14 | 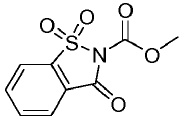 | -2.29 |
| Compound D15 | 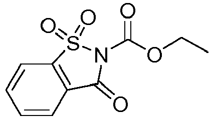 | -2.26 |
| Compound D16 | 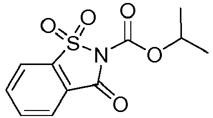 | -2.22 |
| Compound D17 | 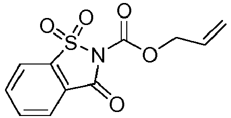 | -2.25 |

### (Stability)

The compounds D13, D14, D15, D16 and D17 used in the examples and the fluoroethylene carbonate (FEC) used in Comparative Examples D14 and D15 were supplied for a preservation test in which they were allowed to stand for 90 days in a steady temperature and humidity environment at a temperature of 40 ±2°C and a humidity of 75 ±5%. The ¹H-nuclear magnetic resonance spectrum (¹H-NMR) of each additive for a non-aqueous electrolyte solution was measured before and after the preservation test, and the stability of each compound was evaluated based on the following scale. Table 15 shows the evaluation results for stability.
G: No change in ¹H-NMR spectrum peaks before and after preservation test.
F: Slight change in ¹H-NMR spectrum peaks before and after preservation test.
P: Distinct change in ¹H-NMR spectrum peaks before and after preservation test.

**Table 15**

| Additive | Stability |
|---|---|
| Compound D13 | G |
| Compound D14 | G |
| Compound D15 | G |
| Compound D16 | G |
| Compound D17 | G |
| FEC | P |

As shown in Table 15, the fluoroethylene carbonate (FEC) used in Comparative Examples D14 and D15 was presumably partially hydrolyzed, and had inferior stability. Compounds D13, D14, D15, D16 and D17 used in the examples, on the other hand, exhibited virtually no change and had excellent stability.

### (Fabrication of positive electrode)

LiMn₂O₄ as a positive electrode active material and carbon black as a conductivity imparting agent were dry blended. The obtained mixture was uniformly dispersed in N-methyl-2-pyrrolidone (NMP) dissolving polyvinylidene fluoride (PVDF) as a binder, to prepare a slurry. The obtained slurry was coated onto both sides of an aluminum metal foil (rectilinear, thickness: 20 µm). After drying the coating film to remove the NMP, the entire foil was pressed to obtain a positive electrode sheet having an aluminum metal foil as the positive electrode collector and a positive electrode active material layer formed on both sides. The solid ratio of the positive electrode active material layer of the positive electrode sheet was positive electrode active material:conductivity imparting agent:PVDF = 80:10:10, as the mass ratio.

### (Preparation of negative electrode)

As a negative electrode sheet there was prepared a commercially available graphite-coated electrode sheet (product name: Electrode sheet negative electrode monolayer, by Hohsen Co.).

### (Fabrication of non-aqueous electrolyte solution secondary battery)

Cell elements were fabricated by laminating a negative electrode sheet, a polyethylene separator, a positive electrode sheet, a polyethylene separator and a negative electrode sheet, in that order, in the different non-aqueous electrolyte solutions obtained in the examples and comparative examples. Each cell element was inserted into a bag formed from a laminate film having an aluminum layer (thickness: 40 µm) and a resin layer covering both sides thereof, with the positive electrode sheet and negative electrode sheet ends protruding out from the bag. Next, each of the non-aqueous electrolyte solutions obtained in the examples and comparative examples was infused into the bag. The bag was vacuum sealed to obtain a sheet-like non-aqueous electrolyte solution secondary battery. In order to increase the adhesiveness between the electrodes, the sheet-like non-aqueous electrolyte solution secondary battery was sandwiched between glass plates and pressed.

### (Evaluation of service capacity maintenance factor and internal resistance ratio)

A procedure of charging the non-aqueous electrolyte solution secondary battery to 4.2 V with a current corresponding to 0.2 C and then discharging to 3 V with a current corresponding to 0.2 C, at 25°C, was repeated for 2 cycles to stabilize the battery.

It was then subjected to a charge-discharge cycle test with a charge rate of 0.3 C, a discharge rate of 0.3 C, a charge final voltage of 4.2 V and a discharge final voltage of 2.5 V. Table 16 shows the service capacity maintenance factor (%) after 200 cycles and the internal resistance ratio after 200 cycles, for each battery.

The "service capacity maintenance factor (%) after 200 cycles" is the ratio (percentage) of the service capacity (mAh) after a 200 cycle test with respect to the service capacity (mAh) after a 10 cycle test. Also, the "internal resistance ratio after 200 cycles" is the relative value of the resistance after a 200 cycle test, where the resistance before the cycle test is defined as 1.

**Table 16**

| | Electrolyte | Solvent | Additive | Service capacity maintenance factor (%) | Internal resistance ratio |
|---|---|---|---|---|---|
| Ex. D11 | LiPF₆ 1.0 mol/L | EC/DEC (30/70) vol% | Compound D13 1.0 mass% | 93 | 1.38 |
| Ex. D12 | LiPF₆ 1.0 mol/L | EC/DEC (30/70) vol% | Compound D14 1.0 mass% | 92 | 1.37 |
| Ex. D13 | LiPF₆ 1.0 mol/L | EC/DEC (30/70) vol% | Compound D15 1.0 mass% | 91 | 1.38 |
| Ex. D14 | LiPF₆ 1.0 mol/L | EC/DEC (30/70) vol% | Compound D16 1.0 mass% | 93 | 1.39 |
| Ex. D15 | LiPF₆ 1.0 mol/L | EC/DEC (30/70) vol% | Compound D17 1.0 mass% | 94 | 1.34 |
| Comp. Ex. D10 | LiPF₆ 1.0 mol/L | EC/DEC (30/70) vol% | None | 79 | 1.83 |
| Comp. Ex. D11 | LiPF₆ 1.0 mol/L | EC/DEC (30/70) vol% | 1,3-Propanesultone 1.0 mass% | 81 | 1.68 |
| Comp. Ex. D12 | LiPF₆ 1.0 mol/L | EC/DEC (30/70) vol% | VC 1.0 mass% | 81 | 1.69 |
| Comp. Ex. D13 | LiPF₆ 1.0 mol/L | EC/DEC (30/70) vol% | VC 2.0 mass% | 82 | 1.53 |
| Comp. Ex. D14 | LiPF₆ 1.0 mol/L | EC/DEC (30/70) vol% | FEC 1.0 mass% | 84 | 1.66 |
| Comp. Ex. D15 | LiPF₆ 1.0 mol/L | EC/DEC (30/70) vol% | FEC 2.0 mass% | 86 | 1.67 |
| Comp. Ex. D16 | LiPF₆ 1.0 mol/L | EC/DEC (30/70) vol% | Saccharin 1.0 mass% | 87 | 1.42 |

### (Measurement of gas generation volume)

Separately from the batteries used for the cycle test, there were prepared non-aqueous electrolyte solution secondary batteries having the same construction, containing the electrolyte solutions of examples D11-D15 and Comparative Examples D1, D14 and D12. A procedure of charging each battery to 4.2 V with a current corresponding to 0.2 C and then discharging to 3 V with a current corresponding to 0.2 C, at 25°C, was repeated for 2 cycles to stabilize the battery.

Next, charging of the battery was again carried out to 4.2 V with the charge rate at 0.3 C, after which the battery was allowed to stand in an environment of 60°C for 168 hours. The battery was then cooled to room temperature. The gas generation volume of the battery after standing was measured by Archimedes' method. The results for the non-aqueous electrolyte solution secondary batteries including the electrolyte solutions of examples D11-D15 and Comparative Examples D1, D14 and D12 are shown in Table 17, as Examples D16-D20 and Comparative Examples D17-D19, respectively.

**Table 17**

| | Electrolyte | Solvent | Additive | Gas generation volume (ml) |
|---|---|---|---|---|
| Ex. D16 | LiPF₆ 1.0 mol/L | EC/DEC (30/70) vol% | Compound D13 1.0 mass% | 0.31 |
| Ex. D17 | LiPF₆ 1.0 mol/L | EC/DEC (30/70) vol% | Compound D14 1.0 mass% | 0.36 |
| Ex. D18 | LiPF₆ 1.0 mol/L | EC/DEC (30/70) vol% | Compound D15 1.0 mass% | 0.35 |
| Ex. D19 | LiPF₆ 1.0 mol/L | EC/DEC (30/70) vol% | Compound D16 1.0 mass% | 0.33 |
| Ex. D20 | LiPF₆ 1.0 mol/L | EC/DEC (30/70) vol% | Compound D17 1.0 mass% | 0.27 |
| Comp. Ex. D17 | LiPF₆ 1.0 mol/L | EC/DEC (30/70) vol% | None | 0.54 |
| Comp. Ex. D18 | LiPF₆ 1.0 mol/L | EC/DEC (30/70) vol% | FEC 1.0 mass% | 0.58 |
| Comp. Ex. D19 | LiPF₆ 1.0 mol/L | EC/DEC (30/70) vol% | VC 1.0 mass% | 0.59 |

From Table 16 and Table 17 it is seen that the non-aqueous electrolyte solution secondary batteries using the non-aqueous electrolyte solutions of each of the examples which included compounds D13, D14, D15, D16 and D17, as compounds of formula (D1) and formula (D2), were excellent in terms of both service capacity maintenance factor during the cycle test and minimizing stored gas generation with charging, compared to the non-aqueous electrolyte solution secondary batteries using the non-aqueous electrolyte solutions of the comparative examples. This strongly suggests that the compounds of formula (D1) and formula (D2), when used in non-aqueous electrolyte solution secondary batteries, form SEI that are stable against charge-discharge cycles and high temperature storage. It also confirmed that the compounds of formula (D1) and formula (D2) are excellent in terms of low increase in internal resistance with charge-discharge cycling.

### <Experimental Example E>

### 1. Preparation of non-aqueous electrolyte solution

### (Example E1)

Ethylene carbonate (EC) and diethyl carbonate (DEC) were mixed in a volume ratio of EC:DEC = 30:70 to prepare a mixed non-aqueous solvent, and LiPF₆ as an electrolyte was dissolved in the mixture to a concentration of 1.0 mol/L. To the obtained solution there was added compound E1 shown in Table 18 as an additive for a non-aqueous electrolyte solution, to prepare a non-aqueous electrolyte solution. The content ratio of the additive for a non-aqueous electrolyte solution (compound E1) was 0.5 mass% based on the total mass of the non-aqueous electrolyte solution.

### (Example E2)

A non-aqueous electrolyte solution was prepared in the same manner as Example E1, except that the content ratio of compound E1 was 1.0 mass%.

### (Example E3)

A non-aqueous electrolyte solution was prepared in the same manner as Example E1, except that the additive for a non-aqueous electrolyte solution was changed from compound E1 to compound E2 shown in Table 18, and the content ratio was 1.0 mass%.

### (Example E4)

A non-aqueous electrolyte solution was prepared in the same manner as Example E1, except that the additive for a non-aqueous electrolyte solution was changed from compound E1 to compound E3 shown in Table 18, and the content ratio was 1.0 mass%.

### (Example E5)

A non-aqueous electrolyte solution was prepared in the same manner as Example E1, except that the additive for a non-aqueous electrolyte solution was changed from compound E1 to compound E4 shown in Table 18, and the content ratio was 1.0 mass%.

### (Example E6)

A non-aqueous electrolyte solution was prepared in the same manner as Example E1, except that the additive for a non-aqueous electrolyte solution was changed from compound E1 to compound E5 shown in Table 18, and the content ratio was 1.0 mass%.

### (Example E7)

A non-aqueous electrolyte solution was prepared in the same manner as Example E1, except that the additive for a non-aqueous electrolyte solution was changed from compound E1 to compound E6 shown in Table 18, and the content ratio was 1.0 mass%.

### (Example E8)

A non-aqueous electrolyte solution was prepared in the same manner as Example E1, except that the additive for a non-aqueous electrolyte solution was changed from compound E1 to compound E7 shown in Table 18, and the content ratio was 1.0 mass%.

### (Example E9)

A non-aqueous electrolyte solution was prepared in the same manner as Example E1, except that the additive for a non-aqueous electrolyte solution was changed from compound E1 to compound E8 shown in Table 18, and the content ratio was 1.0 mass%.

### (Comparative Example E1)

A non-aqueous electrolyte solution was prepared in the same manner as Example E1, except that compound E1 was not added.

### (Comparative Example E2)

A non-aqueous electrolyte solution was prepared in the same manner as Example E1, except that the additive for a non-aqueous electrolyte solution was changed from compound E1 to 1,3-propanesultone, and the content ratio was 1.0 mass%.

### (Comparative Example E3)

A non-aqueous electrolyte solution was prepared in the same manner as Example E1, except that the additive for a non-aqueous electrolyte solution was changed from compound E1 to vinylene carbonate (VC), and the content ratio was 1.0 mass%.

### (Comparative Example E4)

A non-aqueous electrolyte solution was prepared in the same manner as Comparative Example E3, except that the content ratio of the vinylene carbonate (VC) was 2.0 mass%.

### (Comparative Example E5)

A non-aqueous electrolyte solution was prepared in the same manner as Example E1, except that the additive for a non-aqueous electrolyte solution was changed from compound E1 to fluoroethylene carbonate (FEC), and the content ratio was 1.0 mass%.

### (Comparative Example E6)

A non-aqueous electrolyte solution was prepared in the same manner as Comparative Example E5, except that the content ratio of the fluoroethylene carbonate (FEC) was 2.0 mass%.

### (Comparative Example E7)

A non-aqueous electrolyte solution was prepared in the same manner as Example E1, except that the additive for a non-aqueous electrolyte solution was changed from compound E1 to phthalimide, and the content ratio was 1.0 mass%.

### (Comparative Example E8)

A non-aqueous electrolyte solution was prepared in the same manner as Example E1, except that the additive for a non-aqueous electrolyte solution was changed from compound E1 to maleimide, and the content ratio was 1.0 mass%.

### (Comparative Example E9)

A non-aqueous electrolyte solution was prepared in the same manner as Example E1, except that the additive for a non-aqueous electrolyte solution was changed from compound E1 to succinimide, and the content ratio was 1.0 mass%.

### (Comparative Example E10)

A non-aqueous electrolyte solution was prepared in the same manner as Example E1, except that the additive for a non-aqueous electrolyte solution was changed from compound E1 to saccharin, and the content ratio was 1.0 mass%.

### 2. Evaluation

### (Calculation of LUMO energy)

The LUMO (lowest unoccupied molecular orbital) energies of compounds E1, E2, E3, E4, E5, E6, E7 and E8 used in the Examples were determined by calculating the semiempirical molecular orbitals using Gaussian 03 software. The calculated LUMO energies are shown in Table 18.

**Table 18**

| | Compound structure | LUMO energy (eV) | | Compound structure | LUMO energy (eV) |
|---|---|---|---|---|---|
| Comp ound E1 | 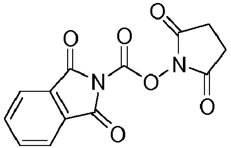 | -2.48 | Comp ound E5 | 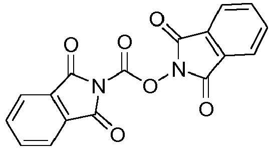 | -2.22 |
| Comp ound E2 | 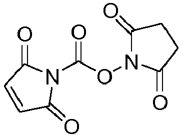 | -1.46 | Comp ound E6 | 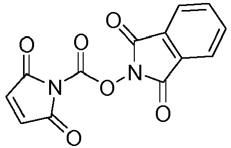 | -2.32 |
| Comp ound E3 | 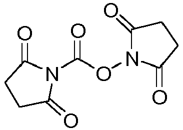 | -1.18 | Comp ound E7 | 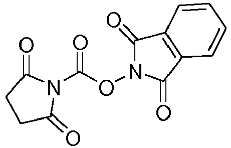 | -2.40 |
| Comp ound E4 | 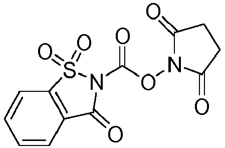 | -2.39 | Comp ound E8 | 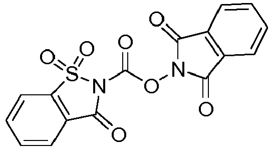 | -2.41 |

### (Stability)

The compounds E1, E2, E3, E4, E5, E6, E7 and E8 used in the examples and the fluoroethylene carbonate (FEC) used in Comparative Examples E5 and E6 were supplied for a preservation test in which they were allowed to stand for 90 days in a steady temperature and humidity environment at a temperature of 40 ±2°C and a humidity of 75 ±5%. The ¹H-nuclear magnetic resonance spectrum (¹H-NMR) of each additive for a non-aqueous electrolyte solution was measured before and after the preservation test, and the stability of each compound was evaluated based on the following scale. Table 19 shows the evaluation results for stability.
G: No change in ¹H-NMR spectrum peaks before and after preservation test.
F: Slight change in ¹H-NMR spectrum peaks before and after preservation test.
P: Distinct change in ¹H-NMR spectrum peaks before and after preservation test.

**Table 19**

| Additive | Stability |
|---|---|
| Compound E1 | G |
| Compound E2 | G |
| Compound E3 | G |
| Compound E4 | G |
| Compound E5 | G |
| Compound E6 | G |
| Compound E7 | G |
| Compound E8 | G |
| FEC | P |

As shown in Table 19, the fluoroethylene carbonate (FEC) used in Comparative Examples E5 and E6 was presumably partially hydrolyzed, and had inferior stability. Compounds E1, E2, E3, E4, E5, E6, E7 and E8 used in the examples, on the other hand, exhibited excellent stability.

### (Fabrication of non-aqueous electrolyte solution secondary battery)

LiMn₂O₄ as a positive electrode active material and carbon black as a conductivity imparting agent were dry blended. The obtained mixture was uniformly dispersed in N-methyl-2-pyrrolidone (NMP) dissolving polyvinylidene fluoride (PVDF) as a binder, to prepare a slurry. The obtained slurry was coated onto both sides of an aluminum metal foil (rectilinear, thickness: 20 µm). After drying the coating film to remove the NMP, the entire foil was pressed to obtain a positive electrode sheet having an aluminum metal foil as the positive electrode collector and a positive electrode active material layer formed on both sides. The solid ratio of the positive electrode active material layer of the obtained positive electrode sheet was positive electrode active material:conductivity imparting agent:PVDF = 90:5:5, as the mass ratio. As a negative electrode sheet there was used a commercially available graphite-coated electrode sheet (product name: Electrode sheet negative electrode monolayer, by Hohsen Co.).

Cell elements were fabricated by laminating a polyethylene separator in the order of a negative electrode, a separator, a positive electrode, a separator and a negative electrode in the different non-aqueous electrolyte solutions obtained in the examples and comparative examples. Each cell element was inserted into a bag, formed of a laminate film comprising aluminum (thickness: 40 µm) having both sides covered with a resin layer, with the positive and negative electrode terminals protruding out. Next, each of the non-aqueous electrolyte solutions obtained in examples E1-E9 and Comparative Examples E1-E10 were infused into the bag and vacuum sealed, to fabricate a sheet-like non-aqueous electrolyte solution secondary battery. Also, in order to increase the adhesiveness between the electrodes, the sheet-like battery was sandwiched between glass plates and pressed, to fabricate a non-aqueous electrolyte solution secondary battery (secondary battery sheet).

### (Evaluation of service capacity maintenance factor and internal resistance ratio)

Each of the obtained non-aqueous electrolyte solution secondary batteries was subjected to a charge-discharge cycle test at 25°C, with a charge rate of 0.3 C, a discharge rate of 0.3 C, a charge final voltage of 4.2 V and a discharge final voltage of 2.5 V. Table 20 shows the service capacity maintenance factor (%) after 200 cycles and the internal resistance ratio after 200 cycles.

The "service capacity maintenance factor (%) after 200 cycles" is the ratio (percentage) of the service capacity (mAh) after a 200 cycle test with respect to the service capacity (mAh) after a 10 cycle test. Also, the "internal resistance ratio after 200 cycles" is the relative value of the resistance after a 200 cycle test, where the resistance before the cycle test is defined as 1.

**Table 20**

| | Electrolyte | Solvent | Additive | Service capacity maintenance factor (%) | Internal resistance ratio |
|---|---|---|---|---|---|
| Ex. E1 | LiPF₆ 1.0 mol/L | EC/DEC (30/70) vol% | Compound E1 0.5 mass% | 88 | 1.23 |
| Ex. E2 | LiPF₆ 1.0 mol/L | EC/DEC (30/70) vol% | Compound E1 1.0 mass% | 92 | 1.38 |
| Ex. E3 | LiPF₆ 1.0 mol/L | EC/DEC (30/70) vol% | Compound E2 1.0 mass% | 92 | 1.33 |
| Ex. E4 | LiPF₆ 1.0 mol/L | EC/DEC (30/70) vol% | Compound E3 1.0 mass% | 94 | 1.31 |
| Ex. E5 | LiPF₆ 1.0 mol/L | EC/DEC (30/70) vol% | Compound E4 1.0 mass% | 93 | 1.36 |
| Ex. E6 | LiPF₆ 1.0 mol/L | EC/DEC (30/70) vol% | Compound E5 1.0 mass% | 94 | 1.35 |
| Ex. E7 | LiPF₆ 1.0 mol/L | EC/DEC (30/70) vol% | Compound E6 1.0 mass% | 92 | 1.33 |
| Ex. E8 | LiPF₆ 1.0 mol/L | EC/DEC (30/70) vol% | Compound E7 1.0 mass% | 92 | 1.39 |
| Ex. E9 | LiPF₆ 1.0 mol/L | EC/DEC (30/70) vol% | Compound E8 1.0 mass% | 91 | 1.35 |
| Comp. Ex. E1 | LiPF₆ 1.0 mol/L | EC/DEC (30/70) vol% | None | 79 | 1.83 |
| Comp. Ex. E2 | LiPF₆ 1.0 mol/L | EC/DEC (30/70) vol% | 1,3-Propanesultone 1.0 mass% | 81 | 1.68 |
| Comp. Ex. E3 | LiPF₆ 1.0 mol/L | EC/DEC (30/70) vol% | VC 1.0 mass% | 81 | 1.69 |
| Comp. Ex. E4 | LiPF₆ 1.0 mol/L | EC/DEC (30/70) vol% | VC 2.0 mass% | 82 | 1.53 |
| Comp. Ex. E5 | LiPF₆ 1.0 mol/L | EC/DEC (30/70) vol% | FEC 1.0 mass% | 84 | 1.66 |
| Comp. Ex. E6 | LiPF₆ 1.0 mol/L | EC/DEC (30/70) vol% | FEC 2.0 mass% | 86 | 1.67 |
| Comp. Ex. E7 | LiPF₆ 1.0 mol/L | EC/DEC (30/70) vol% | Phthalimide 1.0 mass% | 87 | 1.45 |
| Comp. Ex. E8 | LiPF₆ 1.0 mol/L | EC/DEC (30/70) vol% | Maleimide 1.0 mass% | 86 | 1.55 |
| Comp. Ex. E9 | LiPF₆ 1.0 mol/L | EC/DEC (30/70) vol% | Succinimide 1.0 mass% | 85 | 1.49 |
| Comp. Ex. E10 | LiPF₆ 1.0 mol/L | EC/DEC (30/70) vol% | Saccharin 1.0 mass% | 87 | 1.42 |

### (Measurement of gas generation volume)

Separately from the battery used for the cycle test, there was prepared a non-aqueous electrolyte solution secondary battery having the same construction, containing each of the electrolyte solutions of the examples and comparative examples. A procedure of charging each battery to 4.2 V with a current corresponding to 0.2 C and then discharging to 3 V with a current corresponding to 0.2 C, at 25°C, was repeated for 3 cycles to stabilize the battery. Next, charging of the battery was again carried out to 4.2 V with the charge rate at 0.3 C, after which the battery was stored at a high temperature of 60°C for 168 hours. The battery was then cooled to room temperature, the volume of the battery was measured by Archimedes' method, and the gas generation volume was determined from the volume change before and after storage.

**Table 21**

| | Electrolyte | Solvent | Additive | Gas generation volume (ml) |
|---|---|---|---|---|
| Ex. E10 | LiPF₆ 1.0 mol/L | EC/DEC (30/70) vol% | Compound E1 1.0 mass% | 0.42 |
| Ex. E11 | LiPF₆ 1.0 mol/L | EC/DEC (30/70) vol% | Compound E2 1.0 mass% | 0.40 |
| Ex. E12 | LiPF₆ 1.0 mol/L | EC/DEC (30/70) vol% | Compound E3 1.0 mass% | 0.41 |
| Ex. E13 | LiPF₆ 1.0 mol/L | EC/DEC (30/70) vol% | Compound E4 1.0 mass% | 0.39 |
| Ex. E14 | LiPF₆ 1.0 mol/L | EC/DEC (30/70) vol% | Compound E5 1.0 mass% | 0.41 |
| Ex. E15 | LiPF₆ 1.0 mol/L | EC/DEC (30/70) vol% | Compound E6 1.0 mass% | 0.44 |
| Ex. E16 | LiPF₆ 1.0 mol/L | EC/DEC (30/70) vol% | Compound E7 1.0 mass% | 0.43 |
| Ex. E17 | LiPF₆ 1.0 mol/L | EC/DEC (30/70) vol% | Compound E8 1.0 mass% | 0.45 |
| Comp. Ex. E11 | LiPF₆ 1.0 mol/L | EC/DEC (30/70) vol% | None | 0.54 |
| Comp. Ex. E12 | LiPF₆ 1.0 mol/L | EC/DEC (30/70) vol% | FEC 1.0 mass% | 0.58 |
| Comp. Ex. E13 | LiPF₆ 1.0 mol/L | EC/DEC (30/70) vol% | VC 1.0 mass% | 0.59 |

From Table 20 it is seen that the non-aqueous electrolyte solution secondary batteries using the non-aqueous electrolyte solutions of each of the examples which included compounds E1, E2, E3, E4, E5, E6, E7 and E8, as compounds of formula (E1) and formula (E2), had high service capacity maintenance factors during the cycle test, compared to the non-aqueous electrolyte solution secondary batteries using the non-aqueous electrolyte solutions of the comparative examples. Also, from Table 21 it is seen that the non-aqueous electrolyte solution secondary batteries using the non-aqueous electrolyte solutions of each of the examples which included compounds E1, E2, E3, E4, E5, E6, E7 and E8, as compounds of formula (E1) and formula (E2), had low gas generation. This strongly suggests that the compounds of formula (E1) or formula (E2), when used in non-aqueous electrolyte solution secondary batteries, form SEI that are stable against charge-discharge cycles and high temperature storage. It also confirmed that the compounds of formula (E1) or formula (E2) are also excellent in terms of low increase in internal resistance with charge-discharge cycling.

### <Experimental Example F>

### 1. Preparation of non-aqueous electrolyte solution

### (Example F1)

Ethylene carbonate (EC) and diethyl carbonate (DEC) were mixed in a volume compositional ratio of EC:DEC = 30:70 to obtain a mixed non-aqueous solvent. In the obtained mixed non-aqueous solvent there was dissolved LiPF₆ as an electrolyte to a concentration of 1.0 mol/L. To the obtained solution there was added compound F1 shown in Table 22 as an additive for a non-aqueous electrolyte solution, to prepare a non-aqueous electrolyte solution. The content ratio of the additive for a non-aqueous electrolyte solution (compound F1) was 0.5 mass% with respect to the total mass of the non-aqueous electrolyte solution.

### (Example F2)

A non-aqueous electrolyte solution was prepared in the same manner as Example F1, except that the content ratio of compound F1 was 1.0 mass%.

### (Example F3)

A non-aqueous electrolyte solution was prepared in the same manner as Example F1, except that the additive for a non-aqueous electrolyte solution was changed from compound F1 to compound F2 shown in Table 22, and the content ratio was 1.0 mass%.

### (Example F4)

A non-aqueous electrolyte solution was prepared in the same manner as Example F1, except that the additive for a non-aqueous electrolyte solution was changed from compound F1 to compound F3 shown in Table 22, and the content ratio was 1.0 mass%.

### (Example F5)

A non-aqueous electrolyte solution was prepared in the same manner as Example F1, except that the additive for a non-aqueous electrolyte solution was changed from compound F1 to compound F4 shown in Table 22, and the content ratio was 1.0 mass%.

### (Example F6)

A non-aqueous electrolyte solution was prepared in the same manner as Example F1, except that the additive for a non-aqueous electrolyte solution was changed from compound F1 to compound F5 shown in Table 22, and the content ratio was 1.0 mass%.

### (Example F7)

A non-aqueous electrolyte solution was prepared in the same manner as Example F1, except that the additive for a non-aqueous electrolyte solution was changed from compound F1 to compound F6 shown in Table 22, and the content ratio was 1.0 mass%.

### (Example F8)

A non-aqueous electrolyte solution was prepared in the same manner as Example F1, except that the additive for a non-aqueous electrolyte solution was changed from compound F1 to compound F7 shown in Table 22, and the content ratio was 1.0 mass%.

### (Example F9)

A non-aqueous electrolyte solution was prepared in the same manner as Example F1, except that the additive for a non-aqueous electrolyte solution was changed from compound F1 to compound F8 shown in Table 22, and the content ratio was 1.0 mass%.

### (Comparative Example F1)

A non-aqueous electrolyte solution was prepared in the same manner as Example F1, except that compound F1 was not added.

### (Comparative Example F2)

A non-aqueous electrolyte solution was prepared in the same manner as Example F1, except that the additive for a non-aqueous electrolyte solution was changed from compound F1 to 1,3-propanesultone, and the content ratio was 1.0 mass%.

### (Comparative Example F3)

A non-aqueous electrolyte solution was prepared in the same manner as Example F1, except that the additive for a non-aqueous electrolyte solution was changed from compound F1 to vinylene carbonate (VC), and the content ratio was 1.0 mass%.

### (Comparative Example F4)

A non-aqueous electrolyte solution was prepared in the same manner as Comparative Example F3, except that the content ratio of the vinylene carbonate (VC) was 2.0 mass%.

### (Comparative Example F5)

A non-aqueous electrolyte solution was prepared in the same manner as Example F1, except that the additive for a non-aqueous electrolyte solution was changed from compound F1 to fluoroethylene carbonate (FEC), and the content ratio was 1.0 mass%.

### (Comparative Example F6)

A non-aqueous electrolyte solution was prepared in the same manner as Comparative Example F5, except that the content ratio of the fluoroethylene carbonate (FEC) was 2.0 mass%.

### (Comparative Example F7)

A non-aqueous electrolyte solution was prepared in the same manner as Example F1, except that the additive for a non-aqueous electrolyte solution was changed from compound F1 to phthalimide, and the content ratio was 1.0 mass%.

### (Comparative Example F8)

A non-aqueous electrolyte solution was prepared in the same manner as Example F1, except that the additive for a non-aqueous electrolyte solution was changed from compound F1 to maleimide, and the content ratio was 1.0 mass%.

### (Comparative Example F9)

A non-aqueous electrolyte solution was prepared in the same manner as Example F1, except that the additive for a non-aqueous electrolyte solution was changed from compound F1 to succinimide, and the content ratio was 1.0 mass%.

### (Comparative Example F10)

A non-aqueous electrolyte solution was prepared in the same manner as Example F1, except that the additive for a non-aqueous electrolyte solution was changed from compound F1 to saccharin, and the content ratio was 1.0 mass%.

### 2. Evaluation

### (Measurement of LUMO energy)

The LUMO (lowest unoccupied molecular orbital) energies of compounds F1, F2, F3, F4, F5, F6, F7 and F8 used in the Examples were determined by calculating the semiempirical molecular orbitals using Gaussian 03 software. The calculated LUMO energies are shown in Table 22.

**Table 22**

| | Compound structure | LUMO energy (eV) | | Compound structure | LUMO energy (eV) |
|---|---|---|---|---|---|
| Compound F1 | 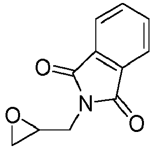 | -2.11 | Compound F5 | 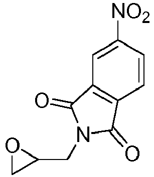 | -2.18 |
| Compound F2 | 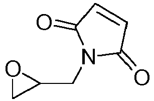 | -2.20 | Compound F6 | 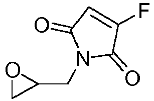 | -2.23 |
| Compound F3 | 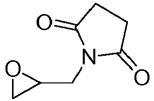 | -0.56 | Compound F7 | 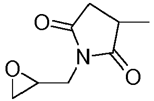 | -0.50 |
| Compound F4 | 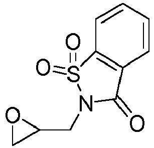 | -2.03 | Compound F8 | 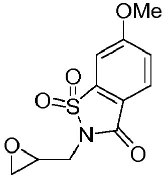 | -1.85 |

### (Stability)

The compounds F1, F2, F3, F4, F5, F6, F7 and F8 used in the examples and the fluoroethylene carbonate (FEC) used in Comparative Examples F5 and F6 were supplied for a preservation test in which they were allowed to stand for 90 days in a steady temperature and humidity environment at a temperature of 40 ±2°C and a humidity of 75 ±5%. The ¹H-nuclear magnetic resonance spectrum (¹H-NMR) of each additive for a non-aqueous electrolyte solution was measured before and after the preservation test, and the stability of each compound was evaluated based on the following scale. Table 23 shows the evaluation results for stability.
G: No change in ¹H-NMR spectrum peaks before and after preservation test.
F: Slight change in ¹H-NMR spectrum peaks before and after preservation test.
P: Distinct change in ¹H-NMR spectrum peaks before and after preservation test.

**Table 23**

| Additive | Stability |
|---|---|
| Compound F1 | G |
| Compound F2 | G |
| Compound F3 | G |
| Compound F4 | G |
| Compound F5 | G |
| Compound F6 | G |
| Compound F7 | G |
| Compound F8 | G |
| FEC | P |

As shown in Table 23, the fluoroethylene carbonate (FEC) used in Comparative Examples F5 and F6 was presumably partially hydrolyzed, and had inferior stability. Compounds F1, F2, F3, F4, F5, F6, F7 and F8 used in the examples, on the other hand, exhibited virtually no change and had excellent stability.

### (Fabrication of positive electrode)

LiMn₂O₄ as a positive electrode active material and carbon black as a conductivity imparting agent were dry blended. The obtained mixture was uniformly dispersed in N-methyl-2-pyrrolidone (NMP) dissolving polyvinylidene fluoride (PVDF) as a binder, to prepare a slurry. The obtained slurry was coated onto both sides of an aluminum metal foil (rectilinear, thickness: 20 µm). After drying the coating film to remove the NMP, the entire foil was pressed to obtain a positive electrode sheet having an aluminum metal foil as the positive electrode collector and a positive electrode active material layer formed on both sides. The solid ratio of the positive electrode active material layer of the obtained positive electrode sheet was positive electrode active material:conductivity imparting agent:PVDF = 90:5:5, as the mass ratio.

### (Preparation of negative electrode)

Separately, as a negative electrode sheet, there was used a commercially available graphite-coated electrode sheet (product name: Electrode sheet negative electrode monolayer, by Hohsen Co.).

### (Fabrication of non-aqueous electrolyte solution secondary battery)

Cell elements were fabricated by laminating a negative electrode sheet, a polyethylene separator, a positive electrode sheet, a polyethylene separator and a negative electrode sheet, in that order, in the non-aqueous electrolyte solutions obtained in the examples and comparative examples. Each cell element was inserted into a bag formed from a laminate film having an aluminum layer (thickness: 40 µm) and a resin layer covering both sides thereof, with the positive electrode sheet and negative electrode sheet ends protruding out from the bag. Next, each of the non-aqueous electrolyte solutions obtained in the examples and comparative examples was infused into the bag. The bag was vacuum sealed to obtain a sheet-like non-aqueous electrolyte solution secondary battery. In order to increase the adhesiveness between the electrodes, the sheet-like non-aqueous electrolyte solution secondary battery was sandwiched between glass plates and pressed.

### (Evaluation of service capacity maintenance factor and internal resistance ratio)

The obtained non-aqueous electrolyte solution secondary batteries were subjected to a charge-discharge cycle test at 25°C, with a charge rate of 0.3 C, a discharge rate of 0.3 C, a charge final voltage of 4.2 V and a discharge final voltage of 2.5 V. The service capacity maintenance factor (%) after 200 cycles and the internal resistance ratio after 200 cycles are shown in Table 3. The "service capacity maintenance factor (%)" after 200 cycles is the value obtained by dividing the service capacity (mAh) after a 200 cycle test by the service capacity (mAh) after a 10 cycle test, and multiplying by 100. Also, the "internal resistance ratio" after 200 cycles is represented by the relative value of the resistance after a 200 cycle test, where the resistance before the cycle test is defined as 1.

**Table 24**

| | Electrolyte | Solvent | Additive | Service capacity maintenance factor (%) | Internal resistance ratio |
|---|---|---|---|---|---|
| Ex. F1 | LiPF₆ 1.0 mol/L | EC/DEC (30/70) vol% | Compound F1 0.5 mass% | 91 | 1.23 |
| Ex. F2 | LiPF₆ 1.0 mol/L | EC/DEC (30/70) vol% | Compound F1 1.0 mass% | 93 | 1.38 |
| Ex. F3 | LiPF₆ 1.0 mol/L | EC/DEC (30/70) vol% | Compound F2 1.0 mass% | 91 | 1.33 |
| Ex. F4 | LiPF₆ 1.0 mol/L | EC/DEC (30/70) vol% | Compound F3 1.0 mass% | 93 | 1.31 |
| Ex. F5 | LiPF₆ 1.0 mol/L | EC/DEC (30/70) vol% | Compound F4 1.0 mass% | 93 | 1.36 |
| Ex. F6 | LiPF₆ 1.0 mol/L | EC/DEC (30/70) vol% | Compound F5 1.0 mass% | 91 | 1.33 |
| Ex. F7 | LiPF₆ 1.0 mol/L | EC/DEC (30/70) vol% | Compound F6 1.0 mass% | 93 | 1.33 |
| Ex. F8 | LiPF₆ 1.0 mol/L | EC/DEC (30/70) vol% | Compound F7 1.0 mass% | 92 | 1.34 |
| Ex. F9 | LiPF₆ 1.0 mol/L | EC/DEC (30/70) vol% | Compound F8 1.0 mass% | 90 | 1.32 |
| Comp. Ex. F1 | LiPF₆ 1.0 mol/L | EC/DEC (30/70) vol% | None | 79 | 1.83 |
| Comp. Ex. F2 | LiPF₆ 1.0 mol/L | EC/DEC (30/70) vol% | 1,3-Propanesultone 1.0 mass% | 81 | 1.68 |
| Comp. Ex. F3 | LiPF₆ 1.0 mol/L | EC/DEC (30/70) vol% | VC 1.0 mass% | 81 | 1.69 |
| Comp. Ex. F4 | LiPF₆ 1.0 mol/L | EC/DEC (30/70) vol% | VC 2.0 mass% | 82 | 1.53 |
| Comp. Ex. F5 | LiPF₆ 1.0 mol/L | EC/DEC (30/70) vol% | FEC 1.0 mass% | 84 | 1.66 |
| Comp. Ex. F6 | LiPF₆ 1.0 mol/L | EC/DEC (30/70) vol% | FEC 2.0 mass% | 86 | 1.67 |
| Comp. Ex. F7 | LiPF₆ 1.0 mol/L | EC/DEC (30/70) vol% | Phthalimide 1.0 mass% | 87 | 1.45 |
| Comp. Ex. F8 | LiPF₆ 1.0 mol/L | EC/DEC (30/70) vol% | Maleimide 1.0 mass% | 86 | 1.55 |
| Comp. Ex. F9 | LiPF₆ 1.0 mol/L | EC/DEC (30/70) vol% | Succinimide 1.0 mass% | 85 | 1.49 |
| Comp. Ex. F10 | LiPF₆ 1.0 mol/L | EC/DEC (30/70) vol% | Saccharin 1.0 mass% | 87 | 1.42 |

### (Measurement of gas generation volume)

Separately from the battery used for the cycle test, there was prepared a non-aqueous electrolyte solution secondary battery having the same construction, containing each of the electrolyte solutions of the examples and comparative examples. A procedure of charging each battery to 4.2 V with a current corresponding to 0.2 C and then discharging to 3 V with a current corresponding to 0.2 C, at 25°C, was repeated for 3 cycles to stabilize the battery. Next, charging was again carried out to 4.2 V with the charge rate at 0.3 C, after which the battery was stored at a high temperature of 60°C for 168 hours. The battery was then cooled to room temperature, the volume of the battery was measured by Archimedes' method, and the gas generation volume was determined from the volume change before and after storage.

**Table 25**

| | Electrolyte | Solvent | Additive | Gas generation volume (ml) |
|---|---|---|---|---|
| Ex. F10 | LiPF₆ 1.0 mol/L | EC/DEC (30/70) vol% | Compound F1 1.0 mass% | 0.43 |
| Ex. F11 | LiPF₆ 1.0 mol/L | EC/DEC (30/70) vol% | Compound F2 1.0 mass% | 0.41 |
| Ex. F12 | LiPF₆ 1.0 mol/L | EC/DEC (30/70) vol% | Compound F3 1.0 mass% | 0.45 |
| Ex. F13 | LiPF₆ 1.0 mol/L | EC/DEC (30/70) vol% | Compound F4 1.0 mass% | 0.43 |
| Ex. F14 | LiPF₆ 1.0 mol/L | EC/DEC (30/70) vol% | Compound F5 1.0 mass% | 0.44 |
| Ex. F15 | LiPF₆ 1.0 mol/L | EC/DEC (30/70) vol% | Compound F6 1.0 mass% | 0.41 |
| Ex. F16 | LiPF₆ 1.0 mol/L | EC/DEC (30/70) vol% | Compound F7 1.0 mass% | 0.39 |
| Ex. F17 | LiPF₆ 1.0 mol/L | EC/DEC (30/70) vol% | Compound F8 1.0 mass% | 0.42 |
| Comp. Ex. F11 | LiPF₆ 1.0 mol/L | EC/DEC (30/70) vol% | None | 0.54 |
| Comp. Ex. F12 | LiPF₆ 1.0 mol/L | EC/DEC (30/70) vol% | FEC 1.0 mass% | 0.58 |
| Comp. Ex. F13 | LiPF₆ 1.0 mol/L | EC/DEC (30/70) vol% | VC 1.0 mass% | 0.59 |

From Table 24 and Table 25 it is seen that the non-aqueous electrolyte solution secondary batteries using the non-aqueous electrolyte solutions of each of the examples which included compound F1, F2, F3, F4, F5, F6, F7 or F8 as compounds of formula (F1), were excellent in terms of both service capacity maintenance factor during the cycle test and minimizing gas generation with charging, compared to the non-aqueous electrolyte solution secondary batteries using the non-aqueous electrolyte solutions of the comparative examples. This strongly suggests that the compounds of formula (F1), when used in non-aqueous electrolyte solution secondary batteries, form SEI that are stable against charge-discharge cycles and high temperature storage. It also confirmed that the compounds of formula (F1) are excellent in terms of low increase in internal resistance with charge-discharge cycling.

### Reference Signs List

1: Power storage device (non-aqueous electrolyte solution secondary battery), 2: positive electrode collector, 3: positive electrode active material layer, 4: positive plate, 5: negative electrode collector, 6: negative electrode active material layer, 7: negative plate, 8: non-aqueous electrolyte solution, 9: separator.

## Claims

1. An additive for a non-aqueous electrolyte solution including a compound represented by the following formula (A1) or formula (A2): 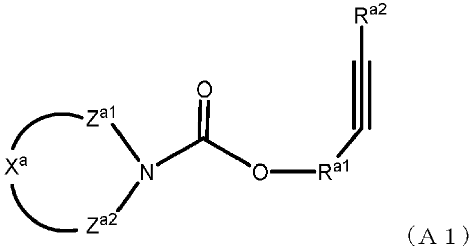 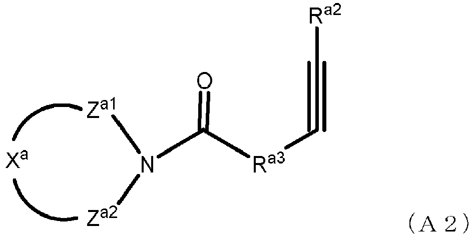 wherein in the formula, X^{a} represents a group that forms a cyclic group together with a nitrogen atom, Z^{a1} and Z^{a2}, Z^{a1} and Z^{a2} each independently represent a sulfonyl or carbonyl group, R^{a1} represents an optionally substituted alkylene group of 1 to 4 carbon atoms, an alkenylene group of 3 or 4 carbon atoms or an alkynylene group of 3 or 4 carbon atoms, R^{a2} represents a hydrogen atom, a halogen atom or an optionally substituted alkyl group of 1 to 4 carbon atoms, R^{a3} represents an oxygen atom, an optionally substituted alkylene group of 1 to 4 carbon atoms, an alkenylene group of 3 or 4 carbon atoms or an alkynylene group of 3 or 4 carbon atoms.

2. The additive for a non-aqueous electrolyte solution according to claim 1, including a compound represented by formula (A1).

3. An additive for a non-aqueous electrolyte solution including a compound represented by the following formula (B1): 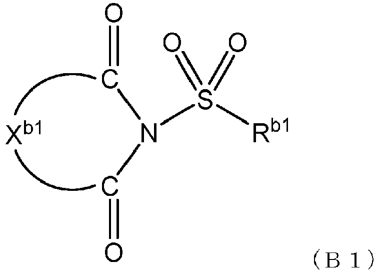 wherein in formula (B1), X^{b1} represents a group that forms a cyclic group together with a nitrogen atom and two carbon atoms, and R^{b1} represents an optionally substituted alkoxy group of 1 to 4 carbon atoms, an optionally substituted alkenyl group of 2 to 4 carbon atoms, an optionally substituted alkynyl group of 2 to 4 carbon atoms, an optionally substituted aryloxy group, an optionally substituted alkenyloxy group of 2 to 6 carbon atoms, an optionally substituted alkynyloxy group of 2 to 4 carbon atoms or an optionally substituted amino group.

4. An additive for a non-aqueous electrolyte solution including a compound represented by the following formula (C1): 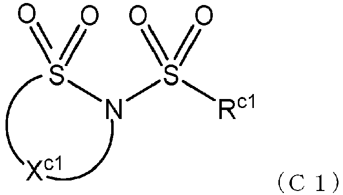 wherein in formula (C1), X^{c1} represents a group that forms a cyclic group together with a sulfur atom and a nitrogen atom, and R^{c1} represents an optionally substituted alkyl group of 1 to 4 carbon atoms, an optionally substituted alkynyl group of 1 to 4 carbon atoms, an optionally substituted alkenyl group of 2 to 4 carbon atoms, an optionally substituted alkoxy group of 1 to 4 carbon atoms, an optionally substituted aryl group, an optionally substituted heteroaryl group, an optionally substituted aryloxy group, an optionally substituted heteroaryloxy group, an optionally substituted alkenyloxy group of 2 to 4 carbon atoms, an optionally substituted alkynyloxy group of 2 to 4 carbon atoms, or an optionally substituted amino group.

5. The additive for a non-aqueous electrolyte solution according to claim 4, wherein the compound represented by formula (C1) is a compound represented by the following formula (C2): 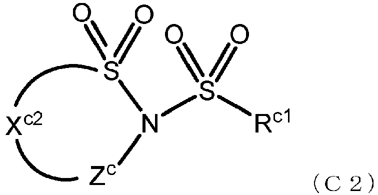 wherein in formula (C2), X^{c2} represents a group that forms a cyclic group together with a sulfur atom, a nitrogen atom and Z^{c}, Z^{c} represents a sulfonyl or carbonyl group, and R^{c1} has the same definition as R^{c1} in formula (C1).

6. An additive for a non-aqueous electrolyte solution including a compound represented by the following formula (D1): 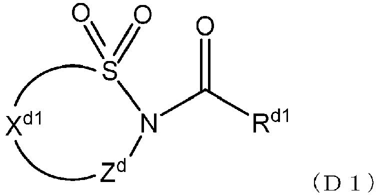 wherein in formula (D1), X^{d1} represents a group that forms a cyclic group together with a sulfur atom, a nitrogen atom and Z^{d}, Z^{d} represents a sulfonyl or carbonyl group, and R^{d1} represents an optionally substituted alkyl group of 1 to 4 carbon atoms, an optionally substituted alkenyl group of 2 to 4 carbon atoms, an optionally substituted alkoxy group of 1 to 4 carbon atoms, an optionally substituted aryl group, an optionally substituted heteroaryl group, an optionally substituted aryloxy group, an optionally substituted heteroaryloxy group, an optionally substituted alkenyloxy group of 2 to 4 carbon atoms or an optionally substituted amino group.

7. An additive for a non-aqueous electrolyte solution including a compound represented by the following formula (D2): 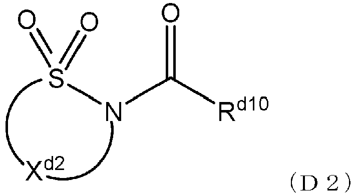 wherein in formula (D2), X^{d2} represents a group that forms a cyclic group together with the sulfur atom and nitrogen atom, and R^{d10} represents an optionally substituted alkenyl group of 2 to 4 carbon atoms, an optionally substituted alkoxy group of 1 to 4 carbon atoms, an optionally substituted aryl group, an optionally substituted heteroaryl group, an optionally substituted aryloxy group, an optionally substituted heteroaryloxy group, an optionally substituted alkenyloxy group of 2 to 4 carbon atoms or an optionally substituted amino group.

8. An additive for a non-aqueous electrolyte solution including a compound represented by the following formula (E1) or formula (E2): 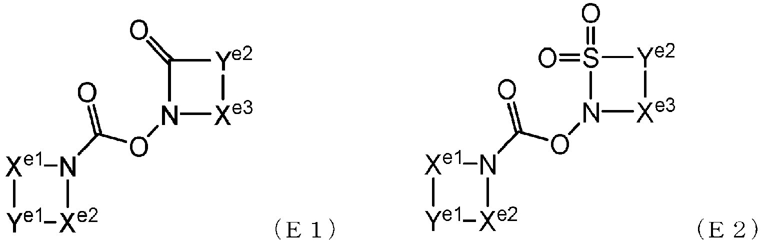 wherein in formula (E1) or formula (E2), X^{e1}, X^{e2} and X^{e3} each independently represent an optionally substituted methylene, sulfonyl or carbonyl group, in formula (E1) or formula (E2), Y^{e1} and Y^{e2} each independently represent an optionally substituted hydrocarbon group of 1 to 6 carbon atoms.

9. The additive for a non-aqueous electrolyte solution according to claim 8, wherein X^{e1}, X^{e2} and X^{e3} are each independently a sulfonyl or carbonyl group.

10. An additive for a non-aqueous electrolyte solution including a compound represented by the following formula (F1): 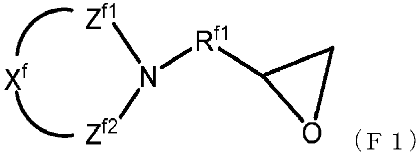 wherein in formula (F1), X^{f} represents a group that forms a cyclic group together with the nitrogen atom, Z^{f1} and Z^{f2}, Z^{f1} and Z^{f2} each independently represent a sulfonyl or carbonyl group, and R^{f1} represents a divalent linker group.

11. The additive for a non-aqueous electrolyte solution according to claim 10, wherein R^{f1} is an optionally substituted alkylene group of 1 to 4 carbon atoms.

12. A non-aqueous electrolyte solution comprising: an additive for a non-aqueous electrolyte solution according to any one of claims 1 to 11; a non-aqueous solvent; and an electrolyte.

13. The non-aqueous electrolyte solution according to claim 12, wherein the electrolyte includes a lithium salt.

14. A power storage device comprising: a non-aqueous electrolyte solution according to claim 12 or 13; a positive electrode; and a negative electrode.

15. A lithium ion battery comprising: a non-aqueous electrolyte solution according to claim 12 or 13; a positive electrode; and a negative electrode.

16. A lithium ion capacitor comprising: a non-aqueous electrolyte solution according to claim 12 or 13; a positive electrode; and a negative electrode.
